# EUROPEAN PATENT SPECIFICATION

(11) **EP 1 851 336 B1**
(45) Date of publication and mention of the grant of the patent: **08.09.2010**
(21) Application number: 06719447.2
(22) Date of filing: 25.01.2006
(51) Int. Cl.: C12Q 1/68

(54) **METHODS FOR QUANTITATING SMALL RNA MOLECULES**
VERFAHREN ZUR QUANTIFIZIERUNG KLEINER RNA-MOLEKÜLE
PROCEDES POUR QUANTIFIER DE PETITES MOLECULES D'ARN

(30) Priority: 25.01.2005 US 647178 P
(43) Date of publication of application: 07.11.2007
(73) Proprietor: Rosetta Inpharmatics LLC, Seattle, WA 98109 (US)
(72) Inventor: RAYMOND, Christopher, K., Seattle, Washington 98115 (US)
(74) Representative: Brady, Paul Andrew
(86) International application number: PCT/US2006/002591
(87) International publication number: WO 2006/081284

(56) References cited:
- WO-A-02/057479
- WO-A-2005/098029
- WO-A-2005/116250
- US-A1- 2003 186 288
- GRAD Y ET AL: "COMPUTATIONAL AND EXPERIMENTAL IDENTIFICATION OF C. ELEGANS MICRORNAS" MOLECULAR CELL, CELL PRESS, CAMBRIDGE, MA, US, vol. 11, no. 5, 1 May 2003 (2003-05-01), pages 1253-1263, XP009032050 ISSN: 1097-2765
- SCHMITTGEN THOMAS D ET AL: "A high-throughput method to monitor the expression of microRNA precursors." NUCLEIC ACIDS RESEARCH. 2004, vol. 32, no. 4, 2004, page e43, XP002364907 ISSN: 1362-4962
- LIU CHANG-GONG ET AL: "An oligonucleotide microchip for genome-wide microRNA profiling in human and mouse tissues" PROCEEDINGS OF THE NATIONAL ACADEMY OF SCIENCES OF USA, NATIONAL ACADEMY OF SCIENCE, WASHINGTON, DC, US, vol. 101, no. 26, 29 June 2004 (2004-06-29), pages 9740-9744, XP002364908 ISSN: 0027-8424
- SCHOENIKE BARRY ET AL: "Quantitative sense-specific determination of murine coronavirus RNA by reverse transcription polymerase chain reaction" JOURNAL OF VIROLOGICAL METHODS, vol. 78, no. 1-2, March 1999 (1999-03), pages 35-49, XP002392494 ISSN: 0166-0934
- GRIFFITH-JONES, S.: "The microRNA Registry" NUCLEIC ACIDS RESEARCH, vol. 32, 2004, pages D109-D111, XP002392427 cited in the application
- CHEN ET AL: "Real-time PCR: advancing RNA interference and microRNA studies" PHARMACEUTICAL DISCOVERY, ADVANSTAR COMMUNICATIONS, INC., DULUTH, MN, US, 1 May 2005 (2005-05-01), XP002364909 ISSN: 1554-2068

## Description

### FIELD OF THE INVENTION

The present invention relates to methods of amplifying and quantitating small RNA molecules.

### BACKGROUND OF THE INVENTION

RNA interference (RNAi) is an evolutionarily conserved process that functions to inhibit gene expression (Bernstein et al. (2001), Nature 409:363-6; Dykxhoorn et al. (2003) Nat. Rev. Mol. Cell. Biol. 4:457-67). The phenomenon of RNAi was first described in *Caenorhabditis elegans,* where injection of double-stranded RNA (dsRNA) led to efficient sequence-specific gene silencing of the mRNA that was complementary to the dsRNA (Fire et al. (1998) Nature 391:806-11). RNAi has also been described in plants as a phenomenon called post-transcriptional gene silencing (PTGS), which is likely used as a viral defense mechanism (Jorgensen (1990) Trends Biotechnol. 8:340-4; Brigneti et al. (1998) EMBO J. 17:6739-46; Hamilton & Baulcombe (1999) Science 286:950-2).

An early indication that the molecules that regulate PTGS were short RNAs processed from longer dsRNA was the identification of short 21 to 22 nucleotide dsRNA derived from the longer dsRNA in plants (Hamilton & Baulcombe (1999) Science 286:950-2). This observation was repeated in *Drosophila* embryo extracts where long dsRNA was found processed into 21-25 nucleotide short RNA by the RNase III type enzyme, Dicer (Elbashir et al. (2001) Nature 411:494-8; Elbashir et al. (2001) EMBO J. 20:6877-88; Elbashir et al. (2001) Genes Dev. 15:188-200). These observations led Elbashir et al. to test if synthetic 21-25 nucleotide synthetic dsRNAs function to specifically inhibit gene expression in *Drosophila* embryo lysates and mammalian cell culture (Elbashir et al. (2001) Nature 411:494-8; Elbashir et al. (2001) EMBO J. 20:6877-88; Elbashir et al. (2001) Genes Dev. 15:188-200). They demonstrated that small interfering RNAs (siRNAs) had the ability to specifically inhibit gene expression in mammalian cell culture without induction of the interferon response.

These observations led to the development of techniques for the reduction, or elimination, of expression of specific genes in mammalian cell culture, such as plasmid-based systems that generate hairpin siRNAs (Brummelkamp et al. (2002) Science 296:550-3; Paddison et al. (2002) Genes Dev. 16:948-58; Paddison et al. (2002) Proc. Natl. Acad. Sci. U.S.A. 99:1443-8; Paul et al. 2002) Nat. Biotechnol. 20:404-8). siRNA molecules can also be introduced into cells, *in vivo*, to inhibit the expression of specific proteins (see, e.g., Soutschek, J., et al., Nature 432 (7014):173-178 (2004)).

Grad Y et al., Mol Cell. 2003 May;11 (5):1253-63 discloses the development of informatic methods to predict miRNAs in the C. elegans genome using sequence conservation and structural similarity to known miRNAs and the generation of 214 candidates, and the use of a PCR approach to verify the expression of ten additional candidates.

Schmittgen TD et al., Nucleic Acids Res. 2004 Feb 25;32(4):e43 discloses a sensitive, high throughput, real-time PCR assay to monitor the expression of miRNA precursors. Gene-specific primers and reverse transcriptase were used to convert the primary precursors and pre-miRNAs to cDNA.

Liu CG et al., Proc Natl Acad Sci U S A. 2004 Jun 29;101(26):9740-4 discloses a methodology for miRNA gene expression profiling based on the development of a microchip containing oligonucleotides corresponding to 245 miRNAs from human and mouse genomes.

US Patent Application Publication No. 2003/186288 discloses a Universal RT-coupled PCR strategy for the specific detection and accurate quantitation of mRNA. Disclosed are Universal reverse transcription (RT) primers, a specific primer mix containing the Universal RT-primers, a transcript specific forward primer and a reverse PCR primer identical to a unique tag sequence, and methods and kits thereof for avoiding the amplification of genomic DNA and/or pseudogenes.

Schoenike B et al., J Virol Methods. 1999 Mar;78(1-2):35-49 discloses that in theory, sense-specific measurement of viral RNAs may be achieved by reverse transcription polymerase chain reaction (RT-PCR) assays which utilize primers of defined polarity during the RT step. The authors note that in practice, however, it has been shown that such assays are prone to artifacts. Using murine coronavirus MHV-4 as a model, the authors describe and validate several modifications of the RT-PCR procedure which eliminate these artifacts. RT-PCR parameters which were optimized include the design of tagged primers, DNase treatment of in vitro transcribed RNA standards, specification of temperature differences between RT and PCR annealing steps, and use of competitive RNA templates for quantitative assays.

siRNA molecules have promise both as therapeutic agents for inhibiting the expression of specific proteins, and as targets for drugs that affect the activity of siRNA molecules that function to regulate the expression of proteins involved in a disease state. A first step in developing such therapeutic agents is to measure the amounts of specific siRNA molecules in different cell types within an organism, and thereby construct an "atlas" of siRNA expression within the body. Additionally, it will be useful to measure changes in the amount of specific siRNA molecules in specific cell types in response to a defined stimulus, or in a disease state.

Short RNA molecules are difficult to quantitate. For example, with respect to the use of PCR to amplify and measure the small RNA molecules, most PCR primers are longer than the small RNA molecules, and so it is difficult to design a primer that has significant overlap with a small RNA molecule, and that selectively hybridizes to the small RNA molecule at the temperatures used for primer extension and PCR amplification reactions.

### SUMMARY OF THE INVENTION

In one aspect, the present invention provides a method for producing DNA molecules from a target microRNA molecule having a length in the range of from 21 nucleotides to 25 nucleotides, the method comprising the steps of: (a) producing a first DNA molecule that is complementary to a target microRNA molecule using primer extension with an extension primer comprising a first portion that hybridizes to a portion of the microRNA molecule and a second portion that hybridizes to the complement of a universal forward primer; and (b) amplifying the first DNA molecule to produce amplified DNA molecules using the universal forward primer and a reverse primer.

In some embodiments of the method, at least one of the forward primer and the reverse primer comprise at least one locked nucleic acid molecule. It will be understood that, in the practice of the present invention, typically numerous (e.g., millions) of individual microRNA molecules are amplified in a sample (e.g., a solution of RNA molecules isolated from living cells).

In another aspect, the present invention provides a method for measuring the amount of a target microRNA having a length in the range of from 21 nucleotides to 25 nucleotides in a sample from a living organism, the method comprising the step of measuring the amount of a target microRNA molecule in a multiplicity of different cell types within a living organism, wherein the amount of the target microRNA molecule is measured by a method comprising the steps of: (1) producing a first DNA molecule complementary to the target microRNA molecule in the sample using primer extension with an extension primer comprising a first portion that hybridizes to a portion of the microRNA molecule and a second portion that hybridizes to the complement of a universal forward primer; (2) amplifying the first DNA molecule to produce amplified DNA molecules using the universal forward primer and a reverse primer; and (3) measuring the amount of the amplified DNA molecules.

In some embodiments of the method, at least one of the forward primer and the reverse primer comprise at least one locked nucleic acid molecule.

Also disclosed are nucleic acid primer molecules consisting of sequence SEQ ID NO:1 to SEQ ID NO: 499, as shown in TABLE 1, TABLE 2, TABLE 6 and TABLE 7. The primer molecules of the invention can be used as primers for detecting mammalian microRNA target molecules, using the methods of the invention described herein.

In another aspect, the present invention provides a kit for detecting at least one mammalian target microRNA comprising at least one primer set specific for the detection of a target microRNA, the primer set comprising: (1) an extension primer for producing a cDNA molecule complementary to a target microRNA, wherein the target microRNA has a length in the range of from 21 mucleotides to 25 nucleotides, the extension primer comprising a first portion that hybridizes to the target microRNA and a second portion having a hydridization sequence that hybridizes to the complement of a universal forward PCR primer; (2) a universal forward PCR primer for amplifying the cDNA molecule, comprising a sequence selected to hybridize to the hybridization sequence on the second portion of the extension primer; and (3) a reverse PCR primer for amplifying the cDNA molecule, comprising a sequence selected to hybridize to a portion of the cDNA molecule.

The extension primer comprises a first portion that hybridizes to the target microRNA molecule and a second portion that includes a hybridization sequence for a universal forward PCR primer. The reverse PCR primer comprises a sequence selected to hybridize to a portion of the cDNA molecule. In some embodiments of the kit, at least one of the universal forward and reverse primers include at least one locked nucleic acid molecule. The kits of the invention may be used to practice various embodiments of the methods of the invention.

The present invention is useful, for example, for quantitating specific microRNA molecules within different types of cells in a living organism, or, for example, for measuring changes in the amount of specific microRNAs in living cells in response to a stimulus (e.g., in response to administration of a drug).

### BRIEF DESCRIPTION OF THE DRAWINGS

The foregoing aspects and many of the attendant advantages of this invention will become more readily appreciated as the same become better understood by reference to the following detailed description, when taken in conjunction with the accompanying drawings, wherein:
FIGURE 1 shows a flow chart of a representative method of the present invention;
FIGURE 2 graphically illustrates the standard curves for assays specific for the detection of microRNA targets miR-95 and miR-424 as described in EXAMPLE 3;
FIGURE 3A is a histogram plot showing the expression profile of miR-1 across a panel of total RNA isolated from twelve tissues as described in EXAMPLE 5;
FIGURE 3B is a histogram plot showing the expression profile of miR-124 across a panel of total RNA isolated from twelve tissues as described in EXAMPLE 5; and
FIGURE 3C is a histogram plot showing the expression profile of miR-150 across a panel of total RNA isolated from twelve tissues as described in EXAMPLE 5.

### DETAILED DESCRIPTION OF THE PREFERRED EMBODIMENT

In accordance with the foregoing, in one aspect, the present invention provides methods for amplifying a microRNA molecule to produce cDNA molecules. The methods include the steps of: (a) using primer extension to make a DNA molecule that is complementary to a target microRNA molecule; and (b) using a universal forward primer and a reverse primer to amplify the DNA molecule to produce amplified DNA molecules. In some embodiments of the method, at least one of the universal forward primer and the reverse primer comprises at least one locked nucleic acid molecule.

As used herein, the term "locked nucleic acid molecule" (abbreviated as LNA molecule) refers to a nucleic acid molecule that includes a 2'-O,4'-C-methylene-β-D-ribofuranosyl moiety. Exemplary 2'-O,4'-C-methylene-β-D-ribofuranosyl moieties, and exemplary LNAs including such moieties, are described, for example, in Petersen, M. and Wengel, J., Trends in Biotechnology 21(2):74-81 (2003).

As used herein, the term "microRNA" refers to an RNA molecule that has a length in the range of from 21 nucleotides to 25 nucleotides. Some microRNA molecules (e.g., siRNA molecules) function in living cells to regulate gene expression.

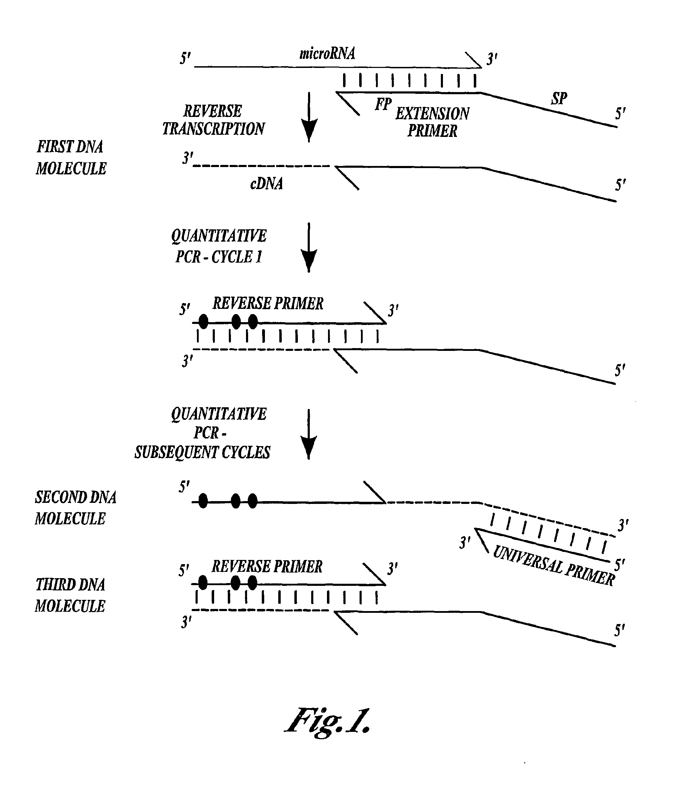

Representative method of the invention. FIGURE 1 shows a flowchart of a representative method of the present invention. In the method represented in FIGURE 1, a microRNA is the template for synthesis of a complementary first DNA molecule. The synthesis of the first DNA molecule is primed by an extension primer, and so the first DNA molecule includes the extension primer and newly synthesized DNA (represented by a dotted line in FIGURE 1). The synthesis of DNA is catalyzed by reverse transcriptase.

The extension primer includes a first portion (abbreviated as FP in FIGURE 1) and a second portion (abbreviated as SP in FIGURE 1). The first portion hybridizes to the microRNA target template, and the second portion includes a nucleic acid sequence that hybridizes with a universal forward primer, as described *infra*.

A quantitative polymerase chain reaction is used to make a second DNA molecule that is complementary to the first DNA molecule. The synthesis of the second DNA molecule is primed by the reverse primer that has a sequence that is selected to specifically hybridize to a portion of the target first DNA molecule. Thus, the reverse primer does not hybridize to nucleic acid molecules other than the first DNA molecule. The reverse primer may optionally include at least one LNA molecule located within the portion of the reverse primer that does not overlap with the extension primer. In FIGURE 1, the LNA molecules are represented by shaded ovals.

A universal forward primer hybridizes to the 3' end of the second DNA molecule and primes synthesis of a third DNA molecule. It will be understood that, although a single microRNA molecule, single first DNA molecule, single second DNA molecule, single third DNA molecule and single extension, forward and reverse primers are shown in FIGURE 1, typically the practice of the present invention uses reaction mixtures that include numerous copies (e.g., millions of copies) of each of the foregoing nucleic acid molecules.

The steps of the methods of the present invention are now considered in more detail.

Preparation of microRNA molecules useful as templates. microRNA molecules useful as templates in the methods of the invention can be isolated from any organism (e.g., eukaryote, such as a mammal) or part thereof, including organs, tissues, and/or individual cells (including cultured cells). Any suitable RNA preparation that includes microRNAs can be used, such as total cellular RNA.

RNA may be isolated from cells by procedures that involve lysis of the cells and denaturation of the proteins contained therein. Cells of interest include wild-type cells, drug-exposed wild-type cells, modified cells, and drug-exposed modified cells.

Additional steps may be employed to remove some or all of the DNA. Cell lysis may be accomplished with a nonionic detergent, followed by microcentrifugation to remove the nuclei and hence the bulk of the cellular DNA. In one embodiment, RNA is extracted from cells of the various types of interest using guanidinium thiocyanate lysis followed by CsCl centrifugation to separate the RNA from DNA (see, Chirgwin et al., 1979, Biochemistry 18:5294-5299). Separation of RNA from DNA can also be accomplished by organic extraction, for example, with hot phenol or phenol/chloroform/isoamyl alcohol.

If desired, RNase inhibitors may be added to the lysis buffer. Likewise, for certain cell types, it may be desirable to add a protein denaturation/digestion step to the protocol.

The sample of RNA can comprise a multiplicity of different microRNA molecules, each different microRNA molecule having a different nucleotide sequence. In a specific embodiment, the microRNA molecules in the RNA sample comprise at least 100 different nucleotide sequences. In other embodiments, the microRNA molecules of the RNA sample comprise at least 500, 1,000, 5,000, 10,000, 20,000, 30,000, 40,000, 50,000, 60,000, 70,000, 80,000 90,000, or 100,000 different nucleotide sequences.

The methods of the invention may be used to detect the presence of any microRNA. For example, the methods of the invention can be used to detect one or more of the microRNA targets described in a database such as "the miRBase sequence database" as described in Griffith-Jones et al. (2004), Nucleic Acids Research 32:D109-D111, and Griffith-Jones et al. (2006), Nucleic Acids Research 34: D140-D144, which is publically accessible on the World Wide Web at the Wellcome Trust Sanger Institute website at http://microrna.sanger.ac.uk/sequences/. A list of exemplary microRNA targets is also described in the following references: Lagos-Quintana et al., Curr. Biol. 12(9):735-9 (2002).

Synthesis of DNA molecules using microRNA molecules as templates. In the practice of the methods of the invention, first DNA molecules are synthesized that are complementary to the microRNA target molecules, and that are composed of an extension primer and newly synthesized DNA (wherein the extension primer primes the synthesis of the newly synthesized DNA). Individual first DNA molecules can be complementary to a whole microRNA target molecule, or to a portion thereof; although typically an individual first DNA molecule is complementary to a whole microRNA target molecule. Thus, in the practice of the methods of the invention, a population of first DNA molecules is synthesized that includes individual DNA molecules that are each complementary to all, or to a portion, of a target microRNA molecule.

The synthesis of the first DNA molecules is catalyzed by reverse transcriptase. Any reverse transcriptase molecule can be used to synthesize the first DNA molecules, such as those derived from Moloney murine leukemia virus (MMLV-RT), avian myeloblastosis virus (AMV-RT), bovine leukemia virus (BLV-RT), Rous sarcoma virus (RSV) and human immunodeficiency virus (HIV-RT). A reverse transcriptase lacking RNaseH activity (e.g., SUPERSCRIPT III^{™} sold by Invitrogen, 1600 Faraday Avenue, PO Box 6482, Carlsbad, California 92008) is preferred in order to minimize the amount of double-stranded cDNA synthesized at this stage. The reverse transcriptase molecule should also preferably be thermostable so that the DNA synthesis reaction can be conducted at as high a temperature as possible, while still permitting hybridization of primer to the microRNA target molecules.

Priming the synthesis of the first DNA molecules. The synthesis of the first DNA molecules is primed using an extension primer. Typically, the length of the extension primer is in the range of from 10 nucleotides to 100 nucleotides, such as 20 to 35 nucleotides. The nucleic acid sequence of the extension primer is incorporated into the sequence of each, synthesized, DNA molecule. The extension primer includes a first portion that hybridizes to a portion of the microRNA molecule. Typically the first portion of the extension primer includes the 3'-end of the extension primer. The first portion of the extension primer typically has a length in the range of from 6 nucleotides to 20 nucleotides, such as from 10 nucleotides to 12 nucleotides. In some embodiments, the first portion of the extension primer has a length in the range of from 3 nucleotides to 25 nucleotides.

The extension primer also includes a second portion that typically has a length of from 18 to 25 nucleotides. For example, the second portion of the extension primer can be 20 nucleotides long. The second portion of the extension primer is located 5' to the first portion of the extension primer. The second portion of the extension primer includes at least a portion of the hybridization site for the universal forward primer. For example, the second portion of the extension primer can include all of the hybridization site for the universal forward primer, or, for example, can include as little as a single nucleotide of the hybridization site for the universal forward primer (the remaining portion of the hybridization site for the forward primer can, for example, be located in the first portion of the extension primer). An exemplary nucleic acid sequence of a second portion of an extension primer is 5' CATGATCAGCTGGGCCAAGA 3' (SEQ ID NO:1).

Amplification of the DNA molecules. In the practice of the methods of the invention, the fist DNA molecules are enzymatically amplified using the polymerase chain reaction. A universal forward primer and a reverse primer are used to prime the polymerase chain reaction. The reverse primer includes a nucleic acid sequence that is selected to specifically hybridize to a portion of a first DNA molecule.

The reverse primer typically has a length in the range of from 10 nucleotides to 100 nucleotides. In some embodiments, the reverse primer has a length in the range of from 12 nucleotides to 20 nucleotides. The nucleotide sequence of the reverse primer is selected to hybridize to a specific target nucleotide sequence under defined hybridization conditions. The reverse primer and extension primer are both present in the PCR reaction mixture, and so the reverse primer should be sufficiently long so that the melting temperature (Tm) is at least 50°C, but should not be so long that there is extensive overlap with the extension primer which may cause the formation of "primer dimers." "Primer dimers" are formed when the reverse primer hybridizes to the extension primer, and uses the extension primer as a substrate for DNA synthesis, and the extension primer hybridizes to the reverse primer, and uses the reverse primer as a substrate for DNA synthesis. To avoid the formation of "primer dimers," typically the reverse primer and the extension primer are designed so that they do not overlap with each other by more than 6 nucleotides. If it is not possible to make a reverse primer having a Tm of at least 50°C, and wherein the reverse primer and the extension primer do not overlap by more than 6 nucleotides, then it is preferable to lengthen the reverse primer (since Tm usually increases with increasing oligonucleotide length) and decrease the length of the extension primer.

The reverse primer primes the synthesis of a second DNA molecule that is complementary to the first DNA molecule. The universal forward primer hybridizes to the portion of the second DNA molecule that is complementary to the second portion of the extension primer which is incorporated into all of the first DNA molecules. The universal forward primer primes the synthesis of third DNA molecules. The universal forward primer typically has a length in the range of from 16 nucleotides to 100 nucleotides. In some embodiments, the universal forward primer has a length in the range of from 16 nucleotides to 30 nucleotides. The universal forward primer may include at least one locked nucleic acid molecule. In some embodiments, the universal forward primer includes from 1 to 25 locked nucleic acid molecules. The nucleic acid sequence of an exemplary universal forward primer is set forth in SEQ ID NO:13.

In general, the greater the number of amplification cycles during the polymerase chain reaction, the greater the amount of amplified DNA that is obtained. On the other hand, too many amplification cycles (e.g., more than 35 amplification cycles) may result in spurious and unintended amplification of non-target double-stranded DNA. Thus, in some embodiments, a desirable number of amplification cycles is between one and 45 amplification cycles, such as from one to 25 amplification cycles, or such as from five to 15 amplification cycles, or such as ten amplification cycles.

Use of LNA molecules and selection of primer hybridization conditions: hybridization conditions are selected that promote the specific hybridization of a primer molecule to the complementary sequence on a substrate molecule. With respect to the hybridization of a 12 nucleotide first portion of an extension primer to a microRNA, it has been found that specific hybridization occurs at a temperature of 50°C. Similarly, it has been found that hybridization of a 20 nucleotide universal forward primer to a complementary DNA molecule, and hybridization of a reverse primer (having a length in the range of from 12-20 nucleotides, such as from 14-16 nucleotides) to a complementary DNA molecule occurs at a temperature of 50°C. By way of example, it is often desirable to design extension, reverse and universal forward primers that each have a hybridization temperature in the range of from 50°C to 60°C.

In some embodiments, LNA molecules can be incorporated into at least one of the extension primer, reverse primer, and universal forward primer to raise the Tm of one, or more, of the foregoing primers to at least 50°C. Incorporation of an LNA molecule into the portion of the reverse primer that hybridizes to the target first DNA molecule, but not to the extension primer, may be useful because this portion of the reverse primer is typically no more than 10 nucleotides in length. For example, the portion of the reverse primer that hybridizes to the target first DNA molecule, but not to the extension primer, may include at least one locked nucleic acid molecule (e.g., from 1 to 25 locked nucleic acid molecules). In some embodiments, two or three locked nucleic acid molecules are included within the first 8 nucleotides from the 5' end of the reverse primer.

The number of LNA residues that must be incorporated into a specific primer to raise the Tm to a desired temperature mainly depends on the length of the primer and the nucleotide composition of the primer. A tool for determining the effect on Tm of one or more LNAs in a primer is available on the Internet Web site of Exiqon, Bygstubben 9, DK-2950 Vedbaek, Denmark.

Although one or more LNAs can be included in any of the primers used in the practice of the present invention, it has been found that the efficiency of synthesis of cDNA is low if an LNA is incorporated into the extension primer. While not wishing to be bound by theory, LNAs may inhibit the activity of reverse transcriptase.

Detecting and measuring the amount of the amplified DNA molecules: the amplified DNA molecules can be detected and quantitated by the presence of detectable marker molecules, such as fluorescent molecules. For example, the amplified DNA molecules can be detected and quantitated by the presence of a dye (e.g., SYBR green) that preferentially or exclusively binds to double stranded DNA during the PCR amplification step of the methods of the present invention. For example, Molecular Probes, Inc. (29851 Willow Creek Road, Eugene, OR 97402) sells quantitative PCR reaction mixtures that include SYBR green dye. By way of further example, another dye (referred to as "BEBO") that can be used to label double stranded DNA produced during real-time PCR is described by Bengtsson, M., et al., Nucleic Acids Research 31(8):e45 (April 15, 2003). Again by way of example, a forward and/or reverse primer that includes a fluorophore and quencher can be used to prime the PCR amplification step of the methods of the present invention. The physical separation of the fluorophore and quencher that occurs after extension of the labeled primer during PCR permits the fluorophore to fluoresce, and the fluorescence can be used to measure the amount of the PCR amplification products. Examples of commercially available primers that include a fluorophore and quencher include Scorpion primers and Uniprimers, which are both sold by Molecular Probes, Inc.

Representative uses of the present invention: The present invention is useful for producing cDNA molecules from microRNA target molecules. The amount of the DNA molecules can be measured which provides a measurement of the amount of target microRNA molecules in the starting material. For example, the methods of the present invention can be used to measure the amount of specific microRNA molecules (e.g., specific siRNA molecules) in living cells. Again by way of example, the present invention can be used to measure the amount of specific microRNA molecules (e.g., specific siRNA molecules) in different cell types in a living body, thereby producing an "atlas" of the distribution of specific microRNA molecules within the body. Again by way of example, the present invention can be used to measure changes in the amount of specific microRNA molecules (e.g., specific siRNA molecules) in response to a stimulus, such as in response to treatment of a population of living cells with a drug.

Thus, in another aspect, the present invention provides methods for measuring the amount of a target microRNA in a multiplicity of different cell types within a living organism (e.g., to make a microRNA "atlas" of the organism). The methods of this aspect of the invention each include the step of measuring the amount of a target microRNA molecule in a multiplicity of different cell types within a living organism, wherein the amount of the target microRNA molecule is measured by a method comprising the steps of: (1) using primer extension to make a DNA molecule complementary to the target microRNA molecule isolated from a cell type of a living organism; (2) using a universal forward primer and a reverse primer to amplify the DNA molecule to produce amplified DNA molecules, and (3) measuring the amount of the amplified DNA molecules. In some embodiments of the methods, at least one of the forward primer and the reverse primer comprises at least one locked nucleic acid molecule. The measured amounts of amplified DNA molecules can, for example, be stored in an interrogatable database in electronic form, such as on a computer-readable medium (e.g., a floppy disc).

Also disclosed are nucleic acid primer molecules consisting of sequence SEQ ID NO:1 to SEQ ID NO: 499, as shown in TABLE 1, TABLE 2, TABLE 6 and TABLE 7. The primer molecules of the invention can be used as primers for detecting mammalian microRNA target molecules, using the methods of the invention described herein.

In another aspect, the present invention provides kits for detecting at least one mammalian target microRNA, the kits comprising one or more primer sets specific for the detection of a target microRNA, each primer set comprising (1) an extension primer for producing a cDNA molecule complementary to a target microRNA, (2) a universal forward PCR primer and (3) a reverse PCR primer for amplifying the cDNA molecule. The extension primer comprises a first portion that hybridizes to the target microRNA molecule and a second portion that includes a hybridization sequence for a universal forward PCR primer. The reverse PCR primer comprises a sequence selected to hybridize to a portion of the cDNA molecule. In some embodiments of the kits, at least one of the universal forward and reverse primers includes at least one locked nucleic acid molecule.

The extension primer, universal forward and reverse primers for inclusion in the kit may be designed to detect any mammalian target microRNA in accordance with the methods described herein. Nonlimiting examples of human target microRNA target molecules and exemplary target-specific extension primers and reverse primers are listed below in TABLE 1, TABLE 2 and TABLE 6. Nonlimiting examples of murine target microRNA target molecules and exemplary target-specific extension primers and reverse primers are listed below in TABLE 7. A nonlimiting example of a universal forward primer is set forth as SEQ ID NO: 13.

In certain embodiments, the kit includes a set of primers comprising an extension primer, reverse and universal forward primers for a selected target microRNA molecule that each have a hybridization temperature in the range of from 50°C to 60°C.

In certain embodiments, the kit includes a plurality of primer sets that may be used to detect a plurality of mammalian microRNA targets, such as two microRNA targets up to several hundred microRNA targets.

In certain embodiments, the kit comprises one or more primer sets capable of detecting at least one or more of the following human microRNA target templates: of miR-1, miR-7, miR-9*, miR-10a, miR-10b, miR-15a, miR-15b, miR-16, miR-17-3p, miR-17-5p, miR-18, miR-19a, miR-19b, miR-20, miR-21, miR-22, miR-23a, miR-23b, miR-24, miR-25, miR-26a, miR-26b, miR-27a, miR-28, miR-29a, miR-29b, miR-29c, miR-30a-5p, miR-30b, miR-30c, miR-30d, miR-30e-5p, miR-30e-3p, miR-31, miR-32, miR-33, miR-34a, miR-34b, miR-34c, miR-92, miR-93, miR-95, miR-96, miR-98, miR-99a, miR-99b, miR-100, miR-101, miR-103, miR-105, miR-106a, miR-107, miR-122, miR-122a, miR-124, miR-124, miR-124a, miR-125a, miR-125b, miR-126, miR-126*, miR-127, miR-128a, miR-128b, miR-129, miR-130a, miR-130b, miR-132, miR-133a, miR-133b, miR-134, miR-135a, miR-135b, miR-136, miR-137, miR-138, miR-139, miR-140, miR-141, miR-142-3p, miR-143, miR-144, miR-145, miR-146, miR-147, miR-148a, miR-148b, miR-149, miR-150, miR-151, miR-152, miR-153, miR-154*, miR-154, miR-155, miR-181a, miR-181b, miR-181c, miR-182*, miR-182, miR-183, miR-184, miR-185, miR-186, miR-187, miR-188, miR-189, miR-190, miR-191, miR-192, miR-193, miR-194, miR-195, miR-196a, miR-196b, miR-197, miR-198, miR-199a*, miR-199a, miR-199b, miR-200a, miR-200b, miR-200c, miR-202, miR-203, miR-204, miR-205, miR-206, miR-208, miR-210, miR-211, miR-212, miR-213, miR-213, miR-214, miR-215, miR-216, miR-217, miR-218, miR-220, miR-221, miR-222, miR-223, miR-224, miR-296, miR-299, miR-301, miR-302a*, miR-302a, miR-302b*, miR-302b, miR-302d, miR-302c*, miR-302c, miR-320, miR-323, miR-324-3p, miR-324-5p, miR-325, miR-326, miR-328, miR-330, miR-331, miR-337, miR-338, miR-339, miR-340, miR-342, miR-345, miR-346, miR-363, miR-367, miR-368, miR-370, miR-371, miR-372, miR-373*, miR-373, miR-374, miR-375, miR-376b, miR-378, miR-379, miR-380-5p, miR-380-3p, miR-381, miR-382, miR-383, miR-410, miR-412, miR-422a, miR-422b, miR-423, miR-424, miR-425, miR-429, miR-431, miR-448, miR-449, miR-450, miR-451, let7a, let7b, let7c, let7d, let7e, let7f, let7g, let7i, miR-376a, and miR-377. The sequences of the above-mentioned microRNA targets are provided in "the miRBase sequence database" as described in Griffith-Jones et al. (2004), Nucleic Acids Research 32:D109-D111, and Griffith-Jones et al. (2006), Nucleic Acids Research 34: D140-D144, which is publically accessible on the World Wide Web at the Wellcome Trust Sanger Institute website at http://microrna.sanger.ac.uk/sequences/.

Exemplary primers for use in accordance with this embodiment of the kit are provided in TABLE 1, TABLE 2 and TABLE 6 below.

In another embodiment, the kit comprises one or more primer sets capable of detecting at least one or more of the following human microRNA target templates: miR-1, miR-7, miR-10b, miR-26a, miR-26b, miR-29a, miR-30e-3p, miR-95, miR-107, miR-141, miR-143, miR-154*, miR-154, miR-155, miR-181a, miR-181b, miR-181c, miR-190, miR-193, miR-194, miR-195, miR-202, miR-206, miR-208, miR-212, miR-221, miR-222, miR-224, miR-296, miR-299, miR-302c*, miR-302c, miR-320, miR-339, miR363, miR-376b, miR379, miR410, miR412, miR424, miR429, miR431, miR449, miR451, let7a, let7b, let7c, let7d, let7e, let7f, let7g, and let7i. Exemplary primers for use in accordance with this embodiment of the kit are provided in TABLE 1, TABLE 2 and TABLE 6 below.

In another embodiment, the kit comprises at least one oligonucleotide primer selected from the group consisting of SEQ ID NO: 2 to SEQ ID NO: 493, as shown in TABLE 1, TABLE 2, TABLE 6 and TABLE 7.

In another embodiment, the kit comprises at least one oligonucleotide primer selected from the group consisting of SEQ ID NO: 47, 48, 49, 50, 55, 56, 81, 82, 83, 84, 91, 92, 103, 104, 123, 124, 145, 146, 193, 194, 197, 198, 221, 222, 223, 224, 225, 226, 227, 228, 229, 230, 239, 240, 247, 248, 253, 254, 255, 256, 257, 258, 277, 278, 285, 286, 287, 288, 293, 294, 301, 302, 309, 310, 311, 312, 315, 316, 317, 318, 319, 320, 333, 334, 335, 336, 337, 338, 359, 360, 369, 370, 389, 390, 393, 394, 405, 406, 407, 408, 415, 416, 419, 420, 421, 422, 425, 426, 429, 430, 431, 432, 433, 434, 435, 436, 437, 438, 439, 440, 441,442,443,444,461 and 462, as shown in TABLE 6.

A kit of the invention can also provide reagents for primer extension and amplification reactions. For example, in some embodiments, the kit may further include one or more of the following components: a reverse transcriptase enzyme, a DNA polymerase enzyme, a Tris buffer, a potassium salt (e.g., potassium chloride), a magnesium salt (e.g., magnesium chloride), a reducing agent (e.g., dithiothreitol), and deoxynucleoside triphosphates (dNTPs).

In various embodiments, the kit may include a detection reagent such as SYBR green dye or BEBO dye that preferentially or exclusively binds to double stranded DNA during a PCR amplification step. In other embodiments, the kit may include a forward and/or reverse primer that includes a fluorophore and quencher to measure the amount of the PCR amplification products.

The kit optionally includes instructions for using the kit in the detection and quantitation of one or more mammalian microRNA targets. The kit can also be optionally provided in a suitable housing that is preferably useful for robotic handling in a high throughput manner.

The following examples merely illustrate the best mode now contemplated for practicing the invention, but should not be construed to limit the invention.

### EXAMPLE 1

This Example describes a representative method of the invention for producing DNA molecules from microRNA target molecules.

Primer extension was conducted as follows (using InVitrogen Superscript III^{®} reverse transcriptase and following the guidelines that were provided with the enzyme). The following reaction mixture was prepared on ice:
1 µl of 10 mM dNTPs
1 µl of 2 µM extension primer
1- 5 µl of target template
4 µl of "5X cDNA buffer"
1 µl of 0.1 M DTT
1 µl of RNAse OUT
1 µl of SuperScript III^{®} enzyme
water to 20 µl

The mixture was incubated at 50°C for 30 minutes, then 85°C for 5 minutes, then cooled to room temperature and diluted 10-fold with TE (10 mM Tris, pH 7.6, 0.1 mM EDTA).

Real-time PCR was conducted using an ABI 7900 HTS detection system (Applied Biosystems, Foster City, California, U.S.A.) by monitoring SYBR® green fluorescence of double-stranded PCR amplicons as a function of PCR cycle number. A typical 10 µl PCR reaction mixture contained:
5 µl of 2X SYBR® green master mix (ABI)
0.8 µl of 10 µM universal forward primer
0.8 µl of 10 µM reverse primer
1.4 µl of water
2.0 µl of target template (10-fold diluted RT reaction).

The reaction was monitored through 40 cycles of standard "two cycle" PCR (95°C - 15 sec, 60°C - 60 sec) and the fluorescence of the PCR products was measured.

The foregoing method was successfully used in eleven primer extension PCR assays for quantitation of endogenous microRNAs present in a sample of total RNA. The DNA sequences of the extension primers, the universal forward primer sequence, and the LNA substituted reverse primers, used in these 11 assays are shown in TABLE 1.

**TABLE 1**

| Target microRNA | Primer number | Primer Name | DNA sequence (5' to 3') | SEQ ID NO |
|---|---|---|---|---|
| gene-specific extension primers¹ | | | | |
| humanb let7a | 357 | let7aP4 | *CATGATCAGCTGGGCCAAGA*AACTATACAACCT | 2 |
| human miR-1 | 337 | miR1P5 | *CATGATCAGCTGGGCCAAGA*TACATACTTCT | 3 |
| human miR-15a | 344 | miR15aP3 | *CATGATCAGCTGGGCCAAGA*CACAAACCATTATG | 4 |
| human miR-16 | 351 | miR16P2 | *CATGATCAGCTGGGCCAAGA*CGCCAATATTTACGT | 5 |
| human miR-21 | 342 | miR21P6 | *CATGATCAGCTGGGCCAAGA*TCAACATCAGT | 6 |
| human miR-24 | 350 | miR24P5 | *CATGATCAGCTGGGCCAAGAC*TGTTCCTGCTG | 7 |
| human miR-122 | 222 | 122-E5F | *CATGATCAGCTGGGCCAAGA*ACAAACACCATTGTCA | 8 |
| human miR-124 | 226 | 124-E5F | *CATGATCAGCTGGGCCAAGA*TGGCATTCACCGCGTG | 9 |
| human miR-143 | 362 | miR143P5 | *CATGATCAGCTGGGCCAAGA*TGAGCTACAGTG | 10 |
| human miR-145 | 305 | miR145P2 | *CATGATCAGCTGGGCCAAGA*AAGGGATTCCTGGGAA | 11 |
| human miR-155 | 367 | miR155P3 | *CATGATCAGCTGGGCCAAGA*CCCCTATCACGAT | 12 |
| ¹- Universal forward primer binding sites are shown in italics. The overlap with the RNA-specific reverse primers are underlined. | | | | |
| universal forward primer | | | | |
| | 230 | E5F | CATGATCAGCTGGGCCAAGA | 13 |

| RNA species-specific reverse primers² | | | | |
|---|---|---|---|---|
| human let7a | 290 | miRlet7a-1,2,3R | TG+AGGT+AGTAGGTTG | 14 |
| human miR-1 | 285 | miR1-1,2R | TG+GAA+TG+TAAAGAAGTA | 15 |
| human miR-15a | 287 | miR15aR | TAG+CAG+CACATAATG | 16 |
| human miR-16 | 289 | miR16-1,2R | T+AGC+AGCACGTAAA | 17 |
| human miR-21 | 286 | miR21R | T+AG+CT+TATCAGACTGAT | 18 |
| human miR-24 | 288 | miR24-1,2R | TGG+CTCAGTTCAGC | 19 |
| human miR-122 | 234 | 122LNAR | T+G+GAG+TGTGACAA | 20 |
| human miR-124 | 235 | 124LNAR | T+TAA+GGCACGCG | 21 |
| human miR-143 | 291 | miR143R | TG+AGA+TGAAGCACTG | 22 |
| human miR-145 | 314 | miR145R2 | GT+CCAGTTTTCCCA | 23 |
| human miR-155 | 293 | miR155R | T+TAA+TG+CTAATCGTGA | 24 |
| ² - LNA molecules are preceded by a "+". Region of overlap of the reverse primers with the corresponding extension primers are underlined. | | | | |

The assay was capable of detecting microRNA in a concentration range of from 2 nM to 20 fM. The assays were linear at least up to a concentration of 2 nM of synthetic microRNA (>1,000,000 copies/cell).

### EXAMPLE 2

This Example describes the evaluation of the minimum sequence requirements for efficient primer-extension mediated cDNA synthesis using a series of extension primers for microRNA assays having gene specific regions that range in length from 12 to 3 base pairs.

Primer Extension Reactions: Primer extension was conducted using the target molecules miR-195 and miR-215 as follows. The target templates miR-195 and miR-215 were diluted to 1nM RNA (100,000 copies/cell) in TE zero plus 100ng/µl total yeast RNA. A no template control (NTC) was prepared with TE zero plus 100ng/µl total yeast RNA.

The reverse transcriptase reactions were carried out as follows (using InVitrogen SuperScript III^{®} reverse transcriptase and following the guidelines that were provided with the enzyme) using a series of extension primers for miR-195 (SEQ ID NO: 25-34) and a series of extension primers for miR-215 (SEQ ID NO: 35-44) the sequences of which are shown below in TABLE 2.

The following reaction mixtures were prepared on ice:

### Set 1: No Template Control

37.5µl water
12.5µl of 10mM dNTPs
12.5µl 0.1 mM DTT
50µl of "5XcDNA buffer"
12.5µl RNAse OUT
12.5µl Superscript III^{®} reverse transcriptase enzyme
12.5µl 1µg/µl Hela cell total RNA (Ambion)
plus 50µl of 2µM extension primer
plus 50µl TEzero + yeast RNA

### Set 2: Spike-in Template

37.5µl water
12.5µl of 10mM dNTPs
12.5µl 0.1 mM DTT
50µl of "5X cDNA buffer"
12.5µl RNAse OUT
12.5µl Superscript III^{®} reverse transcriptase enzyme (InVitrogen)
12.5µl 1µg/µl Hela cell total RNA (Ambion)
plus 50µl of 2µM extension primer
plus 50µl 1 nM RNA target template (miR-195 or miR-215) serially diluted in 10-fold increments

The reactions were incubated at 50°C for 30 minutes, then 85°C for 5 minutes, and cooled to 4C and diluted 10-fold with TE (10mM Tris, pH 7.6, 0.1 mM EDTA).

Quantitative Real-Time PCR reactions: Following reverse transcription, quadruplicate measurements of cDNA were made by quantitative real-time (qPCR) using an ABI 7900 HTS detection system (Applied Biosystems, Foster City, California, U.S.A.) by monitoring SYBR® green fluorescence of double-stranded PCR amplicons as a function of PCR cycle number. The following reaction mixture was prepared:
5µl of 2X SYBR green master mix (ABI)
0.8µl of 10µM universal forward primer (SEQ ID NO: 13)
0.8µl of 10µM reverse primer (miR-195RP:SEQ ID NO: 45 or miR215RP: SEQ ID NO: 46)
1.4µl of water
2.0µl of target template (10-fold diluted miR-195 or miR-215 RT reaction)

Quantitative real-time PCR was performed for each sample in quadruplicate, using the manufacturer's recommended conditions. The reactions were monitored through 40 cycles of standard "two cycle" PCR (95°C - 15 sec, 60°C - 60 sec) and the fluorescence of the PCR products were measured and disassociation curves were generated. The DNA sequences of the extension primers, the universal forward primer sequence, and the LNA substituted reverse primers, used in the miR-195 and miR-215 assays are shown below in TABLE 2. The assay results for miR-195 are shown below in TABLE 3 and the assay results for miR-215 are shown below in TABLE 4.

**TABLE 2**

| Target microRNA | Primer number | Primer Name | DNA sequence (5' to 3') | SEQ ID NO: |
|---|---|---|---|---|
| gene-specific extension primers¹ | | | | |
| miR-195 | 646 | mir195-GS1 | *CATGATCAGCTGGGCCAAGA*GCCAATATTTCT | 25 |
| miR-195 | 647 | mir195-GS2 | *CATGATCAGCTGGGCCAAGA*GCCAATATTTC | 26 |
| miR-195 | 648 | mir195-GS3 | *CATGATCAGCTGGGCCAAGA*GCCAATATTT | 27 |
| miR-195 | 649 | mir195-GS4 | *CATGATCAGCTGGGCCAAGA*GCCAATATT | 28 |
| miR-195 | 650 | mir195-GS5 | *CATGATCAGCTGGGCCAAGA*GCCAATAT | 29 |
| miR-195 | 651 | mir195-GS6 | *CATGATCAGCTGGGCCAAGA*GCCAATA | 30 |
| miR-195 | 652 | mir195-GS7 | *CATGATCAGCTGGGCCAAGA*GCCAAT | 31 |
| miR-195 | 653 | mir195-GS8 | *CATGATCAGCTGGGCCAAGA*GCCAA | 32 |
| miR-195 | 654 | mir195-GS9 | *CATGATCAGCTGGGCCAAGA*GCCA | 33 |
| miR-195 | 655 | mir195-GS10 | *CATGATCAGCTGGGCCAAGA*GCC | 34 |
| miR-215 | 656 | mir215-GS1 | *CATGATCAGCTGGGCCAAGA*GTCTGTCAATTC | 35 |
| miR-215 | 657 | mir215-GS2 | *CATGATCAGCTGGGCCAAGA*GTCTGTCAATT | 36 |
| miR-215 | 658 | mir215-GS3 | *CATGATCAGCTGGGCCAAGA*GTCTGTCAAT | 37 |
| miR-215 | 659 | mir215-GS4 | *CATGATCAGCTGGGCCAAGA*GTCTGTCAA | 38 |
| miR-215 | 660 | mir215-GS5 | *CATGATCAGCTGGGCCAAGA*GTCTGTCA | 39 |
| miR-215 | 661 | mir215-GS6 | *CATGATCAGCTGGGCCAAGA*GTCTGTC | 40 |
| miR-215 | 662 | mir215-GS7 | *CATGATCAGCTGGGCCAAGA*GTCTGT | 41 |
| miR-215 | 663 | mir215-GS8 | *CATGATCAGCTGGGCCAAGA*GTCTG | 42 |
| miR-215 | 664 | mir215-GS9 | *CATGATCAGCTGGGCCAAGA*GTCT | 43 |
| miR-215 | 665 | mir215-GS10 | *CATGATCAGCTGGGCCAAGA*GTC | 44 |
| ¹ - Universal forward primer binding sites are shown in italics. | | | | |

| RNA species-specific reverse primers² | | | | |
|---|---|---|---|---|
| miR-195 | 442 | mir195RP | T+AGC+AGCACAGAAAT | 45 |
| miR-215 | 446 | mir215RP | A+T+GA+CCTATGAATTG | 46 |
| ² - The "+" symbol precedes the LNA molecules. | | | | |

### Results:

The sensitivity of each assay was measured by the cycle threshold (Ct) value which is defined as the cycle count at which fluorescence was detected in an assay containing microRNA target template. The lower this Ct value (e.g. the fewer number of cycles), the more sensitive was the assay. For microRNA samples, it was generally observed that while samples that contain template and no template controls both eventually cross the detection threshold, the samples with template do so at a much lower cycle number. The ΔCt value is the difference between the number of cycles (Ct) between template containing samples and no template controls, and serves as a measure of the dynamic range of the assay. Assays with a high dynamic range allow measurements of very low microRNA copy numbers. Accordingly, desirable characteristics of a microRNA detection assay include high sensitivity (low Ct value) and broad dynamic range (ΔCt ≥ 12) between the signal of a sample containing target template and a no template background control sample.

The results of the miR195 and miR215 assays using extension primers having a gene specific portion ranging in size from 12 nucleotides to 3 nucleotides are shown below in TABLE 3 and TABLE 4, respectively. The results of these experiments unexpectedly demonstrate that gene-specific priming sequences as short as 3 nucleotides exhibit template specific priming. For both the miR-195 assay sets (shown in TABLE 3) and the miR-215 assay sets (shown in TABLE 4), the results demonstrate that the dynamic range (ΔCt) for both sets of assays are fairly consistent for extension primers having gene specific regions that are greater or equal to 8 nucleotides in length. The dynamic range of the assay (ΔCt) begins to decrease for extension primers having gene specific regions below 8 nucleotides, with a reduction in assay specificity below 7 nucleotides in the miR-195 assays, and below 6 nucleotides in the miR-215 assays. A melting point analysis of the miR-215 samples demonstrated that even at 3 nucleotides, there is specific PCR product present in the plus template samples (data not shown). Taken together, these data demonstrate that the gene specific region of extension primers is ideally ≥ 8 nucleotides, but can be as short as 3 nucleotides in length.

**TABLE 3: miR195 Assay Results**

| GS Primer Length | Ct: No Template Control | Ct: Plus Template | Δ Ct |
|---|---|---|---|
| 12 | 34.83 | 20.00 | 14.82 |
| 12 | 34.19 | 19.9 | 14.3 |
| 11 1 | 40.0 | 19.8 | 20.2 |
| 10 | 36.45 | 21.2 | 15.2 |
| 9 | 36.40 | 22.2 | 14.2 |
| 8 | 40.0 | 23.73 | 16.27 |
| 7 | 36.70 | 25.96 | 10.73 |
| 6 | 30.95 | 26.58 | 4.37 |
| 5 | 30.98 | 31.71 | -0.732 |
| 4 | 32.92 | 33.28 | -0.364 |
| 3 | 35.98 | 35.38 | -0.605 |
| Ct=the cycle count where the fluorescence exceeds the threshold of detection. ΔCt = the difference between the Ct value with template and no template. | | | |

**TABLE 4: miR215 Assay Results**

| GS Primer Length | Ct: No Template Control | Ct: Plus Template | Δ Ct |
|---|---|---|---|
| 12 | 33.4 | 13.57 | 19.83 |
| 12 | 33.93 | 14.15 | 19.77 |
| 11 1 | 35.51 | 15.76 | 19.75 |
| 10 | 35.33 | 15.49 | 19.84 |
| 9 | 36.02 | 16.84 | 19.18 |
| 8 | 35.79 | 17.07 | 18.72 |
| 7 | 32.29 | 17.58 | 14.71 |
| 6 | 34.38 | 20.62 | 13.75 |
| 5 | 34.41 | 28.65 | 5.75 |
| 4 | 36.36 | 33.92 | 2.44 |
| 3 | 35.09 | 33.38 | 1.70 |
| Ct=the cycle count where the fluorescence exceeds the threshold of detection. ΔCt = the difference between the Ct value with template and no template. | | | |

### EXAMPLE 3

This Example describes assays and primer sets designed for quantitative analysis of human microRNA expression patterns.

### Primer Design:

*microRNA target templates*: the sequence of the target templates as described herein are publically available accessible on the World Wide Web at the Wellcome Trust Sanger Institute website in the "miRBase sequence database" as described in Griffith-Jones et al. (2004), Nucleic Acids Research 32:D109-D111 and and Griffith-Jones et al. (2006), Nucleic Acids Research 34: D140-D144.

*Extension primers*: gene specific primers for primer extension of a microRNA to form a cDNA followed by quantitative PCR (qPCR) amplification were designed to (1) convert the RNA template into cDNA; (2) to introduce a "universal" PCR binding site (SEQ ID NO:1) to one end of the cDNA molecule; and (3) to extend the length of the cDNA to facilitate subsequent monitoring by qPCR.

*Reverse primers*: unmodified reverse primers and locked nucleic acid (LNA) containing reverse primers (RP) were designed to quantify the primer-extended, full length cDNA in combination with a generic universal forward primer (SEQ ID NO:13). For the locked nucleic acid containing reverse primers, two or three LNA modified bases were substituted within the first 8 nucleotides from the 5' end of the reverse primer oligonucleotide, as shown below in the exemplary reverse primer sequences provided in TABLE 6. The LNA base substitutions were selected to raise the predicted Tm of the primer by the highest amount, and the final predicted Tm of the selected primers were specified to be preferably less than or equal to 55°C.

An example describing an assay utilizing an exemplary set of primers the detection of miR-95 and miR-424 is described below.

Primer Extension Reactions: primer extension was conducted using DNA templates corresponding to miR-95 and miR-424 as follows. The DNA templates were diluted to 0 nM, 1 nM, 100 pM, 10 pM and 1 pM dilutions in TE zero (10 mM Tris pH7.6, 0.1 mM EDTA) plus 100ng/µl yeast total RNA (Ambion, Austin TX).

The reverse transcriptase reactions were carried out using the following primers:
Extension primers: (diluted to 500 nM)
miR-95GSP CATGATCAGCTGGGCCAAGATGCTCAATAA (SEQ ID NO: 123)
miR-424GSP CATGATCAGCTGGGCCAAGATTCAAAACAT (SEQ ID NO:415)
Reverse primers: (diluted to 10 mM).
miR-95_RP4 TT+CAAC+GGGTATTTATTGA (SEQ ID NO: 124)
miR-424RP2 C+AG+CAGCAATTCATGTTTT (SEQ ID NO: 416)

### Reverse Transcription (per reaction):

2µl water
2µl of "5X cDNA buffer" (InVitrogen, Carlsbad, CA)
0.5µl of 0.1 mM DTT (InVitrogen, Carlsbad, CA)
0.5µl of 10 mM dNTPs (InVitrogen, Carlsbad, CA)
0.5µl RNAse OUT (InVitrogen, Carlsbad, CA)
0.5µl Superscript III^{®} reverse transcriptase enzyme (InVitrogen, Carlsbad, CA)
2µl of extension primer plus 2µl of template dilution.

The reactions were mixed and incubated at 50°C for 30 minutes, then 85°C for 5 minutes, and cooled to 4C and diluted 10-fold with TE zero.

### Quantitative Real-Time PCR Reactions: (per reaction)

5µl 2X SYBR mix (Applied Biosystems, Foster City, CA)
1.4µl water
0.8µl universal primer (CATGATCAGCTGGGCCAAGA (SEQ ID NO: 13))
2.0µl of diluted reverse transcription (RT) product from above.

Quantitative real-time PCR was performed for each sample in quadruplicate, using the manufacturer's recommended conditions. The reactions were monitored through 40 cycles of standard "two cycle" PCR (95°C - 15 sec, 60°C - 60 sec) and the fluorescence of the PCR products were measured and disassociation curves were generated. The DNA sequences of the extension primers, the universal forward primer sequence, and the LNA substituted reverse primers, used in the representative miR-95 and miR-424 assays as well as primer sets for 212 different human microRNA templates are shown below in TABLE 6. Primer sets for assays requiring extensive testing and design modification to achieve a sensitive assay with a high dynamic range are indicated in TABLE 6 with the symbol # following the primer name.

### Results:

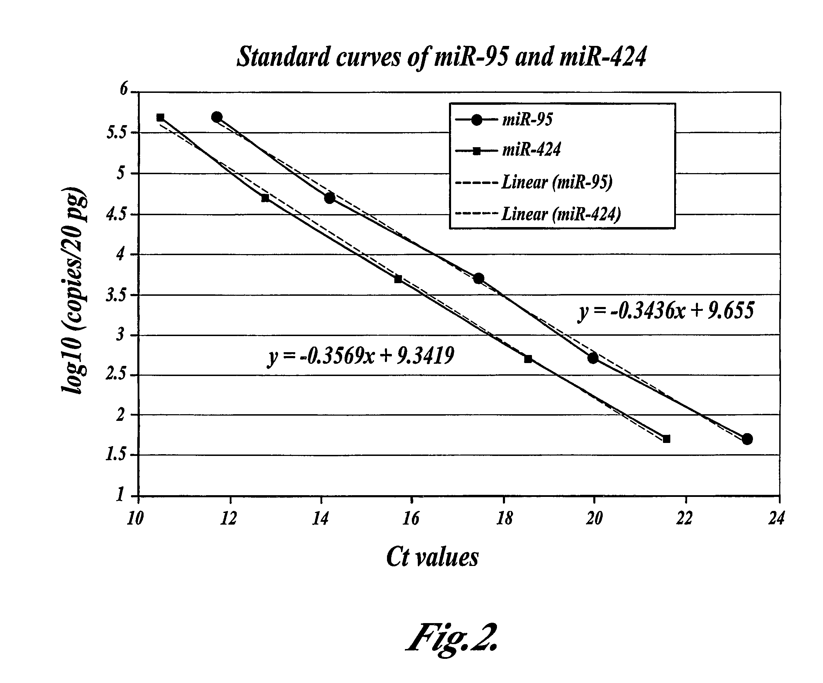

TABLE 5 shows the Ct values (averaged from four samples) from the miR-95 and miR-424 assays, which are plotted in the graph shown in FIGURE 2. The results of these assays are provided as representative examples in order to explain the significance of the assay parameters shown in TABLE 6 designated as slope (column 6), intercept (column 7) and background (column 8).

As shown in TABLE 5, the Ct value for each template at various concentrations is provided. The Ct values (x-axis) are plotted as a function of template concentration (y-axis) to generate a standard curve for each assay, as shown in FIGURE 2. The slope and intercept define the assay measurement characteristics that permit an estimation of number of copies/cell for each microRNA. For example, when the Ct values for 50µg total RNA input for the miR-95 assay are plotted, a standard curve is generated with a slope and intercept of -.03569 and 9.655, respectively. When these standard curve parameters are applied to the Ct of an unknown sample (x), they yield log 10 (copies/20pg total RNA) (y). Because the average cell yields 20 pg of total RNA, these measurements equate to copies of microRNA/cell. The background provides an estimate of the minimum copy number that can be measured in a sample and is computed by inserting the no template control (NTC) value into this equation. In this example, as shown in TABLE 6, miR-95 yields a background of 1.68 copies/20 pg at 50µg of RNA input.

As further shown in TABLE 6, reverse primers that do not contain LNA may also be used in accordance with the methods of the invention. See, e.g. SEQ ID NO: 494-499. The sensitivity and dynamic range of the assays using non-LNA containing reverse primers SEQ ID NO: 494-499, yielded similar results to the corresponding assays using LNA-containing reverse primers.

**TABLE 5**

| Ct Values (averaged from four samples) | | | | | | |
|---|---|---|---|---|---|---|
| Template concentration | 10 nM | 1 nM | 0.1 nM | 0.01 nM | 0.001 nM | NTC |
| copies/20 pg RNA (50 µg input) | 500,000 | 50,000 | 5000 | 500 | 50 | |
| Template concentration | 10 nM | 1 nM | 0.1 nM | 0.01 nM | 0.001 nM | NTC |
| copies/20 pg RNA (5 µg input) | 5,000,000 | 500,000 | 50,000 | 5000 | 500 | |
| miR-95 | 11.71572163 | 14.17978 | 17.46353 | 19.97259 | 23.33171 | 27.44383 |
| miR-424 | 10.47708975 | 12.76806 | 15.69251 | 18.53729 | 21.56897 | 23.2813 |
| log10 (copies for 50 µg input) | 5.698970004 | 4.69897 | 3.69897 | 2.69897 | 1.69897 | |

**TABLE 6: Primers to detect human microRNA target templates**

| **Human Target micro RNA** | **Extension Primer Name** | **Extension Primer Sequence** | **Reverse Primer Name** | **Reverse Primer Sequence** | **Slope** | **Intercept** | **Background RNA input 50ug 5ug** | |
|---|---|---|---|---|---|---|---|---|
| # denotes primers for assays that required extensive testing and primer design modification to achieve optimal assay results including high sensitivity and high dynamic range. | | | | | | | | |
| miR-1 | miR1GSP10^{#} | CATGATCAGCTGGGCCAAGATACATACTTC SEQ ID NO:47 | miR-1RP^{#} | T+G+GAA+TG+TAAAGAAGT SEQ ID NO:48 | -0.2758 | 8.3225 | 2.44 | 24.36 |
| miR-7 | miR-7GSP10^{#} | CATGATCAGCTGGGCCAAGACAACAAAATC SEQ ID NO:49 | miR-7_RP6^{#} | T+GGAA+GACTAGTGATTTT SEQ ID NO:50 | -0.2982 | 10.435 | 11.70 | 116.99 |
| miR-9* | miR-9*GSP | CATGATCAGCTGGGCCAAGAACTTTCGGTT SEQ ID NO:51 | miR-9*RP | TAAA+GCT+AGATAACCG SEQ ID NO:52 | -0.2405 | 8.9145 | 3.71 | 37.15 |
| miR-10a | miR-10aGSP | CATGATCAGCTGGGCCAAGACACAAATTCG SEQ ID NO:53 | miR-10aRP | T+AC+CCTGTAGATCCG SEQ ID NO:54 | -0.2755 | 8.6976 | 0.09 | 0.94 |
| miR-10b | miR-10b_GSP11^{#} | CATGATCAGCTGGGCCAAGAACAAATTCGGT SEQ ID NO:55 | miR-10b_RP2^{#} | TA+CCC+TGT+AGAACCGA SEQ ID NO:56 | -0.3505 | 8.7109 | 0.55 | 5.52 |
| miR-15a | miR-15aGSP | CATGATCAGCTGGGCCAAGACACAAACCAT SEQ ID NO:57 | miR-15aRP | T+AG+CAGCACATAATG SEQ ID NO:58 | -0.2831 | 8.4519 | 4.40 | 44.01 |
| miR-15b | miR-15bGSP2 | CATGATCAGCTGGGCCAAGATGTAAACCA SEQ ID NO:59 | miR-15bRP | T+AG+CAGCACATCAT SEQ ID NO:60 | -0.2903 | 8.4206 | 0.18 | 1.84 |
| miR-16 | miR-16GSP2 | CATGATCAGCTGGGCCAAGACGCCAATAT SEQ ID NO:61 | miR-16RP | T+AG+CAGCACGTAAA SEQ ID NO:62 | -0.2542 | 9.3689 | 1.64 | 16.42 |
| miR-17-3p | miR-17-3pGSP | CATGATCAGCTGGGCCAAGAACAAGTGCCT SEQ ID NO:63 | miR-17-3pRP | A+CT+GCAGTGAAGGC SEQ ID NO:64 | -0.2972 | 8.2625 | 1.08 | 10.78 |
| miR-17-5p | miR-17-5pGSP2 | CATGATCAGCTGGGCCAAGAACTACCTGC SEQ ID NO:65 | miR-17-5pRP | C+AA+AGTGCTTACAGTG SEQ ID NO:66 | -0.2956 | 7.9101 | 0.13 | 1.32 |
| miR-19a | miR-19aGSP2 | CATGATCAGCTGGGCCAAGATCAGTTTTG SEQ ID NO:67 | miR-19aRP | TG+TG+CAAATCTATGC SEQ ID NO:68 | -0.2984 | 9.461 | 0.02 | 0.23 |
| miR-19b | miR-19bGSP | CATGATCAGCTGGGCCAAGATCAGTTTTGC SEQ ID NO:69 | miR-19bRP | TG+TG+CAAATCCATG SEQ ID NO:70 | -0.294 | 8.1434 | 2.26 | 22.55 |
| miR-20 | miR-20GSP3 | CATGATCAGCTGGGCCAAGACTACCTGC SEQ ID NO:71 | miR-20RP | T+AA+AGTGCTTATAGTGCA SEQ ID NO:72 | -0.2979 | 7.9929 | 0.16 | 1.60 |
| miR-21 | miR-21GSP2 | CATGATCAGCTGGGCCAAGATCAACATCA SEQ ID NO: 73 | miR-21RP | T+AG+CTTATCAGACTGATG SEQ ID NO:74 | -0.2849 | 8.1624 | 1.80 | 17.99 |
| miR-23a | miR-23aGSP | CATGATCAGCTGGGCCAAGAGGAAATCCCT SEQ ID NO:75 | miR-23aRP | A+TC+ACATTGCCAGG SEQ ID NO:76 | -0.3172 | 9.4253 | 2.41 | 24.08 |
| miR-23b | miR-23bGSP | CATGATCAGCTGGGCCAAGAGGTAATCCCT SEQ ID NO:77 | miR-23bRP | A+TC+ACATTGCCAGG SEQ ID NO:78 | -0.2944 | 9.0985 | 5.39 | 53.85 |
| miR-25 | miR-25GSP | CATGATCAGCTGGGCCAAGATCAGACCGAG SEQ ID NO:79 | miR-25RP | C+AT+TGCACTTGTCTC SEQ ID NO:80 | -0.3009 | 8.2482 | 1.52 | 15.19 |
| miR-26a | miR-26aGSP9^{#} | CATGATCAGCTGGGCCAAGAGCCTATCCT SEQ ID NO:81 | miR-26aRP2# | TT+CA+AGTAATCCAGGAT SEQ ID NO:82 | -0.2807 | 8.558 | 0.26 | 2.56 |
| miR-26b | miR-26bGSP9^{#} | CATGATCAGCTGGGCCAAGAAACCTATCC SEQ ID NO:83 | miR-26bRP2# | TT+CA+AGT+AATTCAGGAT SEQ ID NO:84 | -0.2831 | 8.7885 | 0.37 | 3.67 |
| miR-27a | miR-27aGSP | CATGATCAGCTGGGCCAAGAGCGGAACTTA SEQ ID NO:85 | miR-27aRP | TT+CA+CAGTGGCTAA SEQ ID NO:86 | -0.2765 | 9.5239 | 5.15 | 51.51 |
| miR-27b | miR-27bGSP | CATGATCAGCTGGGCCAAGAGCAGAACTTA SEQ ID NO:87 | miR-27bRP | TT+CA+CAGTGGCTAA SEQ ID NO:88 | -0.28 | 9.5483 | 5.97 | 59.71 |
| miR-28 | miR-28GSP | CATGATCAGCTGGGCCAAGACTCAATAGAC SEQ ID NO:89 | miR-28RP | A+AG+GAGCTCACAGT SEQ ID NO:90 | -0.3226 | 10.071 | 7.19 | 71.87 |
| miR-29a | miR-29aGSP8^{#} | CATGATCAGCTGGGCCAAGAAACCGATT SEQ ID NO:91 | miR-29aRP2# | T+AG+CACCATCTGAAAT SEQ ID NO:92 | -0.29 | 8.8731 | 0.04 | 0.38 |
| miR-29b | miR-29bGSP2 | CATGATCAGCTGGGCCAAGAAACACTGAT SEQ ID NO:93 | miR-29bRP2 | T+AG+CACCATTTGAAATCA G SEQ ID NO:94 | -0.3162 | 9.6276 | 3.56 | 35.57 |
| miR-30a-5p | miR-30a-5pGSP | CATGATCAGCTGGGCCAAGACTTCCAGTCG SEQ ID NO:95 | miR-30a-5pRP | T+GT+AAACATCCTCGAC SEQ ID NO:96 | -0.2772 | 9.0694 | 1.92 | 19.16 |
| miR-30b | miR-30bGSP | CATGATCAGCTGGGCCAAGAAGCTGAGTGT SEQ ID NO:97 | miR-30bRP | TGT+AAA+CATCCTACACT SEQ ID NO:98 | -0.2621 | 8.5974 | 0.11 | 1.13 |
| miR-30c | miR-30cGSP | CATGATCAGCTGGGCCAAGAGCTGAGAGTG SEQ ID NO:99 | miR-30cRP | TGT+AAA+CATCCTACACT SEQ ID NO:100 | -0.2703 | 8.699 | 0.15 | 1.48 |
| miR-30d | miR-30dGSP | CATGATCAGCTGGGCCAAGACTTCCAGTCG SEQ ID NO:101 | miR-30dRP | T+GTAAA+CATCCCCG SEQ ID NO:102 | -0.2506 | 9.3875 | 0.23 | 2.31 |
| miR-30e-3p | miR-30e-3pGSP9^{#} | CATGATCAGCTGGGCCAAGAGCTGTAAAC SEQ ID NO:103 | miR-30e-3pRP5^{#} | CTTT+CAGT+CGGATGTTT SEQ ID NO:104 | -0.325 | 11.144 | 6.37 | 63.70 |
| miR-30e-5p | miR-30e-5pGSP | CATGATCAGCTGGGCCAAGATCCAGTCAAG SEQ ID NO:105 | miR-30e-5pRP | TG+TAAA+CATCCTTGAC SEQ ID NO:106 | -0.2732 | 8.1604 | 8.50 | 85.03 |
| miR-31 | miR-31GSP | CATGATCAGCTGGGCCAAGACAGCTATGCC SEQ ID NO:107 | miR-31RP | G+GC+AAGATGCTGGC SEQ ID NO:108 | -0.3068 | 8.2605 | 3.74 | 37.43 |
| miR-32 | miR-32GSP | CATGATCAGCTGGGCCAAGAGCAACTTAGT SEQ ID NO:109 | miR-32RP | TATTG+CA+CATTACTAAG SEQ ID NO:110 | -0.2785 | 8.9581 | 0.39 | 3.93 |
| miR-33 | miR-33GSP2 | CATGATCAGCTGGGCCAAGACAATGCAAC SEQ ID NO:111 | miR-33RP | G+TG+CATTGTAGTTGC SEQ ID NO:112 | -0.3031 | 8.42 | 2.81 | 28.14 |
| miR-34a | miR-34aGSP | CATGATCAGCTGGGCCAAGAAACAACCAGC SEQ ID NO:113 | miR-34aRP | T+GG+CAGTGTCTTAG SEQ ID NO:114 | -0.3062 | 9.1522 | 2.40 | 23.99 |
| miR-34b | miR-34bGSP | CATGATCAGCTGGGCCAAGACAATCAGCTA SEQ ID NO:115 | miR-34bRP | TA+GG+CAGTGTCATT SEQ ID NO:116 | -0.3208 | 9.054 | 0.04 | 0.37 |
| miR-34c | miR-34cGSP | CATGATCAGCTGGGCCAAGAGCAATCAGCT SEQ ID NO:117 | miR-34cRP | A+GG+CAGTGTAGTTA SEQ ID NO:118 | -0.2995 | 10.14 | 1.08 | 10.83 |
| miR-92 | miR-92GSP | CATGATCAGCTGGGCCAAGACAGGCCGGGA SEQ ID NO:119 | miR-92RP | T+AT+TGCACTTGTCCC SEQ ID NO:120 | -0.3012 | 8.6908 | 8.92 | 89.17 |
| miR-93 | miR-93GSP | CATGATCAGCTGGGCCAAGACTACCTGCAC SEQ ID NO:121 | miR-93RP | AA+AG+TGCTGTTCGT SEQ ID NO:122 | -0.3025 | 7.9933 | 4.63 | 46.30 |
| miR-95 | miR-95GSP^{#} | CATGATCAGCTGGGCCAAGATGCTCAATAA SEQ ID NO:123 | miR-95_RP4^{#} | TT+CAAC+GGGTATTTATTG A SEQ ID NO:124 | -0.3436 | 9.655 | 1.68 | 16.80 |
| miR-96 | miR-96GSP | CATGATCAGCTGGGCCAAGAGCAAAAATGT SEQ ID NO:125 | miR-96RP | T+TT+GGCACTAGCAC SEQ ID NO:126 | -0.2968 | 9.2611 | 0.00 | 0.05 |
| miR-98 | miR-98GSP | CATGATCAGCTGGGCCAAGAAACAATACAA SEQ ID NO:127 | miR-98RP | TGA+GGT+AGTAAGTTG SEQ ID NO:128 | -0.2797 | 9.5654 | 1.05 | 10.48 |
| miR-99a | miR-99aGSP | CATGATCAGCTGGGCCAAGACACAAGATCG SEQ ID NO:129 | miR-99aRP | A+AC+CCGTAGATCCG SEQ ID NO:130 | -0.2768 | 8.781 | 0.21 | 2.08 |
| miR-99b | miR-99bGSP | CATGATCAGCTGGGCCAAGACGCAAGGTCG SEQ ID NO:131 | miR-99bRP | C+AC+CCGTAGAACCG SEQ ID NO:132 | -0.2747 | 7.9855 | 0.25 | 2.53 |
| miR-100 | miR-100GSP | CATGATCAGCTGGGCCAAGACACAAGTTCG SEQ ID NO:133 | miR-100RP | A+AC+CCGTAGATCCG SEQ ID NO:134 | -0.2902 | 8.669 | 0.04 | 0.35 |
| miR-101 | miR-101GSP | CATGATCAGCTGGGCCAAGACTTCAGTTAT SEQ ID NO:135 | miR-101RP | TA+CAG+TACTGTGATAACT SEQ ID NO:136 | -0.3023 | 8.2976 | 0.46 | 4.63 |
| miR-103 | miR-103GSP | CATGATCAGCTGGGCCAAGATCATAGCCCT SEQ ID NO:137 | miR-103RP | A+GC+AGCATTGTACA SEQ ID NO:138 | -0.3107 | 8.5776 | 0.02 | 0.21 |
| miR-105 | miR-105GSP | CATGATCAGCTGGGCCAAGAACAGGAGTCT SEQ ID NO:139 | miR-105RP | T+CAAA+TGCTCAGACT SEQ ID NO:140 | -0.2667 | 8.9832 | 0.93 | 9.28 |
| miR-106a | miR-106aGSP | CATGATCAGCTGGGCCAAGAGCTACCTGCA SEQ ID NO:141 | miR-106aRP | AAA+AG+TGCTTACAGTG SEQ ID NO:142 | -0.3107 | 8.358 | 0.03 | 0.31 |
| miR-106b | miR-106bGSP | CATGATCAGCTGGGCCAAGAATCTGCACTG SEQ ID NO:143 | miR-106bRP | T+AAAG+TGCTGACAGT SEQ ID NO:144 | -0.2978 | 8.7838 | 0.10 | 1.04 |
| miR-107 | miR-107GSP8^{#} | CATGATCAGCTGGGCCAAGATGATAGCC SEQ ID NO:145 | miR-107RP2^{#} | A+GC+AGCATTGTACAG SEQ ID NO:146 | -0.304 | 9.1666 | 0.34 | 3.41 |
| miR-122a | miR-122aGSP | CATGATCAGCTGGGCCAAGAACAAACACCA SEQ ID NO:147 | miR-122aRP | T+GG+AGTGTGACAAT SEQ ID NO:148 | -0.3016 | 8.1479 | 0.06 | 0.58 |
| miR-124a | miR-124aGSP | CATGATCAGCTGGGCCAAGATGGCATTCAC SEQ ID NO:149 | miR-124aRP | T+TA+AGGCACGCGGT SEQ ID NO:150 | -0.3013 | 8.6906 | 0.56 | 5.63 |
| miR-125a | miR-125aGSP | CATGATCAGCTGGGCCAAGACACAGGTTAA SEQ ID NO:151 | miR-125aRP | T+CC+CTGAGACCCTT SEQ ID NO:152 | -0.2938 | 8.6754 | 0.09 | 0.91 |
| miR-125b | miR-125bGSP | CATGATCAGCTGGGCCAAGATCACAAGTTA SEQ ID NO:153 | miR-125bRP | T+CC+CTGAGACCCTA SEQ ID NO:154 | -0.283 | 8.1251 | 0.20 | 1.99 |
| miR-126 | miR-126GSP | CATGATCAGCTGGGCCAAGAGCATTATTAC SEQ ID NO:155 | miR-126RP | T+CG+TACCGTGAGTA SEQ ID NO:156 | -0.26 | 8.937 | 0.18 | 1.80 |
| miR-126* | miR-126*GSP3 | CATGATCAGCTGGGCCAAGACGCGTACC SEQ ID NO:157 | miR-126*RP | C+ATT+ATTA+CTTTTGGTA CG SEQ ID NO:158 | -0.2969 | 8.184 | 3.58 | 35.78 |
| miR-127 | miR-127GSP | CATGATCAGCTGGGCCAAGAAGCCAAGCTC SEQ ID NO:159 | miR-127RP | T+CG+GATCCGTCTGA SEQ ID NO:160 | -0.2432 | 9.1013 | 1.11 | 11.13 |
| miR-128a | miR-128aGSP | CATGATCAGCTGGGCCAAGAAAAAGAGACC SEQ ID NO:161 | miR-128aRP | T+CA+CAGTGAACCGG SEQ ID NO:162 | -0.2866 | 8.0867 | 0.16 | 1.60 |
| miR-128b | miR-128bGSP | CATGATCAGCTGGGCCAAGAGAAAGAGACC SEQ ID NO:163 | miR-128bRP | T+CA+CAGTGAACCGG SEQ ID NO:164 | -0.2923 | 8.0608 | 0.07 | 0.74 |
| miR-129 | miR-129GSP | CATGATCAGCTGGGCCAAGAGCAAGCCCAG SEQ ID NO:165 | miR-129RP | CTTTT+TG+CGGTCTG SEQ ID NO:166 | -0.2942 | 9.7731 | 0.88 | 8.85 |
| miR-130a | miR-130aGSP | CATGATCAGCTGGGCCAAGAATGCCCTTTT SEQ ID NO:167 | miR-130aRP | C+AG+TGCAATGTTAAAAG SEQ ID NO:168 | -0.2943 | 8.7465 | 1.28 | 12.78 |
| miR-130b | miR-130bGSP | CATGATCAGCTGGGCCAAGAATGCCCTTTC SEQ ID NO:169 | miR-130bRP | C+AG+TGCAATGATGA SEQ ID NO:170 | -0.2377 | 9.1403 | 3.14 | 31.44 |
| miR-132 | miR-132GSP | CATGATCAGCTGGGCCAAGACGACCATGGC SEQ ID NO:171 | miR-132RP | T+AA+CAGTCTACAGCC SEQ ID NO:172 | -0.2948 | 8.1167 | 0.11 | 1.13 |
| miR-133a | miR-133aGSP | CATGATCAGCTGGGCCAAGAACAGCTGGTT SEQ ID NO:173 | miR-133aRP | T+TG+GTCCCCTTCAA SEQ ID NO:174 | -0.295 | 9.3679 | 0.10 | 1.04 |
| miR-133b | miR-133bGSP | CATGATCAGCTGGGCCAAGATAGCTGGTTG SEQ ID NO:175 | miR-133bRP | T+TG+GTCCCCTTCAA SEQ ID NO:176 | -0.3062 | 8.3649 | 0.02 | 0.18 |
| miR-134 | miR-134GSP | CATGATCAGCTGGGCCAAGACCCTCTGGTC SEQ ID NO:177 | miR-134RP | T+GT+GACTGGTTGAC SEQ ID NO:178 | -0.2965 | 9.0483 | 0.14 | 1.39 |
| miR-135a | miR-135aGSP | CATGATCAGCTGGGCCAAGATCACATAGGA SEQ ID NO:179 | miR-135aRP | T+AT+GGCTTTTTATTCCT SEQ ID NO:180 | -0.2914 | 8.092 | 1.75 | 17.50 |
| miR-135b | miR-135bGSP | CATGATCAGCTGGGCCAAGACACATAGGAA SEQ ID NO:181 | miR-135bRP | T+AT+GGCTTTTCATTCC SEQ ID NO:182 | -0.2962 | 7.8986 | 0.05 | 0.49 |
| miR-136 | miR-136GSP | CATGATCAGCTGGGCCAAGATCCATCATCA SEQ ID NO:183 | miR-136RP | A+CT+CCATTTGTTTTGATG SEQ ID NO:184 | -0.3616 | 10.229 | 0.68 | 6.77 |
| miR-137 | miR-137GSP | CATGATCAGCTGGGCCAAGACTACGCGTAT SEQ ID NO:185 | miR-137RP | T+AT+TGCTTAAGAATACGC SEQ ID NO:186 | -0.2876 | 8.234 | 8.57 | 85.71 |
| miR-138 | miR-138GSP2 | CATGATCAGCTGGGCCAAGACGGCCTGAT SEQ ID NO:187 | miR-138RP | A+GC+TGGTGTTGTGA SEQ ID NO:188 | -0.3023 | 9.0814 | 0.22 | 2.19 |
| miR-139 | miR-139GSP | CATGATCAGCTGGGCCAAGAAGACACGTGC SEQ ID NO:189 | miR-139RP | T+CT+ACAGTGCACGT SEQ ID NO:190 | -0.2983 | 8.1141 | 6.92 | 69.21 |
| miR-140 | miR-140GSP | CATGATCAGCTGGGCCAAGACTACCATAGG SEQ ID NO:191 | miR-140RP | A+GT+GGTTTTACCCT SEQ ID NO:192 | -0.2312 | 8.3231 | 0.13 | 1.34 |
| miR-141 | miR-141GSP9^{#} | CATGATCAGCTGGGCCAAGACCATCTTTA SEQ ID NO:193 | miR-141RP2^{#} | TAA+CAC+TGTCTGGTAA SEQ ID NO:194 | -0.2805 | 9.6671 | 0.13 | 1.26 |
| miR-142-3p | miR-142-3pGSP3 | CATGATCAGCTGGGCCAAGATCCATAAA SEQ ID NO:195 | miR-142-3pRP | TGT+AG+TGTTTCCTACT SEQ ID NO:196 | -0.2976 | 8.4046 | 0.03 | 0.27 |
| miR-143 | miR-143GSP8# | CATGATCAGCTGGGCCAAGATGAGCTAC SEQ ID NO:197 | miR-143RP2# | T+GA+GATGAAGCACTG SEQ ID NO:198 | -0.3008 | 9.2675 | 0.37 | 3.71 |
| miR-144 | miR-144GSP2 | CATGATCAGCTGGGCCAAGACTAGTACAT SEQ ID NO:199 | miR-144RP | TA+CA+GTAT+AGATGATG SEQ ID NO:200 | -0.2407 | 9.4441 | 0.95 | 9.52 |
| miR-145 | miR-145GSP2 | CATGATCAGCTGGGCCAAGAAAGGGATTC SEQ ID NO:201 | miR-145RP | G+TC+CAGTTTTCCCA SEQ ID NO:202 | -0.2937 | 8.0791 | 0.39 | 3.86 |
| miR-146 | miR-146GSP3 | CATGATCAGCTGGGCCAAGAAACCCATG SEQ ID NO:203 | miR-146RP | T+GA+GAACTGAATTCCA SEQ ID NO:204 | -0.2861 | 8.8246 | 0.08 | 0.75 |
| miR-147 | miR-147GSP | CATGATCAGCTGGGCCAAGAGCAGAAGCAT SEQ ID NO:205 | miR-147RP | G+TG+TGTGGAAATGC SEQ ID NO:206 | -0.2989 | 8.8866 | 1.65 | 16.47 |
| miR-148a | miR-148aGSP2 | CATGATCAGCTGGGCCAAGAACAAAGTTC SEQ ID NO:207 | miR-148aRP2 | T+CA+GTGCACTACAGAACT SEQ ID NO:208 | -0.2928 | 9.4654 | 1.27 | 12.65 |
| miR-148b | miR-148bGSP2 | CATGATCAGCTGGGCCAAGAACAAAGTTC SEQ ID NO:209 | miR-148bRP | T+CA+GTGCATCACAG SEQ ID NO:210 | -0.2982 | 10.417 | 0.24 | 2.44 |
| miR-149 | miR-149GSP2 | CATGATCAGCTGGGCCAAGAGGAGTGAAG SEQ ID NO:211 | miR-149RP | T+CT+GGCTCCGTGTC SEQ ID NO:212 | -0.2996 | 8.3392 | 2.15 | 21.50 |
| miR-150 | miR-150GSP3 | CATGATCAGCTGGGCCAAGACACTGGTA SEQ ID NO:213 | miR-150RP | T+CT+CCCAACCCTTG SEQ ID NO:214 | -0.2943 | 8.3945 | 0.06 | 0.56 |
| miR-151 | miR-151GSP2 | CATGATCAGCTGGGCCAAGACCTCAAGGA SEQ ID NO:215 | miR-151RP | A+CT+AGACTGAAGCTC SEQ ID NO:216 | -0.2975 | 8.651 | 0.16 | 1.60 |
| miR-152 | miR-152GSP2 | CATGATCAGCTGGGCCAAGACCCAAGTTC SEQ ID NO:217 | miR-152RP | T+CA+GTGCATGACAG SEQ ID NO:218 | -0.2741 | 8.7404 | 0.33 | 3.25 |
| miR-153 | miR-153GSP2 | CATGATCAGCTGGGCCAAGATCACTTTTG SEQ ID NO:219 | miR-153RP | TTG+CAT+AGTCACAAAA SEQ ID NO:220 | -0.2723 | 9.5732 | 3.32 | 33.19 |
| miR-154* | miR-154*GSP9^{#} | CATGATCAGCTGGGCCAAGAAATAGGTCA SEQ ID NO:221 | miR-154*RP2^{#} | AATCA+TA+CACGGTTGAC SEQ ID NO:222 | -0.3056 | 8.8502 | 0.07 | 0.74 |
| miR-154 | miR-154GSP9^{#} | CATGATCAGCTGGGCCAAGACGAAGGCAA SEQ ID NO:223 | miR-154RP3^{#} | TA+GGTTA+TCCGTGTT SEQ ID NO:224 | -0.3062 | 9.3947 | 0.10 | 0.96 |
| miR-155 | miR-155GSP8^{#} | CATGATCAGCTGGGCCAAGACCCCTATC SEQ ID NO:225 | miR-155RP2^{#} | TT+AA+TGCTAATCGTGATA GG SEQ ID NO:226 | -0.3201 | 8.474 | 5.49 | 54.91 |
| miR-181a | miR-181aGSP9^{#} | CATGATCAGCTGGGCCAAGAACTCACCGA SEQ ID NO:227 | miR-181aRP2^{#} | AA+CATT+CAACGCTGTC SEQ ID NO:228 | -0.2919 | 7.968 | 1.70 | 17.05 |
| miR-181c | miR-181cGSP9^{#} | CATGATCAGCTGGGCCAAGAACTCACCGA SEQ ID NO:229 | miR-181cRP2^{#} | AA+CATT+CAACCTGTCG SEQ ID NO:230 | -0.3102 | 7.9029 | 1.08 | 10.78 |
| miR-182* | miR-182*GSP | CATGATCAGCTGGGCCAAGATAGTTGGCAA SEQ ID NO:231 | miR-182*RP | T+GG+TTCTAGACTTGC SEQ ID NO:232 | -0.2978 | 8.5876 | 4.25 | 42.47 |
| miR-182 | miR-182GSP2 | CATGATCAGCTGGGCCAAGATGTGAGTTC SEQ ID NO:233 | miR-182RP | TTT+GG+CAATGGTAG SEQ ID NO:234 | -0.2863 | 9.0854 | 1.52 | 15.20 |
| miR-183 | miR-183GSP2 | CATGATCAGCTGGGCCAAGACAGTGAATT SEQ ID NO:235 | miR-183RP | T+AT+GGCACTGGTAG SEQ ID NO:236 | -0.2774 | 9.9254 | 1.95 | 19.51 |
| miR-184 | miR-184GSP2 | CATGATCAGCTGGGCCAAGAACCCTTATC SEQ ID NO:237 | miR-184RP | T+GG+ACGGAGAACTG SEQ ID NO:238 | -0.2906 | 7.9585 | 0.05 | 0.49 |
| miR-186 | miR-186GSP9^{#} | CATGATCAGCTGGGCCAAGAAAGCCCAAA SEQ ID NO:239 | miR-186RP3^{#} | CA+AA+GAATT+CTCCTTTT GG SEQ ID NO:240 | -0.2861 | 8.6152 | 0.32 | 3.18 |
| miR-187 | miR-187GSP | CATGATCAGCTGGGCCAAGACGGCTGCAAC SEQ ID NO:241 | miR-187RP | T+CG+TGTCTTGTGTT SEQ ID NO:242 | -0.2953 | 7.9329 | 1.23 | 12.31 |
| miR-188 | miR-188GSP | CATGATCAGCTGGGCCAAGAACCCTCCACC SEQ ID NO:243 | miR-188RP | C+AT+CCCTTGCATGG SEQ ID NO:244 | -0.2925 | 8.0782 | 8.49 | 84.92 |
| miR-189 | miR-189GSP2 | CATGATCAGCTGGGCCAAGAACTGATATC SEQ ID NO:245 | miR-189RP | G+TG+CCTACTGAGCT SEQ ID NO:246 | -0.2981 | 8.8964 | 0.21 | 2.08 |
| miR-190 | miR-190GSP9^{#} | CATGATCAGCTGGGCCAAGAACCTAATAT SEQ ID NO:247 | miR-190RP4^{#} | T+GA+TA+TGTTTGATATAT TAG SEQ ID NO:248 | -0.3317 | 9.8766 | 0.43 | 4.34 |
| miR-191 | miR-191GSP2 | CATGATCAGCTGGGCCAAGAAGCTGCTTT SEQ ID NO:249 | miR-191RP2 | C+AA+CGGAATCCCAAAAG SEQ ID NO:250 | -0.299 | 9.0317 | 0.41 | 4.07 |
| miR-192 | miR-192GSP2 | CATGATCAGCTGGGCCAAGAGGCTGTCAA SEQ ID NO:251 | miR-192RP | C+TGA+CCTATGAATTGAC SEQ ID NO:252 | -0.2924 | 9.5012 | 1.10 | 10.98 |
| miR-193 | miR-193GSP9^{#} | CATGATCAGCTGGGCCAAGACTGGGACTT SEQ ID NO:253 | miR-193RP2^{#} | AA+CT+GGCCTACAAAG SEQ ID NO:254 | -0.3183 | 8.9942 | 0.17 | 1.72 |
| miR-194 | mir194GSP8^{#} | CATGATCAGCTGGGCCAAGATCCACATG SEQ ID NO:255 | mir194RP^{#} | TG+TAA+CAGCAACTCCA SEQ ID NO:256 | -0.3078 | 8.8045 | 0.37 | 3.69 |
| miR-195 | miR-195GSP9^{#} | CATGATCAGCTGGGCCAAGAGCCAATATT SEQ ID NO:257 | miR-195RP3^{#} | T+AG+CAG+CACAGAAATA SEQ ID NO:258 | -0.2955 | 10.213 | 0.76 | 7.58 |
| miR-196b | miR-196bGSP | CATGATCAGCTGGGCCAAGACCAACAACAG SEQ ID NO:259 | miR-196bRP | TA+GGT+AGTTTCCTGT SEQ ID NO:260 | -0.301 | 8.1641 | 1.47 | 14.66 |
| miR-196a | miR-196aGSP | CATGATCAGCTGGGCCAAGACCAACAACAT SEQ ID NO:261 | miR-196aRP | TA+GG+TAGTTTCATGTTG SEQ ID NO:262 | -0.2932 | 8.0448 | 8.04 | 80.37 |
| miR-197 | miR-197GSP2 | CATGATCAGCTGGGCCAAGAGCTGGGTGG SEQ ID NO:263 | miR-197RP | TT+CA+CCACCTTCTC SEQ ID NO:264 | -0.289 | 8.2822 | 0.71 | 7.10 |
| miR-198 | miR-198GSP3 | CATGATCAGCTGGGCCAAGACCTATCTC SEQ ID NO:265 | miR-198RP | G+GT+CCAGAGGGGAG SEQ ID NO:266 | -0.2986 | 8.1359 | 0.31 | 3.15 |
| miR-199a* | miR-199a*GSP2 | CATGATCAGCTGGGCCAAGAAACCAATGT SEQ ID NO:267 | miR-199a*RP | T+AC+AGTAGTCTGCAC SEQ ID NO:268 | -0.3029 | 9.0509 | 0.25 | 2.52 |
| miR-199a | miR-199aGSP2 | CATGATCAGCTGGGCCAAGAGAACAGGTA SEQ ID NO:269 | miR-199aRP | C+CC+AGTGTTCAGAC SEQ ID NO:270 | -0.3187 | 9.2268 | 0.12 | 1.16 |
| miR-199b | miR-199bGSP | CATGATCAGCTGGGCCAAGAGAACAGATAG SEQ ID NO:271 | miR-199bRP | C+CC+AGTGTTTAGAC SEQ ID NO:272 | -0.3165 | 9.3935 | 2.00 | 20.04 |
| miR-200a | miR-200aGSP2 | CATGATCAGCTGGGCCAAGAACATCGTTA SEQ ID NO:273 | miR-200aRP | TAA+CAC+TGTCTGGT SEQ ID NO:274 | -0.2754 | 9.1227 | 0.08 | 0.78 |
| miR-200b | miR-200bGSP2 | CATGATCAGCTGGGCCAAGAGTCATCATT SEQ ID NO:275 | miR-200bRP | TAATA+CTG+CCTGGTAAT SEQ ID NO:276 | -0.2935 | 8.5461 | 0.08 | 0.85 |
| miR-202 | miR-202 GSP10^{#} | CATGATCAGCTGGGCCAAGATTTTCCCATG SEQ ID NO:277 | miR-202RP^{#} | A+GA+GGTATA+GGGCAT SEQ ID NO:278 | -0.2684 | 9.056 | 0.25 | 2.48 |
| miR-203 | miR-203GSP2 | CATGATCAGCTGGGCCAAGACTAGTGGTC SEQ ID NO:279 | miR-203RP | G+TG+AAATGTTTAGGACC SEQ ID NO:280 | -0.2852 | 8.1279 | 1.60 | 16.03 |
| miR-204 | miR-204GSP2 | CATGATCAGCTGGGCCAAGAAGGCATAGG SEQ ID NO:281 | miR-204RP | T+TC+CCTTTGTCATCC SEQ ID NO:282 | -0.2925 | 8.7648 | 0.16 | 1.59 |
| miR-205 | miR-205GSP | CATGATCAGCTGGGCCAAGACAGACTCCGG SEQ ID NO:283 | miR-205RP | T+CCTT+CATTCCACC SEQ ID NO:284 | -0.304 | 8.2407 | 9.21 | 92.15 |
| miR-206 | mir206GSP7^{#} | CATGATCAGCTGGGCCAAGACCACACA SEQ ID NO:285 | miR-206RP# | T+G+GAA+TGTAAGGAAGT GT SEQ ID NO:286 | -0.2815 | 8.2206 | 0.29 | 2.86 |
| miR-208 | miR-208_GSP13^{#} | CATGATCAGCTGGGCCAAGAACAAGCTTTTTGC SEQ ID NO:287 | miR-208_RP4^{#} | ATAA+GA+CG+AGCAAAAA G SEQ ID NO:288 | -0.2072 | 7.9097 | 57.75 | 577.52 |
| miR-210 | miR-210GSP | CATGATCAGCTGGGCCAAGATCAGCCGCTG SEQ ID NO:289 | miR-210RP | C+TG+TGCGTGTGACA SEQ ID NO:290 | -0.2717 | 8.249 | 0.18 | 1.77 |
| miR-211 | miR-211GSP2 | CATGATCAGCTGGGCCAAGAAGGCGAAGG SEQ ID NO:291 | miR-211RP | T+TC+CCTTTGTCATCC SEQ ID NO:292 | -0.2926 | 8.3106 | 0.10 | 1.00 |
| miR-212 | miR-212GSP9^{#} | CATGATCAGCTGGGCCAAGAGGCCGTGAC SEQ ID NO:293 | miR-212RP2# | T+AA+CAGTCTCCAGTCA SEQ ID NO:294 | -0.2916 | 8.0745 | 0.59 | 5.86 |
| miR-213 | miR-213GSP | CATGATCAGCTGGGCCAAGAGGTACAATCA SEQ ID NO:295 | miR-213RP | A+CC+ATCGACCGTTG SEQ ID NO:296 | -0.2934 | 8.1848 | 2.96 | 29.59 |
| miR-214 | miR-214GSP | CATGATCAGCTGGGCCAAGACTGCCTGTCT SEQ ID NO:297 | miR-214RP | A+CA+GCAGGCACAGA SEQ ID NO:298 | -0.2947 | 7.82 | 0.84 | 8.44 |
| miR-215 | miR-215GSP2 | CATGATCAGCTGGGCCAAGAGTCTGTCAA SEQ ID NO:299 | miR-215RP | A+TGA+CCTATGAATTGAC SEQ ID NO:300 | -0.2932 | 8.9273 | 1.51 | 15.05 |
| miR-216 | miR-216GSP9^{#} | CATGATCAGCTGGGCCAAGACACAGTTGC SEQ ID NO:301 | mir216RP^{#} | TAA+TCT+CAGCTGGCA SEQ ID NO:302 | -0.273 | 8.5829 | 0.95 | 9.50 |
| miR-217 | miR-217GSP2 | CATGATCAGCTGGGCCAAGAATCCAATCA SEQ ID NO:303 | miR-217RP2 | T+AC+TGCATCAGGAACTGA SEQ ID NO:304 | -0.3089 | 9.6502 | 0.07 | 0.71 |
| miR-218 | miR-218GSP2 | CATGATCAGCTGGGCCAAGAACATGGTTA SEQ ID NO:305 | miR-218RP | TTG+TGCTT+GATCTAAC SEQ ID NO:306 | -0.2778 | 8.4363 | 1.00 | 10.05 |
| miR-220 | miR-220GSP | CATGATCAGCTGGGCCAAGAAAAGTGTCAG SEQ ID NO:307 | miR-220RP | C+CA+CACCGTATCTG SEQ ID NO:308 | -0.2755 | 9.0728 | 8.88 | 88.75 |
| miR-221 | miR-221GSP9^{#} | CATGATCAGCTGGGCCAAGAGAAACCCAG SEQ ID NO:309 | miR-221RP^{#} | A+GC+TACATTGTCTGC SEQ ID NO:310 | -0.2886 | 8.5743 | 0.12 | 1.17 |
| miR-222 | miR-222GSP8^{#} | CATGATCAGCTGGGCCAAGAGAGACCCA SEQ ID NO:311 | miR-222RP^{#} | A+GC+TACATCTGGCT SEQ ID NO:312 | -0.283 | 8.91 | 1.64 | 16.41 |
| miR-223 | miR-223GSP | CATGATCAGCTGGGCCAAGAGGGGTATTTG SEQ ID NO:313 | miR-223RP | TG+TC+AGTTTGTCAAA SEQ ID NO:314 | -0.2998 | 8.6669 | 0.94 | 9.44 |
| miR-224 | miR-224GSP8^{#} | CATGATCAGCTGGGCCAAGATAAACGGA SEQ ID NO:315 | miR-224RP2# | C+AAG+TCACTAGTGGTT SEQ ID NO:316 | -0.2802 | 7.5575 | 0.56 | 5.63 |
| miR-296 | miR-296GSP9^{#} | CATGATCAGCTGGGCCAAGAACAGGATTG SEQ ID NO:317 | miR-296RP2# | A+GG+GCCCCCCCTCAA SEQ ID NO:318 | -0.3178 | 8.3856 | 0.10 | 0.96 |
| miR-299 | miR-299GSP9^{#} | CATGATCAGCTGGGCCAAGAATGTATGTG SEQ ID NO:319 | miR-299RP# | T+GG+TTTACCGTCCC SEQ ID NO:320 | -0.3155 | 7.9383 | 1.30 | 12.96 |
| miR-301 | miR-301GSP | CATGATCAGCTGGGCCAAGAGCTTTGACAA SEQ ID NO:321 | miR-301RP | C+AG+TGCAATAGTATTGT SEQ ID NO:322 | -0.2839 | 8.314 | 2.55 | 25.52 |
| miR-302a* | miR-302a*GSP | CATGATCAGCTGGGCCAAGAAAAGCAAGTA SEQ ID NO:323 | miR-302a*RP | TAAA+CG+TGGATGTAC SEQ ID NO:324 | -0.2608 | 8.3921 | 0.04 | 0.41 |
| miR-302a | miR-302aGSP | CATGATCAGCTGGGCCAAGATCACCAAAAC SEQ ID NO:325 | miR-302aRP | T+AAG+TGCTTCCATGT SEQ ID NO:326 | -0.2577 | 9.6657 | 2.17 | 21.67 |
| miR-302b* | miR-302b*GSP | CATGATCAGCTGGGCCAAGAAGAAAGCACT SEQ ID NO:327 | miR-302b*RP | A+CTTTAA+CATGGAAGTG SEQ ID NO:328 | -0.2702 | 8.5153 | 0.02 | 0.24 |
| miR-302b | miR-302bGSP | CATGATCAGCTGGGCCAAGACTACTAAAAC SEQ ID NO:329 | miR-302bRP | T+AAG+TGCTTCCATGT SEQ ID NO:330 | -0.2398 | 9.1459 | 5.11 | 51.11 |
| miR-302d | miR-302dGSP | CATGATCAGCTGGGCCAAGAACACTCAAAC SEQ ID NO:331 | miR-302dRP | T+AAG+TGCTTCCATGT SEQ ID NO:332 | -0.2368 | 8.5602 | 5.98 | 59.78 |
| miR-302c* | miR-302c*_GSP9^{#} | CATGATCAGCTGGGCCAAGACAGCAGGTA SEQ ID NO:333 | miR-302c*_RP2^{#} | TT+TAA+CAT+GGGGGTACC SEQ ID NO:334 | -0.312 | 8.2904 | 0.33 | 3.28 |
| miR-302c | miR-302cGSP9# | CATGATCAGCTGGGCCAAGACCACTGAAA SEQ ID NO:335 | miR-302cRP5# | T+AAG+TGCTTCCATGTTTC A SEQ ID NO:336 | -0.2945 | 8.381 | 14.28 | 142.76 |
| miR-320 | miR-320_GSP8^{#} | CATGATCAGCTGGGCCAAGATTCGCCCT SEQ ID NO:337 | miR-320_RP3^{#} | AAAA+GCT+GGGTTGAGAG G SEQ ID NO:338 | -0.2677 | 7.8956 | 6.73 | 67.29 |
| miR-323 | miR-323GSP | CATGATCAGCTGGGCCAAGAAGAGGTCGAC SEQ ID NO:339 | miR-323RP | G+CA+CATTACACGGT SEQ ID NO:340 | -0.2878 | 8.2546 | 0.19 | 1.92 |
| miR-324-3p | miR-324-3pGSP | CATGATCAGCTGGGCCAAGACCAGCAGCAC SEQ ID NO:341 | miR-324-3pRP | C+CA+CTGCCCCAGGT SEQ ID NO:342 | -0.2698 | 8.5223 | 2.54 | 25.41 |
| miR-324-5p | miR-324-5pGSP | CATGATCAGCTGGGCCAAGAACACCAATGC SEQ ID NO:343 | miR-324-5pRP | C+GC+ATCCCCTAGGG SEQ ID NO:344 | -0.2861 | 7.6865 | 0.06 | 0.62 |
| miR-325 | miR-325GSP | CATGATCAGCTGGGCCAAGAACACTTACTG SEQ ID NO:345 | miR-325RP | C+CT+AGTAGGTGTCC SEQ ID NO:346 | -0.2976 | 8.1925 | 0.01 | 0.14 |
| miR-326 | miR-326GSP | CATGATCAGCTGGGCCAAGACTGGAGGAAG SEQ ID NO:347 | miR-326RP | C+CT+CTGGGCCCTTC SEQ ID NO:348 | -0.2806 | 7.897 | 0.59 | 5.87 |
| miR-328 | miR-328GSP | CATGATCAGCTGGGCCAAGAACGGAAGGGC SEQ ID NO:349 | miR-328RP | C+TG+GCCCTCTCTGC SEQ ID NO:350 | -0.293 | 7.929 | 3.17 | 31.69 |
| miR-330 | miR-330GSP | CATGATCAGCTGGGCCAAGATCTCTGCAGG SEQ ID NO:351 | miR-330RP | G+CA+AAGCACACGGC SEQ ID NO:352 | -0.3009 | 7.7999 | 0.13 | 1.30 |
| miR-331 | miR-331GSP | CATGATCAGCTGGGCCAAGATTCTAGGATA SEQ ID NO:353 | miR-331RP | G+CC+CCTGGGCCTAT SEQ ID NO:354 | -0.2816 | 8.1643 | 0.45 | 4.54 |
| miR-337 | miR-337GSP | CATGATCAGCTGGGCCAAGAAAAGGCATCA SEQ ID NO:355 | miR-337RP | T+CC+AGCTCCTATATG SEQ ID NO:356 | -0.2968 | 8.7313 | 0.10 | 1.02 |
| miR-338 | miR-338GSP | CATGATCAGCTGGGCCAAGATCAACAAAAT SEQ ID NO:357 | miR-338RP2 | T+CC+AGCATCAGTGATTT SEQ ID NO:358 | -0.2768 | 8.5618 | 0.52 | 5.17 |
| miR-339 | miR-339GSP9^{#} | CATGATCAGCTGGGCCAAGATGAGCTCCT SEQ ID NO:359 | miR-339RP2# | T+CC+CTGTCCTCCAGG SEQ ID NO:360 | -0.303 | 8.4873 | 0.27 | 2.72 |
| miR-340 | miR-340GSP | CATGATCAGCTGGGCCAAGAGGCTATAAAG SEQ ID NO:361 | miR-340RP | TC+CG+TCTCAGTTAC SEQ ID NO:362 | -0.2846 | 9.6673 | 0.15 | 1.45 |
| miR-342 | miR-342GSP3 | CATGATCAGCTGGGCCAAGAGACGGGTG SEQ ID NO:363 | miR-342RP | T+CT+CACACAGAAATCG SEQ ID NO:364 | -0.293 | 8.1553 | 4.69 | 46.85 |
| miR-345 | miR-345GSP | CATGATCAGCTGGGCCAAGAGCCCTGGACT SEQ ID NO:365 | miR-345RP | T+GC+TGACTCCTAGT SEQ ID NO:366 | -0.2909 | 8.468 | 0.04 | 0.40 |
| miR-346 | miR-346GSP | CATGATCAGCTGGGCCAAGAAGAGGCAGGC SEQ ID NO:367 | miR-346RP | T+GT+CTGCCCGCATG SEQ ID NO:368 | -0.2959 | 8.1958 | 0.25 | 2.54 |
| miR-363 | miR-363 GSP10^{#} | CATGATCAGCTGGGCCAAGATACAGATGGA SEQ ID NO:369 | miR-363RP^{#} | AAT+TG+CAC+GGTATCC SEQ ID NO:370 | -0.2362 | 8.9762 | 0.44 | 4.36 |
| miR-367 | miR-367GSP | CATGATCAGCTGGGCCAAGATCACCATTGC SEQ ID NO:371 | miR-367RP | AAT+TG+CACTTTAGCAAT SEQ ID NO:372 | -0.2819 | 8.6711 | 0.00 | 0.03 |
| miR-368 | miR-368GSP | CATGATCAGCTGGGCCAAGAAAACGTGGAA SEQ ID NO:373 | miR-368RP2 | A+CATAGA+GGAAATTCCA C SEQ ID NO:374 | -0.2953 | 8.0067 | 6.01 | 60.11 |
| miR-370 | miR-370GSP | CATGATCAGCTGGGCCAAGACCAGGTTCCA SEQ ID NO:375 | miR-370RP | G+CC+TGCTGGGGTGG SEQ ID NO:376 | -0.2825 | 8.3162 | 1.45 | 14.55 |
| miR-371 | miR-371GSP | CATGATCAGCTGGGCCAAGAACACTCAAAA SEQ ID NO:377 | miR-371RP | G+TG+CCGCCATCTTT SEQ ID NO:378 | -0.295 | 7.8812 | 2.51 | 25.12 |
| miR-372 | miR-372GSP | CATGATCAGCTGGGCCAAGAACGCTCAAAT SEQ ID NO:379 | miR-372RP | A+AA+GTGCTGCGACA SEQ ID NO:380 | -0.2984 | 8.9183 | 0.05 | 0.53 |
| miR-373* | miR-373*GSP | CATGATCAGCTGGGCCAAGAGGAAAGCGCC SEQ ID NO:381 | miR-373*RP | A+CT+CAAAATGGGGG SEQ ID NO:382 | -0.2705 | 8.4513 | 0.20 | 1.99 |
| miR-373 | miR-373GSP | CATGATCAGCTGGGCCAAGAACACCCCAAA SEQ ID NO:383 | miR-373RP2 | GA+AG+TGCTTCGATTTTGG SEQ ID NO:384 | -0.307 | 7.9056 | 9.13 | 91.32 |
| miR-374 | miR-374GSP2 | CATGATCAGCTGGGCCAAGACACTTATCA SEQ ID NO:385 | miR-374RP | TT+AT+AATA+CAACCTGAT AAG SEQ ID NO:386 | -0.2655 | 9.3795 | 9.16 | 91.60 |
| miR-375 | miR-375GSP | CATGATCAGCTGGGCCAAGATCACGCGAGC SEQ ID NO:387 | miR-375RP | TT+TG+TTCGTTCGGC SEQ ID NO:388 | -0.3041 | 8.1181 | 0.09 | 0.90 |
| miR-376b | miR-376b GSP8^{#} | CATGATCAGCTGGGCCAAGAAACATGGA SEQ ID NO:389 | miR-376bRP^{#} | AT+CAT+AGA+GGAAAATCC A SEQ ID NO:390 | -0.2934 | 9.0188 | 1.07 | 10.74 |
| miR-378 | miR-378GSP | CATGATCAGCTGGGCCAAGAACACAGGACC SEQ ID NO:391 | miR-378RP | C+TC+CTGACTCCAGG SEQ ID NO:392 | -0.2899 | 8.1467 | 0.07 | 0.73 |
| miR-379 | miR-379_GSP7^{#} | CATGATCAGCTGGGCCAAGATACGTTC SEQ ID NO:393 | miR-379RP2^{#} | T+GGT+AGACTATGGAACG SEQ ID NO:394 | -0.2902 | 8.2149 | 10.89 | 108.86 |
| miR-380-5p | miR-380-5pGSP | CATGATCAGCTGGGCCAAGAGCGCATGTTC SEQ ID NO:395 | miR-380-5pRP | T+GGT+TGACCATAGA SEQ ID NO:396 | -0.2462 | 9.4324 | 1.30 | 13.04 |
| miR-380-3p | miR-380-3pGSP | CATGATCAGCTGGGCCAAGAAAGATGTGGA SEQ ID NO:397 | miR-380-3pRP | TA+TG+TAATATGGTCCACA SEQ ID NO:398 | -0.3037 | 8.0356 | 3.69 | 36.89 |
| miR-381 | miR-381GSP2 | CATGATCAGCTGGGCCAAGAACAGAGAGC SEQ ID NO:399 | miR-381RP2 | TATA+CAA+GGGCAAGCT SEQ ID NO:400 | -0.3064 | 8.8704 | 1.72 | 17.16 |
| miR-382 | miR-382GSP | CATGATCAGCTGGGCCAAGACGAATCCACC SEQ ID NO:401 | miR-382RP | G+AA+GTTGTTCGTGGT SEQ ID NO:402 | -0.2803 | 7.6738 | 0.66 | 6.57 |
| miR-383 | miR-383GSP | CATGATCAGCTGGGCCAAGAAGCCACAATC SEQ ID NO:403 | miR-383RP2 | A+GATC+AGAAGGTGATTG T SEQ ID NO:404 | -0.2866 | 8.1463 | 0.54 | 5.45 |
| miR-410 | miR-410 GSP9# | CATGATCAGCTGGGCCAAGAACAGGCCAT SEQ ID NO:405 | miR-410RP^{#} | AA+TA+TAA+CA+CAGATGG C SEQ ID NO:406 | -0.2297 | 8.5166 | 4.27 | 42.71 |
| miR-412 | miR-412 GSP10^{#} | CATGATCAGCTGGGCCAAGAACGGCTAGTG SEQ ID NO:407 | miR-412RP^{#} | A+CTT+CACCTGGTCCACTA SEQ ID NO:408 | -0.3001 | 7.9099 | 4.24 | 42.37 |
| miR-422a | miR-422aGSP | CATGATCAGCTGGGCCAAGAGGCCTTCTGA SEQ ID NO:409 | miR-422aRP | C+TG+GACTTAGGGTC SEQ ID NO:410 | -0.3079 | 9.3108 | 5.95 | 59.54 |
| miR-422b | miR-422bGSP | CATGATCAGCTGGGCCAAGAGGCCTTCTGA SEQ ID NO:411 | miR-422bRP | C+TG+GACTTGGAGTC SEQ ID NO:412 | -0.2993 | 8.9437 | 4.86 | 48.56 |
| miR-423 | miR-423GSP | CATGATCAGCTGGGCCAAGACTGAGGGGCC SEQ ID NO:413 | miR-423RP | A+GC+TCGGTCTGAGG SEQ ID NO:414 | -0.3408 | 9.2274 | 6.06 | 60.62 |
| miR-424 | miR-424GSP^{#} | CATGATCAGCTGGGCCAAGATTCAAAACAT SEQ ID NO:415 | miR-424RP2# | C+AG+CAGCAATTCATGTTT T SEQ ID NO:416 | -0.3569 | 9.3419 | 10.78 | 107.85 |
| miR-425 | miR-425GSP | CATGATCAGCTGGGCCAAGAGGCGGACACG SEQ ID NO:417 | miR-425RP | A+TC+GGGAATGTCGT SEQ ID NO:418 | -0.2932 | 7.9786 | 0.39 | 3.93 |
| miR-429 | miR-429_GSP11^{#} | CATGATCAGCTGGGCCAAGAACGGTTTTACC SEQ ID NO:419 | miR-429RP5^{#} | T+AATAC+TG+TCTGGTAAA A SEQ ID NO:420 | -0.2458 | 8.2805 | 16.21 | 162.12 |
| miR-431 | miR-431 GSP10^{#} | CATGATCAGCTGGGCCAAGATGCATGACGG SEQ ID NO:421 | miR-431RP^{#} | T+GT+CTTGCAGGCCG SEQ ID NO:422 | -0.3107 | 7.7127 | 7.00 | 70.05 |
| miR-448 | miR-448GSP | CATGATCAGCTGGGCCAAGAATGGGACATC SEQ ID NO:423 | miR-448RP | TTG+CATA+TGTAGGATG SEQ ID NO:424 | -0.3001 | 8.4969 | 0.12 | 1.16 |
| miR-449 | miR-449GSP10^{#} | CATGATCAGCTGGGCCAAGAACCAGCTAAC SEQ ID NO:425 | miR-449RP2# | T+GG+CAGTGTATTGTTAGC SEQ ID NO:426 | -0.3225 | 8.4953 | 2.57 | 25.70 |
| miR-450 | miR-450GSP | CATGATCAGCTGGGCCAAGATATTAGGAAC SEQ ID NO:427 | miR-450RP | TTTT+TG+CGATGTGTT SEQ ID NO:428 | -0.2906 | 8.1404 | 0.48 | 4.82 |
| miR-451 | miR-451 GSP10^{#} | CATGATCAGCTGGGCCAAGAAAACTCAGTA SEQ ID NO:429 | miR-451RP^{#} | AAA+CCG+TTA+CCATTACT GA SEQ ID NO:430 | -0.2544 | 8.0291 | 1.73 | 17.35 |
| let7a | let7a-GSP2^{#} | CATGATCAGCTGGGCCAAGAAACTATAC SEQ ID NO:431 | let7a-RP^{#} | T+GA+GGTAGTAGGTTG SEQ ID NO:432 | -0.3089 | 9.458 | 0.04 | 0.38 |
| let7b | let7b-GSP2^{#} | CATGATCAGCTGGGCCAAGAAACCACAC SEQ ID NO:433 | let7b-RP^{#} | T+GA+GGTAGTAGGTTG SEQ ID NO:432 | -0.2978 | 7.9144 | 0.05 | 0.54 |
| let7c | let7c-GSP2^{#} | CATGATCAGCTGGGCCAAGAAACCATAC SEQ ID NO:434 | let7c-RP^{#} | T+GA+GGTAGTAGGTTG SEQ ID NO:432 | -0.308 | 7.9854 | 0.01 | 0.14 |
| let7d | let7d-GSP2^{#} | CATGATCAGCTGGGCCAAGAACTATGCA SEQ ID NO:435 | let7d-RP^{#} | A+GA+GGTAGTAGGTTG SEQ ID NO:436 | -0.3238 | 8.3359 | 0.06 | 0.57 |
| let7e | let7e-GSP2^{#} | CATGATCAGCTGGGCCAAGAACTATACA SEQ ID NO:437 | let7e-RP^{#} | T+GA+GGTAGGAGGTTG SEQ ID NO:438 | -0.3284 | 9.7594 | 0.22 | 2.20 |
| let7f | let7f-GSP2^{#} | CATGATCAGCTGGGCCAAGAAACTATAC SEQ ID NO:439 | let7f-RP^{#} | T+GA+GGTAGTAGATTG SEQ ID NO:440 | -0.2901 | 11.107 | 0.32 | 3.18 |
| let7g | let7g-GSP2^{#} | CATGATCAGCTGGGCCAAGAACTGTACA SEQ ID NO:441 | let7g-RP^{#} | T+GA+GGTAGTAGTTTG SEQ ID NO:442 | -0.3469 | 9.8235 | 0.16 | 1.64 |
| let7i | let7i-GSP2^{#} | CATGATCAGCTGGGCCAAGAACAGCACA SEQ ID NO:443 | let7i-RP^{#} | T+GA+GGTAGTAGTTTG SEQ ID NO:444 | -0.321 | 10.82 | 0.20 | 1.99 |
| miR-377 | miR-377GSP | CATGATCAGCTGGGCCAAGAACAAAAGTTG SEQ ID NO:445 | miR-377RP2 | AT+CA+CACAAAGGCAAC SEQ ID NO:446 | -0.2979 | 10.612 | 13.45 | 134.48 |
| miR-376a | miR-376a_GSP7 | CATGATCAGCTGGGCCAAGAACGTGGA SEQ ID NO:447 | miR-376a_RP5 | AT+CAT+AGA+GGAAAATCC SEQ ID NO:448 | -0.2938 | 10.045 | 63.00 | 630.00 |
| miR-22 | miR-22GSP | CATGATCAGCTGGGCCAAGAACAGTTCTTC SEQ ID NO:449 | miR-22RP | A+AG+CTGCCAGTTGA SEQ ID NO:450 | -0.2862 | 8.883 | 20.46 | 204.58 |
| miR-200c | miR-200cGSP2 | CATGATCAGCTGGGCCAAGACCATCATTA SEQ ID NO:451 | miR-200cRP | T+AA+TACTGCCGGGT SEQ ID NO:452 | -0.3094 | 11.5 | 15.99 | 159.91 |
| miR-24 | miR-24GSP | CATGATCAGCTGGGCCAAGACTGTTCCTGC SEQ ID NO:453 | miR-24RP | T+GG+CTCAGTTCAGC SEQ ID NO:454 | -0.3123 | 8.6824 | 24.34 | 243.38 |
| miR-29cDNA | miR-29cGSP10 | CATGATCAGCTGGGCCAAGAACCGATTTCA SEQ ID NO:455 | miR-29cRP | T+AG+CACCATTTGAAAT SEQ ID NO:456 | -0.2975 | 8.8441 | 23.22 | 232.17 |
| miR-18 | miR-18GSP | CATGATCAGCTGGGCCAAGATATCTGCACT SEQ ID NO:457 | miR-18RP | T+AA+GGTGCATCTAGT SEQ ID NO:458 | -0.3209 | 9.0999 | 14.90 | 149.01 |
| miR-185 | miR-185GSP | CATGATCAGCTGGGCCAAGAGAACTGCCTT SEQ ID NO:459 | miR-185RP | T+GG+AGAGAAAGGCA SEQ ID NO:460 | -0.3081 | 8.9289 | 15.73 | 157.32 |
| miR181b | miR-181bGSP8^{#} | CATGATCAGCTGGGCCAAGACCCACCGA SEQ ID NO:461 | miR-181bRP2^{#} | AA+CATT+CATTGCTGTC SEQ ID NO:462 | -0.3115 | 10.846 | 15.87 | 158.67 |
| miR-128a | miR-128aGSP | CATGATCAGCTGGGCCAAGAAAAAGAGACC SEQ ID NO:161 | miR-128anLRP | TCACAGTGAACCGGT SEQ ID NO: 494 | approx. -0.2866 | approx. 8.0867 | approx. 0.16 | approx. 1.60 |
| miR-138 | miR-138GSP2 | CATGATCAGCTGGGCCAAGACGGCCTGAT SEQ ID NO:187 | miR-138nLRP | AGCTGGTGTTGTGAA SEQ ID NO: 495 | approx. -0.3023 | approx. 9.0814 | approx. 0.22 | approx. 2.19 |
| miR-143 | miR-143GSP8^{#} | CATGATCAGCTGGGCCAAGATGAGCTAC SEQ ID NO:197 | miR-143nLRP | TGAGATGAAGCACTGT SEQ ID NO: 496 | approx. -0.3008 | approx. 9.2675 | approx. 0.37 | approx. 3.71 |
| miR-150 | miR-150GSP3 | CATGATCAGCTGGGCCAAGACACTGGTA SEQ ID NO:213 | miR-150nLRP | TCTCCCAACCCTTGTA SEQ ID NO: 497 | approx. -0.2943 | approx. 8.3945 | approx. *0.06* | approx. 0.56 |
| miR-181a | miR-181aGSP9^{#} | CATGATCAGCTGGGCCAAGAACTCACCGA SEQ ID NO:227 | miR-181anLRP | AACATTCAACGCTGT SEQ ID NO: 498 | approx. -0.2919 | approx. 7.968 | approx. 1.70 | approx. 17.05 |
| miR-194 | mir194GSP8^{#} | CATGATCAGCTGGGCCAAGATCCACATG SEQ ID NO:255 | miR-194nLRP | TGTAACAGCAACTCCA SEQ ID NO: 499 | approx. -0.3078 | approx. 8.8045 | approx. 0.37 | approx. 3.69 |

### EXAMPLE 4

This Example describes assays and primers designed for quantitative analysis of murine miNRA expression patterns.

Methods: The representative murine microRNA target templates described in TABLE 7 are publically available accessible on the World Wide Web at the Wellcome Trust Sanger Institute website in the "miRBase sequence database" as described in Griffith-Jones et al. (2004), Nucleic Acids Research 32:D109-D111 and and Griffith-Jones et al. (2006), Nucleic Acids Research 34: D140-D144. As indicated below in TABLE 7, the murine microRNA templates are either totally identical to the corresponding human microRNA templates, identical in the overlapping sequence with differing ends, or contain one or more base pair changes as compared to the human microRNA sequence. The murine microRNA templates that are identical or that have identical overlapping sequence to the corresponding human templates can be assayed using the same primer sets designed for the human microRNA templates, as indicated in TABLE 7. For the murine microRNA templates with one or more base pair changes in comparison to the corresponding human templates, primer sets have been designed specifically for detection of the murine microRNA, and these primers are provided in TABLE 7. The extension primer reaction and quantitative PCR reactions for detection of the murine microRNA templates may be carried out as described in EXAMPLE 3.

**TABLE 7: Primers to detect murine microRNA target templates**

| **Mouse Target microRNA:** | **Extension Primer Name** | **Extension Primer Sequence** | **Reverse Primer Name** | **Reverse Primer Sequence** | **Mouse microRNA as compared to Human microRNA,** |
|---|---|---|---|---|---|
| **miR-1** | miR1GSP10 | CATGATCAGCTGGGCCAAGATACATACTTC SEQ ID NO:47 | miR-1RP | T+G+GAA+TG+TAAAGAAGT SEQ ID NO:48 | Identical |
| **miR-7** | miR-7GSP10 | CATGATCAGCTGGGCCAAGAAACAAAATC SEQ ID NO: 486 | miR-7_RP6 | T+GGAA+GACTTGTGATTTT SEQ ID NO: 487 | one or more base pairs differ |
| **miR-9*** | miR-9*GSP | CATGATCAGCTGGGCCAAGAACTTTCGGTT SEQ ID NO:51 | miR-9*RP | TAAA+GCT+AGATAACCG SEQ ID NO:52 | Identical overlapping sequence, ends differ |
| **miR-10a** | miR-10aGSP | CATGATCAGCTGGGCCAAGACACAAATTCG SEQ ID NO:53 | miR-10aRP | T+AC+CCTGTAGATCCG SEQ ID NO:54 | Identical |
| **miR-10b** | miR-10b_GSP11 | CATGATCAGCTGGGCCAAGAACACAAATTC G SEQ ID NO: 492 | miR-10b_RP2 | C+CC+TGT+AGAACCGAAT SEQ ID NO: 493 | one or more base pairs differ |
| **miR-15a** | miR-15aGSP | CATGATCAGCTGGGCCAAGACACAAACCAT SEQ ID NO:57 | miR-15aRP | T+AG+CAGCACATAATG SEQ ID NO:58 | Identical |
| **miR-15b** | miR-15bGSP2 | CATGATCAGCTGGGCCAAGATGTAAACCA SEQ ID NO:59 | miR-15bRP | T+AG+CAGCACATCAT SEQ ID NO:60 | Identical |
| **miR-16** | miR-16GSP2 | CATGATCAGCTGGGCCAAGACGCCAATAT SEQ ID NO:61 | miR-16RP | T+AG+CAGCACGTAAA SEQ ID NO:62 | Identical |
| **miR-17-3p** | miR-17-3pGSP | CATGATCAGCTGGGCCAAGAACAAGTGCCC SEQ ID NO: 463 | miR-17-3pRP | A+CT+GCAGTGAGGGC SEQ ID NO: 464 | one or more base pairs differ |
| **miR-17-5p** | miR-17-5pGSP2 | CATGATCAGCTGGGCCAAGAACTACCTGC SEQ ID NO:65 | miR-17-5pRP | C+AA+AGTGCTTACAGTG SEQ ID NO:66 | Identical |
| **miR-19a** | miR-19aGSP2 | CATGATCAGCTGGGCCAAGATCAGTTTTG SEQ ID NO:67 | miR-19aRP | TG+TG+CAAATCTATGC SEQ ID NO:68 | Identical |
| **miR-19b** | miR-19bGSP | CATGATCAGCTGGGCCAAGATCAGTTTTGC SEQ ID NO:69 | miR-19bRP | TG+TG+CAAATCCATG SEQ ID NO:70 | Identical |
| **miR-20** | miR-20GSP3 | CATGATCAGCTGGGCCAAGACTACCTGC SEQ ID NO:71 | miR-20RP | T+AA+AGTGCTTATAGTGCA SEQ ID NO:72 | Identical |
| **miR-21** | miR-21GSP2 | CATGATCAGCTGGGCCAAGATCAACATCA SEQ ID NO: 73 | miR-21RP | T+AG+CTTATCAGACTGATG SEQ ID NO:74 | Identical |
| **miR-23a** | miR-23aGSP | CATGATCAGCTGGGCCAAGAGGAAATCCCT SEQ ID NO:75 | miR-23aRP | A+TC+ACATTGCCAGG SEQ ID NO:76 | Identical |
| **miR-23b** | miR-23bGSP | CATGATCAGCTGGGCCAAGAGGTAATCCCT SEQ ID NO:77 | miR-23bRP | A+TC+ACATTGCCAGG SEQ ID NO:78 | Identical |
| **miR-24** | miR-24P5 | CATGATCAGCTGGGCCAAGACTGTTCCTGC TG SEQ ID NO: 7 | miR24-1,2R | TGG+CTCAGTTCAGC SEQ ID NO: 19 | Identical |
| **miR-25** | miR-25GSP | CATGATCAGCTGGGCCAAGATCAGACCGAG SEQ ID NO:79 | miR-25RP | C+AT+TGCACTTGTCTC SEQ ID NO:80 | Identical |
| **miR-26a** | miR-26aGSP9 | CATGATCAGCTGGGCCAAGAGCCTATCCT SEQ ID NO:81 | miR-26aRP2 | TT+CA+AGTAATCCAGGAT SEQ ID NO:82 | Identical |
| **miR-26b** | miR-26bGSP9 | CATGATCAGCTGGGCCAAGAAACCTATCC SEQ ID NO:83 | miR-26bRP2 | TT+CA+AGT+AATTCAGGAT SEQ ID NO:84 | Identical |
| **miR-27a** | miR-27aGSP | CATGATCAGCTGGGCCAAGAGCGGAACTTA SEQ ID NO:85 | miR-27aRP | TT+CA+CAGTGGCTAA SEQ ID NO:86 | Identical |
| **miR-27b** | miR-27bGSP | CATGATCAGCTGGGCCAAGAGCAGAACTTA SEQ ID NO:87 | miR-27bRP | TT+CA+CAGTGGCTAA SEQ ID NO:88 | Identical |
| **miR-28** | miR-28GSP | CATGATCAGCTGGGCCAAGACTCAATAGAC SEQ ID NO:89 | miR-28RP | A+AG+GAGCTCACAGT SEQ ID NO:90 | Identical |
| **miR-29a** | miR-29aGSP8 | CATGATCAGCTGGGCCAAGAAACCGATT SEQ ID NO:91 | miR-29aRP2 | T+AG+CACCATCTGAAAT SEQ ID NO:92 | Identical |
| **miR-29b** | miR-29bGSP2 | CATGATCAGCTGGGCCAAGAAACACTGAT SEQ ID NO:93 | miR-29bRP2 | T+AG+CACCATTTGAAATCAG SEQ ID NO:94 | Identical |
| **miR-30a-5p** | miR-30a-5pGSP | CATGATCAGCTGGGCCAAGACTTCCAGTCG SEQ ID NO:95 | miR-30a-5pRP | T+GT+AAACATCCTCGAC SEQ ID NO:96 | Identical |
| **miR-30b** | miR-30bGSP | CATGATCAGCTGGGCCAAGAAGCTGAGTGT SEQ ID NO:97 | miR-30bRP | TGT+AAA+CATCCTACACT SEQ ID NO:98 | Identical |
| **miR-30c** | miR-30cGSP | CATGATCAGCTGGGCCAAGAGCTGAGAGTG SEQ ID NO:99 | miR-30cRP | TGT+AAA+CATCCTACACT SEQ ID NO:100 | Identical |
| **miR-30d** | miR-30dGSP | CATGATCAGCTGGGCCAAGACTTCCAGTCG SEQ ID NO:101 | miR-30dRP | T+GTAAA+CATCCCCG SEQ ID NO:102 | Identical |
| **miR-30e-3p** | miR-30e-3pGSP9 | CATGATCAGCTGGGCCAAGAGCTGTAAAC SEQ ID NO:103 | miR-30e-3pRP5 | CTTT+CAGT+CGGATGTTT SEQ ID NO:104 | Identical |
| **miR-31** | miR-31GSP | CATGATCAGCTGGGCCAAGACAGCTATGCC SEQ ID NO:107 | miR-31RP | G+GC+AAGATGCTGGC SEQ ID NO:108 | Identical overlapping sequence, ends differ |
| **miR-32** | miR-32GSP | CATGATCAGCTGGGCCAAGAGCAACTTAGT SEQ ID NO:109 | miR-32RP | TATTG+CA+CATTACTAAG SEQ ID NO:110 | Identical |
| **miR-33** | miR-33GSP2 | CATGATCAGCTGGGCCAAGACAATGCAAC SEQ ID NO:111 | miR-33RP | G+TG+CATTGTAGTTGC SEQ ID NO:112 | Identical |
| **miR-34a** | miR-34aGSP | CATGATCAGCTGGGCCAAGAAACAACCAGC SEQ ID NO:113 | miR-34aRP | T+GG+CAGTGTCTTAG SEQ ID NO:114 | Identical |
| **miR-34b** | miR-34bGSP | CATGATCAGCTGGGCCAAGACAATCAGCTA SEQ ID NO: 115 | miR-34bRP | TA+GG+CAGTGTAATT SEQ ID NO: 482 | one or more base pairs differ |
| **miR-34c** | miR-34cGSP | CATGATCAGCTGGGCCAAGAGCAATCAGCT SEQ ID NO:117 | miR-34cRP | A+GG+CAGTGTAGTTA SEQ ID NO:118 | Identical |
| **miR-92** | miR-92GSP | CATGATCAGCTGGGCCAAGACAGGCCGGGA SEQ ID NO:119 | miR-92RP | T+AT+TGCACTTGTCCC SEQ ID NO:120 | Identical |
| **miR-93** | miR-93GSP | CATGATCAGCTGGGCCAAGACTACCTGCAC SEQ ID NO:121 | miR-93RP | AA+AG+TGCTGTTCGT SEQ ID NO:122 | Identical overlapping sequence, ends differ |
| **miR-96** | miR-96GSP | CATGATCAGCTGGGCCAAGAGCAAAAATGT SEQ ID NO:125 | miR-96RP | T+TT+GGCACTAGCAC SEQ ID NO:126 | Identical overlapping sequence, ends differ |
| **miR-98** | miR-98GSP | CATGATCAGCTGGGCCAAGAAACAATACAA SEQ ID NO:127 | miR-98RP | TGA+GGT+AGTAAGTTG SEQ ID NO:128 | Identical |
| **miR-99a** | miR-99aGSP | CATGATCAGCTGGGCCAAGACACAAGATCG SEQ ID NO:129 | miR-99aRP | A+AC+CCGTAGATCCG SEQ ID NO:130 | Identical overlapping sequence, ends differ |
| **miR-99b** | miR-99bGSP | CATGATCAGCTGGGCCAAGACGCAAGGTCG SEQ ID NO:131 | miR-99bRP | C+AC+CCGTAGAACCG SEQ ID NO:132 | Identical |
| **miR-100** | miR-100GSP | CATGATCAGCTGGGCCAAGACACAAGTTCG SEQ ID NO:133 | miR-100RP | A+AC+CCGTAGATCCG SEQ ID NO:134 | Identical |
| **miR-101** | miR-101GSP | CATGATCAGCTGGGCCAAGACTTCAGTTAT SEQ ID NO:135 | miR-101RP | TA+CAG+TACTGTGATAACT SEQ ID NO:136 | Identical |
| **miR-103** | miR-103GSP | CATGATCAGCTGGGCCAAGATCATAGCCCT SEQ ID NO:137 | miR-103RP | A+GC+AGCATTGTACA SEQ ID NO:138 | Identical |
| **miR-106a** | miR-106aGSP | CATGATCAGCTGGGCCAAGATACCTGCAC SEQ ID NO: 472 | miR-106aRP | CAA+AG+TGCTAACAGTG SEQ ID NO: 473 | one or more base pairs differ |
| **miR-106b** | miR-106bGSP | CATGATCAGCTGGGCCAAGAATCTGCACTG SEQ ID NO:143 | miR-106bRP | T+AAAG+TGCTGACAGT SEQ ID NO:144 | Identical |
| **miR-107** | miR-107GSP8 | CATGATCAGCTGGGCCAAGATGATAGCC SEQ ID NO:145 | miR-107RP2 | A+GC+AGCATTGTACAG SEQ ID NO:146 | Identical |
| **miR-122a** | miR-122aGSP | CATGATCAGCTGGGCCAAGAACAAACACCA SEQ ID NO:147 | miR-122aRP | T+GG+AGTGTGACAAT SEQ ID NO:148 | Identical |
| **miR-124a** | miR-124aGSP | CATGATCAGCTGGGCCAAGATGGCATTCAC SEQ ID NO:149 | miR-124aRP | T+TA+AGGCACGCGGT SEQ ID NO:150 | Identical overlapping sequence, ends differ |
| **miR-125a** | miR-125aGSP | CATGATCAGCTGGGCCAAGACACAGGTTAA SEQ ID NO:151 | miR-125aRP | T+CC+CTGAGACCCTT SEQ ID NO:152 | Identical |
| **miR-125b** | miR-125bGSP | CATGATCAGCTGGGCCAAGATCACAAGTTA SEQ ID NO:153 | miR-125bRP | T+CC+CTGAGACCCTA SEQ ID NO:154 | Identical |
| **miR-126** | miR-126GSP | CATGATCAGCTGGGCCAAGAGCATTATTAC SEQ ID NO:155 | miR-126RP | T+CG+TACCGTGAGTA SEQ ID NO:156 | Identical |
| **miR-126*** | miR-126*GSP3 | CATGATCAGCTGGGCCAAGACGCGTACC SEQ ID NO:157 | miR-126*RP | C+ATT+ATTA+CTTTTGGTACG SEQ ID NO:158 | Identical |
| **miR-127** | miR-127GSP | CATGATCAGCTGGGCCAAGAAGCCAAGCTC SEQ ID NO:159 | miR-127RP | T+CG+GATCCGTCTGA SEQ ID NO:160 | Identical overlapping sequence, ends differ |
| **miR-128a** | miR-128aGSP | CATGATCAGCTGGGCCAAGAAAAAGAGACC SEQ ID NO:161 | miR-128aRP | T+CA+CAGTGAACCGG SEQ ID NO:162 | Identical |
| **miR-128b** | miR-128bGSP | CATGATCAGCTGGGCCAAGAGAAAGAGACC SEQ ID NO:163 | miR-128bRP | T+CA+CAGTGAACCGG SEQ ID NO:164 | Identical |
| **miR-130a** | miR-130aGSP | CATGATCAGCTGGGCCAAGAATGCCCTTTT SEQ ID NO:167 | miR-130aRP | C+AG+TGCAATGTTAAAAG SEQ ID NO:168 | Identical. |
| **miR-130b** | miR-130bGSP | CATGATCAGCTGGGCCAAGAATGCCCTTTC SEQ ID NO:169 | miR-130bRP | C+AG+TGCAATGATGA SEQ ID NO:170 | Identical |
| **miR-132** | miR-132GSP | CATGATCAGCTGGGCCAAGACGACCATGGC SEQ ID NO:171 | miR-132RP | T+AA+CAGTCTACAGCC SEQ ID NO:172 | Identical |
| **miR-133a** | miR-133aGSP | CATGATCAGCTGGGCCAAGAACAGCTGGTT SEQ ID NO:173 | miR-133aRP | T+TG+GTCCCCTTCAA SEQ ID NO:174 | Identical |
| **miR-133b** | miR-133bGSP | CATGATCAGCTGGGCCAAGATAGCTGGTTG SEQ ID NO:175 | miR-133bRP | T+TG+GTCCCCTTCAA SEQ ID NO:176 | Identical |
| **miR-134** | miR-134GSP | CATGATCAGCTGGGCCAAGACCCTCTGGTC SEQ ID NO:177 | miR-134RP | T+GT+GACTGGTTGAC SEQ ID NO:178 | Identical overlapping sequence, ends differ |
| **miR-135a** | miR-135aGSP | CATGATCAGCTGGGCCAAGATCACATAGGA SEQ ID NO:179 | miR-135aRP | T+AT+GGCTTTTTATTCCT SEQ ID NO:180 | Identical |
| **miR-135b** | miR-135bGSP | CATGATCAGCTGGGCCAAGACACATAGGAA SEQ ID NO:181 | miR-135bRP | T+AT+GGCTTTTCATTCC SEQ ID NO:182 | Identical |
| **miR-136** | miR-136GSP | CATGATCAGCTGGGCCAAGATCCATCATCA SEQ ID NO:183 | miR-136RP | A+CT+CCATTTGTTTTGATG SEQ ID NO:184 | Identical |
| **miR-137** | miR-137GSP | CATGATCAGCTGGGCCAAGACTACGCGTAT SEQ ID NO:185 | miR-137RP | T+AT+TGCTTAAGAATACGC SEQ ID NO:186 | Identical overlapping sequence, ends differ |
| **miR-138** | miR-138GSP2 | CATGATCAGCTGGGCCAAGACGGCCTGAT SEQ ID NO:187 | miR-138RP | A+GC+TGGTGTTGTGA SEQ ID NO:188 | Identical |
| **miR-139** | miR-139GSP | CATGATCAGCTGGGCCAAGAAGACACGTGC SEQ ID NO:189 | miR-139RP | T+CT+ACAGTGCACGT SEQ ID NO:190 | Identical |
| **miR-140** | miR-140GSP | CATGATCAGCTGGGCCAAGACTACCATAGG SEQ ID NO:191 | miR-140RP | A+GT+GGTTTTACCCT SEQ ID NO:192 | Identical overlapping sequence, ends differ |
| **miR-141** | miR-141GSP9 | CATGATCAGCTGGGCCAAGACCATCTTTA SEQ ID NO:193 | miR-141RP2 | TAA+CAC+TGTCTGGTAA SEQ ID NO:194 | Identical |
| **miR-142-3p** | miR-142-3pGSP3 | CATGATCAGCTGGGCCAAGATCCATAAA SEQ ID NO:195 | miR-142-3pRP | TGT+AG+TGTTTCCTACT SEQ ID NO:196 | Identical overlapping sequence, ends differ |
| **miR-143** | miR-143GSP8 | CATGATCAGCTGGGCCAAGATGAGCTAC SEQ ID NO:197 | miR-143RP2 | T+GA+GATGAAGCACTG SEQ ID NO:198 | Identical |
| **miR-144** | miR-144GSP2 | CATGATCAGCTGGGCCAAGACTAGTACAT SEQ ID NO:199 | miR-144RP | TA+CA+GTAT+AGATGATG SEQ ID NO:200 | Identical |
| **miR-145** | miR-145GSP2 | CATGATCAGCTGGGCCAAGAAAGGGATTC SEQ ID NO:201 | miR-145RP | G+TC+CAGTTTTCCCA SEQ ID NO:202 | Identical |
| **miR-146** | miR-146GSP3 | CATGATCAGCTGGGCCAAGAAACCCATG SEQ ID NO:203 | miR-146RP | T+GA+GAACTGAATTCCA SEQ ID NO:204 | Identical |
| **miR-148a** | miR-148aGSP2 | CATGATCAGCTGGGCCAAGAACAAAGTTC SEQ ID NO:207 | miR-148aRP2 | T+CA+GTGCACTACAGAACT SEQ ID NO:208 | Identical |
| **miR-148b** | miR-148bGSP2 | CATGATCAGCTGGGCCAAGAACAAAGTTC SEQ ID NO:209 | miR-148bRP | T+CA+GTGCATCACAG SEQ ID NO:210 | Identical |
| **miR-149** | miR-149GSP2 | CATGATCAGCTGGGCCAAGAGGAGTGAAG SEQ ID NO:211 | miR-149RP | T+CT+GGCTCCGTGTC SEQ ID NO:212 | Identical |
| **miR-150** | miR-150GSP3 | CATGATCAGCTGGGCCAAGACACTGGTA SEQ ID NO:213 | miR-150RP | T+CT+CCCAACCCTTG SEQ ID NO:214 | Identical |
| **miR-151** | miR-151GSP2 | CATGATCAGCTGGGCCAAGACCTCAAGGA SEQ ID NO: 215 | miR-151RP | A+CT+AGACTGAGGCTC SEQ ID NO: 477 | one or more base pairs differ |
| **miR-152** | miR-152GSP2 | CATGATCAGCTGGGCCAAGACCCAAGTTC SEQ ID NO:217 | miR-152RP | T+CA+GTGCATGACAG SEQ ID NO:218 | Identical |
| **miR-153** | miR-153GSP2 | CATGATCAGCTGGGCCAAGATCACTTTTG SEQ ID NO:219 | miR-153RP | TTG+CAT+AGTCACAAAA SEQ ID NO:220 | Identical overlapping sequence, ends differ |
| **miR-154** | miR-154GSP9 | CATGATCAGCTGGGCCAAGACGAAGGCAA SEQ ID NO:223 | miR-154RP3 | TA+GGTTA+TCCGTGTT SEQ ID NO:224 | Identical |
| **miR-155** | miR-155GSP8 | CATGATCAGCTGGGCCAAGACCCCTATC SEQ ID NO: 225 | miR-155RP2 | TT+AA+TGCTAATTGTGATAGG SEQ ID NO: 489 | one or more base pairs differ |
| **miR-181a** | miR-181aGSP9 | CATGATCAGCTGGGCCAAGAACTCACCGA SEQ ID NO:227 | miR-181aRP2 | AA+CATT+CAACGCTGTC SEQ ID NO:228 | Identical |
| **miR-181c** | miR-181cGSP9 | CATGATCAGCTGGGCCAAGAACTCACCGA SEQ ID NO:229 | miR-181cRP2 | AA+CATT+CAACCTGTCG SEQ ID NO:230 | Identical |
| **miR-182** | miR-182*GSP | CATGATCAGCTGGGCCAAGATAGTTGGCAA SEQ ID NO:231 | miR-182*RP | T+GG+TTCTAGACTTGC SEQ ID NO:232 | Identical |
| **miR-183** | miR-183GSP2 | CATGATCAGCTGGGCCAAGACAGTGAATT SEQ ID NO:235 | miR-183RP | T+AT+GGCACTGGTAG SEQ ID NO:236 | Identical |
| **miR-184** | miR-184GSP2 | CATGATCAGCTGGGCCAAGAACCCTTATC SEQ ID NO:237 | miR-184RP | T+GG+ACGGAGAACTG SEQ ID NO:238 | Identical |
| **miR-186** | miR-186GSP9 | CATGATCAGCTGGGCCAAGAAAGCCCAAA SEQ ID NO:239 | miR-186RP3 | CA+AA+GAATT+CTCCTTTTGG SEQ ID NO:240 | Identical |
| **miR-187** | miR-187GSP | CATGATCAGCTGGGCCAAGACGGCTGCAAC SEQ ID NO:241 | miR-187RP | T+CG+TGTCTTGTGTT SEQ ID NO:242 | Identical overlapping sequence ends differ |
| **miR-188** | miR-188GSP | CATGATCAGCTGGGCCAAGAACCCTCCACC SEQ ID NO:243 | miR-188RP | C+AT+CCCTTGCATGG SEQ ID NO:244 | Identical |
| **miR-189** | miR-189GSP2 | CATGATCAGCTGGGCCAAGAACTGATATC SEQ ID NO:245 | miR-189RP | G+TG+CCTACTGAGCT SEQ ID NO:246 | Identical |
| **miR-190** | miR-190GSP9 | CATGATCAGCTGGGCCAAGAACCTAATAT SEQ ID NO:247 | miR-190RP4 | T+GA+TA+TGTTTGATATATTAG SEQ ID NO:248 | Identical |
| **miR-191** | miR-191GSP2 | CATGATCAGCTGGGCCAAGAAGCTGCTTT SEQ ID NO:249 | miR-191RP2 | C+AA+CGGAATCCCAAAAG SEQ ID NO:250 | Identical |
| **miR-192** | miR-192GSP2 | CATGATCAGCTGGGCCAAGAGGCTGTCAA SEQ ID NO:251 | miR-192RP | C+TGA+CCTATGAATTGAC SEQ ID NO:252 | Identical overlapping sequence, ends differ |
| **miR-193** | miR-193GSP9 | CATGATCAGCTGGGCCAAGACTGGGACTT SEQ ID NO:253 | miR-193RP2 | AA+CT+GGCCTACAAAG SEQ ID NO:254 | Identical |
| **miR-194** | mir194GSP8 | CATGATCAGCTGGGCCAAGATCCACATG SEQ ID NO:255 | mir194RP | TG+TAA+CAGCAACTCCA SEQ ID NO:256 | Identical |
| **miR-195** | miR-195GSP9 | CATGATCAGCTGGGCCAAGAGCCAATATT SEQ ID NO:257 | miR-195RP3 | T+AG+CAG+CACAGAAATA SEQ ID NO:258 | Identical |
| **miR-196a** | miR-196aGSP | CATGATCAGCTGGGCCAAGACCAACAACAT SEQ ID NO:261 | miR-196aRP | TA+GG+TAGTTTCATGTTG SEQ ID NO:262 | Identical |
| **miR-196b** | miR-196bGSP | CATGATCAGCTGGGCCAAGACCAACAACAG SEQ ID NO:259 | miR-196bRP | TA+GGT+AGTTTCCTGT SEQ ID NO:260 | Identical |
| **miR-199a*** | miR-199a*GSP2 | CATGATCAGCTGGGCCAAGAAACCAATGT SEQ ID NO:267 | miR-199a*RP | T+AC+AGTAGTCTGCAC SEQ ID NO:268 | Identical |
| **miR-199a** | miR-199aGSP2 | CATGATCAGCTGGGCCAAGAGAACAGGTA SEQ ID NO:269 | miR-199aRP | C+CC+AGTGTTCAGAC SEQ ID NO:270 | Identical |
| **miR-199b** | miR-199bGSP | CATGATCAGCTGGGCCAAGAGAACAGGTAG SEQ ID NO: 475 | miR-199bRP | C+CC+AGTGTTTAGAC SEQ ID NO: 272 | one or more base pairs differ |
| **miR-200a** | miR-200aGSP2 | CATGATCAGCTGGGCCAAGAACATCGTTA SEQ ID NO:273 | miR-200aRP | TAA+CAC+TGTCTGGT SEQ ID NO:274 | Identical |
| **miR-200b** | miR-200bGSP2 | CATGATCAGCTGGGCCAAGAGTCATCATT SEQ ID NO:275 | miR-200bRP | TAATA+CTG+CCTGGTAAT SEQ ID NO:276 | Identical |
| **miR-203** | miR-203GSP2 | CATGATCAGCTGGGCCAAGACTAGTGGTC SEQ ID NO:279 | miR-203RP | G+TG+AAATGTTTAGGACC SEQ ID NO:280 | Identical overlapping sequence, ends differ |
| **miR-204** | miR-204GSP2 | CATGATCAGCTGGGCCAAGAAGGCATAGG SEQ ID NO:281 | miR-204RP | T+TC+CCTTTGTCATCC SEQ ID NO:282 | Identical overlapping sequence, ends differ |
| **miR-205** | miR-205GSP | CATGATCAGCTGGGCCAAGACAGACTCCGG SEQ ID NO:283 | miR-205RP | T+CCTT+CATTCCACC SEQ ID NO:284 | Identical |
| **miR-206** | mir206GSP7 | CATGATCAGCTGGGCCAAGACCACACA SEQ ID NO:285 | miR-206RP | T+G+GAA+TGTAAGGAAGTGT SEQ ID NO:286 | Identical |
| **miR-208** | miR-208_GSP13 | CATGATCAGCTGGGCCAAGAACAAGCTTTT TGC SEQ ID NO:287 | miR-208_RP4 | ATAA+GA+CG+AGCAAAAAG SEQ ID NO:288 | Identical |
| **miR-210** | miR-210GSP | CATGATCAGCTGGGCCAAGATCAGCCGCTG SEQ ID NO:289 | miR-210RP | C+TG+TGCGTGTGACA SEQ ID NO:290 | Identical |
| **miR-211** | miR-211GSP2 | CATGATCAGCTGGGCCAAGAAGGCAAAGG SEQ ID NO: 491 | miR-211RP | T+TC+CCTTTGTCATCC SEQ ID NO: 292 | one or more base pairs differ |
| **miR-212** | miR-212GSP9 | CATGATCAGCTGGGCCAAGAGGCCGTGAC SEQ ID NO:293 | miR-212RP2 | T+AA+CAGTCTCCAGTCA SEQ ID NO:294 | Identical |
| **miR-213** | miR-213GSP | CATGATCAGCTGGGCCAAGAGGTACAATCA SEQ ID NO:295 | miR-213RP | A+CC+ATCGACCGTTG SEQ ID NO:296 | Identical |
| **miR-214** | miR-214GSP | CATGATCAGCTGGGCCAAGACTGCCTGTCT SEQ ID NO:297 | miR-214RP | A+CA+GCAGGCACAGA SEQ ID NO:298 | Identical |
| **miR-215** | miR-215GSP2 | CATGATCAGCTGGGCCAAGAGTCTGTCAA SEQ ID NO: 299 | miR-215RP | A+TGA+CCTATGATTTGAC SEQ ID NO: 469 | one or more base pairs differ |
| **mIR-216** | miR-216GSP9 | CATGATCAGCTGGGCCAAGACACAGTTGC SEQ ID NO:301 | mir216RP | TAA+TCT+CAGCTGGCA SEQ ID NO:302 | Identical |
| **miR-217** | miR-217GSP2 | CATGATCAGCTGGGCCAAGAATCCAGTCA SEQ ID NO:481 | miR-217RP2 | T+AC+TGCATCAGGAACTGA SEQ ID NO: 304 | one or more base pairs differ |
| **miR-218** | miR-218GSP2 | CATGATCAGCTGGGCCAAGAACATGGTTA SEQ ID NO:305 | miR-218RP | TTG+TGCTT+GATCTAAC SEQ ID NO:306 | Identical |
| **miR-221** | miR-221GSP9 | CATGATCAGCTGGGCCAAGAGAAACCCAG SEQ ID NO:309 | miR-221RP | A+GC+TACATTGTCTGC SEQ ID NO:310 | Identical overlapping sequence, ends differ |
| **miR-222** | miR-222GSP8 | CATGATCAGCTGGGCCAAGAGAGACCCA SEQ ID NO:311 | miR-222RP | A+GC+TACATCTGGCT SEQ ID NO:312 | Identical |
| **miR-223** | miR-223GSP | CATGATCAGCTGGGCCAAGAGGGGTATTTG SEQ ID NO:313 | miR-223RP | TG+TC+AGTTTGTCAAA SEQ ID NO:314 | Identical |
| **miR-224** | miR-224GSP8 | CATGATCAGCTGGGCCAAGATAAACGGA SEQ ID NO:315 | miR-224RP2 | C+AAG+TCACTAGTGGTT SEQ ID NO:316 | Identical overlapping sequence, ends differ |
| **miR-296** | miR-296GSP9 | CATGATCAGCTGGGCCAAGAACAGGATTG SEQ ID NO:317 | miR-296RP2 | A+GG+GCCCCCCCTCAA SEQ ID NO:318 | Identical |
| **miR-299** | miR-299GSP9 | CATGATCAGCTGGGCCAAGAATGTATGTG SEQ ID NO:319 | miR-299RP | T+GG+TTTACCGTCCC SEQ ID NO:320 | Identical |
| **miR-301** | miR-301GSP | CATGATCAGCTGGGCCAAGAGCTTTGACAA SEQ ID NO:321 | miR-301RP | C+AG+TGCAATAGTATTGT SEQ ID NO:322 | Identical |
| **miR-302a** | miR-302aGSP | CATGATCAGCTGGGCCAAGATCACCAAAAC SEQ ID NO:325 | miR-302aRP | T+AAG+TGCTTCCATGT SEQ ID NO:326 | Identical |
| **miR-320** | miR-320_GSPB | CATGATCAGCTGGGCCAAGATTCGCCCT SEQ ID NO:337 | miR-320_RP3 | AAAA+GCT+GGGTTGAGAGG SEQ ID NO:338 | Identical |
| **miR-323** | miR-323GSP | CATGATCAGCTGGGCCAAGAAGAGGTCGAC SEQ ID NO:339 | miR-323RP | G+CA+CATTACACGGT SEQ ID NO:340 | Identical |
| **miR-324-3p** | miR-324-3pGSP | CATGATCAGCTGGGCCAAGACCAGCAGCAC SEQ ID NO:341 | miR-324-3pRP | C+CA+CTGCCCCAGGT SEQ ID NO:342 | Identical |
| **miR-324-5p** | miR-324-5pGSP | CATGATCAGCTGGGCCAAGAACACCAATGC SEQ ID NO:343 | miR-324-5pRP | C+GC+ATCCCCTAGGG SEQ ID NO:344 | Identical overlapping sequence, ends differ |
| **miR-325** | miR-325GSP | CATGATCAGCTGGGCCAAGAACACTTACTG SEQ ID NO: 345 | miR-325RP | C+CT+AGTAGGTGCTC SEQ ID NO: 476 | one or more base pairs differ |
| **miR-326** | miR-326GSP | CATGATCAGCTGGGCCAAGACTGGAGGAAG SEQ ID NO:347 | miR-326RP | C+CT+CTGGGCCCTTC SEQ ID NO:348 | Identical overlapping sequence, ends differ |
| **miR-328** | miR-328GSP | CATGATCAGCTGGGCCAAGAACGGAAGGGC SEQ ID NO:349 | miR-328RP | C+TG+GCCCTCTCTGC SEQ ID NO:350 | Identical |
| **miR-330** | miR-330GSP | CATGATCAGCTGGGCCAAGATCTCTGCAGG SEQ ID NO: 351 | miR-330RP | G+CA+AAGCACAGGGC SEQ ID NO: 478 | one or more base pairs differ |
| **miR-331** | miR-331GSP | CATGATCAGCTGGGCCAAGATTCTAGGATA SEQ ID NO:353 | miR-331RP | G+CC+CCTGGGCCTAT SEQ ID NO:354 | Identical |
| **miR-337** | miR-337GSP | CATGATCAGCTGGGCCAAGAAAAGGCATCA SEQ ID NO:355 | miR-337RP | T+TC+AGCTCCTATATG SEQ ID NO: 490 | one or more base pairs differ |
| **miR-338** | miR-338GSP | CATGATCAGCTGGGCCAAGATCAACAAAAT SEQ ID NO:357 | miR-338RP2 | T+CC+AGCATCAGTGATTT SEQ ID NO:358 | Identical |
| **miR-339** | miR-339GSP9 | CATGATCAGCTGGGCCAAGATGAGCTCCT SEQ ID NO:359 | miR-339RP2 | T+CC+CTGTCCTCCAGG SEQ ID NO:360 | Identical |
| **miR-340** | miR-340GSP | CATGATCAGCTGGGCCAAGAGGCTATAAAG SEQ ID NO:361 | miR-340RP | TC+CG+TCTCAGTTAC SEQ ID NO:362 | Identical |
| **miR-342** | miR-342GSP3 | CATGATCAGCTGGGCCAAGAGACGGGTG SEQ ID NO:363 | miR-342RP | T+CT+CACACAGAAATCG SEQ ID NO:364 | Identical |
| **miR-345** | miR-345GSP | CATGATCAGCTGGGCCAAGAGCACTGGACT SEQ ID NO: 484 | miR-345RP | T+GC+TGACCCCTAGT SEQ ID NO: 485 | one or more base pairs differ |
| **miR-346** | miR-346GSP | CATGATCAGCTGGGCCAAGAAGAGGCAGGC SEQ ID NO: 367 | miR-346RP | T+GT+CTGCCCGAGTG SEQ ID NO: 488 | one or more base pairs differ |
| **miR-363** | miR-363 GSP10 | CATGATCAGCTGGGCCAAGATACAGATGGA SEQ ID NO:369 | miR-363RP | AAT+TG+CAC+GGTATCC SEQ ID NO:370 | Identical |
| **miR-370** | miR-370GSP | CATGATCAGCTGGGCCAAGACCAGGTTCCA SEQ ID NO:375 | miR-370RP | G+CC+TGCTGGGGTGG SEQ ID NO:376 | Identical overlapping sequence, ends differ |
| **miR-375** | miR-375GSP | CATGATCAGCTGGGCCAAGATCACGCGAGC SEQ ID NO:387 | miR-375RP | TT+TG+TTCGTTCGGC SEQ ID NO:388 | Identical |
| **miR-376a** | miR-376aGSP3 | CATGATCAGCTGGGCCAAGAACGTGGAT SEQ ID NO: 467 | miR-376aRP2 | A+TCGTAGA+GGAAAATCCAC SEQ ID NO: 468 | one or more base pairs differ |
| **miR-378** | miR-378GSP | CATGATCAGCTGGGCCAAGAACACAGGACC SEQ ID NO:391 | miR-378RP | C+TC+CTGACTCCAGG SEQ ID NO:392 | Identical |
| miR-379 | miR-379_GSP7 | CATGATCAGCTGGGCCAAGATACGTTC SEQ ID NO:393 | miR-379RP2 | T+GGT+AGACTATGGAACG SEQ ID NO:394 | Identical overlapping sequence, ends differ |
| miR-380-5p | miR-380-5pGSP | CATGATCAGCTGGGCCAAGAGCGCATGTTC SEQ ID NO:395 | miR-380-5pRP | T+GGT+TGACCATAGA SEQ ID NO:396 | Identical differ |
| miR-380-3p | miR-380-3pGSP | CATGATCAGCTGGGCCAAGAAAGATGTGGA SEQ ID NO: 395 | miR-380-3pRP | TA+TG+TAGTATGGTCCACA SEQ ID NO: 483 | one or more base pairs |
| miR-381 | miR-381GSP2 | CATGATCAGCTGGGCCAAGAACAGAGAGC SEQ ID NO:399 | miR-381RP2 | TATA+CAA+GGGCAAGCT SEQ ID NO:400 | Identical |
| miR-382 | miR-382GSP | CATGATCAGCTGGGCCAAGACGAATCCACC SEQ ID NO:401 | miR-382RP | G+AA+GTTGTTCGTGGT SEQ ID NO:402 | Identical |
| miR-383 | miR-383GSP | CATGATCAGCTGGGCCAAGAAGCCACAGTC SEQ ID NO:465 | miR-383RP2 | A+GATC+AGAAGGTGACTGT SEQ ID NO: 466 | one or more base pairs differ |
| miR-384 | miR-384_GSP9 | CATGATCAGCTGGGCCAAGATGTGAACAA SEQ ID NO:470 | miR-384_RP5 | ATT+CCT+AG+AAATTGTTC SEQ ID NO: 471 | one or more base pairs |
| miR-410 | miR-410 GSP9 | CATGATCAGCTGGGCCAAGAACAGGCCAT SEQ ID NO:405 | miR-410RP | AA+TA+TAA+CA+CAGATGGC SEQ ID NO:406 | Identical |
| miR-412 | miR-412 GSP10 | CATGATCAGCTGGGCCAAGAACGGCTAGTG SEQ ID NO:407 | miR-412RP | A+CTT+CACCTGGTCCACTA SEQ ID NO:408 | Identical differ |
| miR-424 | miR-424GSP | CATGATCAGCTGGGCCAAGATCCAAAACAT SEQ ID NO:474 | miR-424RP2 | C+AG+CAGCAATTCATGTTTT SEQ ID NO: 414 | one or more base pairs |
| miR-425 | miR-425GSP | CATGATCAGCTGGGCCAAGAGGCGGACACG SEQ ID NO:417 | miR-425RP | A+TC+GGGAATGTCGT SEQ ID NO:418 | Identical differ |
| miR-429 | miR-429_GSP11 | CATGATCAGCTGGGCCAAGAACGGCATTACC SEQ ID NO:479 | miR-429RP5 | T+AATAC+TG+TCTGGTAATG SEQ ID NO: 480 | one or more base pairs |
| miR-431 | miR-431 GSP10 | CATGATCAGCTGGGCCAAGATGCATGACGG SEQ ID NO:421 | miR-431RP | T+GT+CTTGCAGGCCG SEQ ID NO:422 | Identical overlapping sequence, ends differ |
| miR-448 | miR-448GSP | CATGATCAGCTGGGCCAAGAATGGGACATC SEQ ID NO:423 | miR-448RP | TTG+CATA+TGTAGGATG SEQ ID NO:424 | Identical |
| miR-449 | miR-449GSP10 | CATGATCAGCTGGGCCAAGAACCAGCTAAC SEQ ID NO:425 | miR-449RP2 | T+GG+CAGTGTATTGTTAGC SEQ ID NO:426 | Identical |
| miR-450 | miR-450GSP | CATGATCAGCTGGGCCAAGATATTAGGAAC SEQ ID NO:427 | miR-450RP | TTTT+TG+CGATGTGTT SEQ ID NO:428 | Identical |
| mIR-451 | miR-451 GSP10 | CATGATCAGCTGGGCCAAGAAAACTCAGTA SEQ ID NO:429 | miR-451RP | AAA+CCG+TTA+CCATTACTGA SEQ ID NO:430 | Identical overlapping sequence, ends differ |
| let7a | let7a-GSP2 | CATGATCAGCTGGGCCAAGAAACTATAC SEQ ID NO:431 | let7a-RP | T+GA+GGTAGTAGGTTG SEQ ID NO:432 | Identical overlapping sequence, ends differ |
| let7b | let7b-GSP2 | CATGATCAGCTGGGCCAAGAAACCACAC SEQ ID NO:433 | let7b-RP | T+GA+GGTAGTAGGTTG SEQ ID NO:432 | Identical |
| let7c | let7c-GSP2 | CATGATCAGCTGGGCCAAGAAACCATAC SEQ ID NO:434 | let7c-RP | T+GA+GGTAGTAGGTTG SEQ ID NO:432 | Identical |
| let7d | let7d-GSP2 | CATGATCAGCTGGGCCAAGAACTATGCA SEQ ID NO:435 | let7d-RP | A+GA+GGTAGTAGGTTG SEQ ID NO:436 | Identical |
| let7e | let7e-GSP2 | CATGATCAGCTGGGCCAAGAACTATACA SEQ ID NO:437 | let7e-RP | T+GA+GGTAGGAGGTTG SEQ ID NO:438 | Identical |
| let7f | let7f-GSP2 | CATGATCAGCTGGGCCAAGAAACTATAC SEQ ID NO:439 | let7f-RP | T+GA+GGTAGTAGATTG SEQ ID NO:440 | Identical overlapping sequence, ends differ |
| let7g | let7g-GSP2 | CATGATCAGCTGGGCCAAGAACTGTACA SEQ ID NO:441 | let7g-RP | T+GA+GGTAGTAGTTTG SEQ ID NO:442 | Identical |
| let7i | let7i-GSP2 | CATGATCAGCTGGGCCAAGAACAGCACA SEQ ID NO:443 | let7i-RP | T+GA+GGTAGTAGTTTG SEQ ID NO:444 | Identical |

### EXAMPLE 5

This Example describes the detection and analysis of expression profiles for three microRNAs in total RNA isolated from twelve different tissues using methods in accordance with an embodiment of the present invention.

Methods: Quantitative analysis of miR-1, miR-124 and miR-150 microRNA templates was determined using 0.5 µg of First Choice total RNA (Ambion, Inc.) per 10 µl primer extension reaction isolated from the following tissues: brain, heart, intestine, kidney, liver, lung, lymph, ovary, skeletal muscle, spleen, thymus and uterus. The primer extension enzyme and quantitative PCR reactions were carried out as described above in EXAMPLE 3, using the following PCR primers:

### miR-1 template:

extension primer: CATGATCAGCTGGGCCAAGATACATACTTC (SEQ ID NO: 47)
reverse primer: T+G+GAA+TG+TAAAGAAGT (SEQ ID NO: 48)
forward primer: CATGATCAGCTGGGCCAAGA (SEQ ID NO: 13)

### miR-124 template:

extension primer: CATGATCAGCTGGGCCAAGATGGCATTCAC (SEQ ID NO: 149) reverse primer: T+TA+AGGCACGCGGT (SEQ ID NO: 150)
forward primer: CATGATCAGCTGGGCCAAGA (SEQ ID NO: 13)

### miR-150 template:

extension primer: CATGATCAGCTGGGCCAAGACACTGGTA (SEQ ID NO: 213)
reverse primer: T+CT+CCCAACCCTTG (SEQ ID NO: 214)
forward primer: CATGATCAGCTGGGCCAAGA (SEQ ID NO: 13)

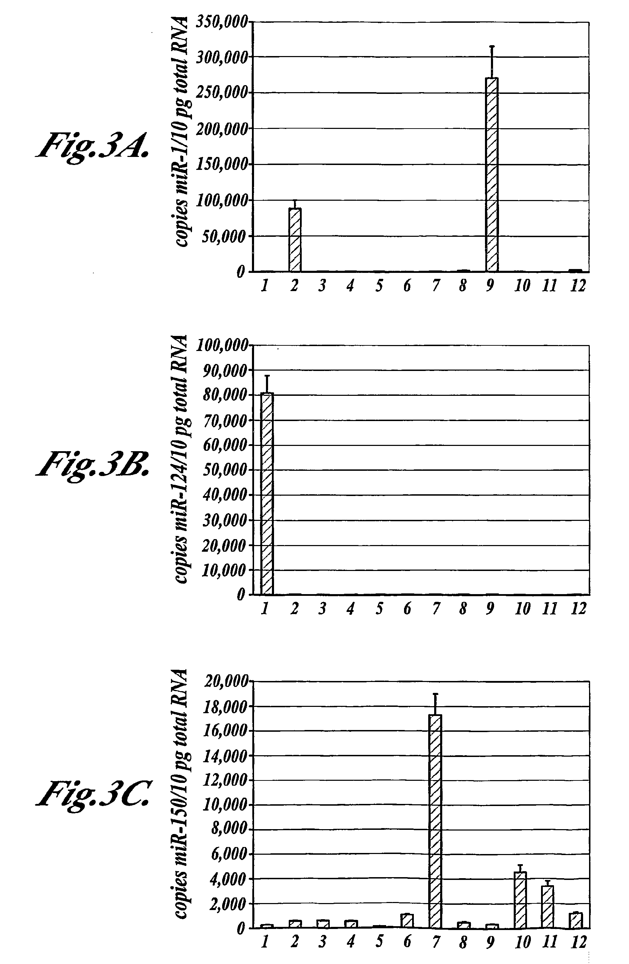

Results: The expression profiles for miR-1, miR-124 and miR-150 are shown in FIGURE 3A, 3B, and 3C, respectively. The data in FIGURES 3A-3C are presented in units of microRNA copies per 10 pg of total RNA (y-axis). These units were chosen since human cell lines typically yield ≤ 10 pg of total RNA per cell. Hence the data shown are estimates of microRNA copies per cell. The numbers on the x-axis correspond to the following tissues: (1) brain, (2) heart, (3) intestine, (4) kidney, (5) liver, (6) lung, (7) lymph, (8) ovary, (9) skeletal muscle, (10) spleen, (11) thymus and (12) uterus.

Consistent with previous reports, very high levels of striated muscle-specific expression were found for miR-1 (as shown in FIGURE 3A), and high levels of brain expression were found for miR-124 (as shown in FIGURE 3B) (see Lagos-Quintana et al., RNA 9:175-179, 2003). Quantitative analysis reveals that these microRNAs are present at tens to hundreds of thousands of copies per cell. These data are in agreement with quantitative Northern blot estimates of miR-1 and miR-124 levels (see Lim et al., Nature 433:769-773, 2005). As shown in FIGURE 3C, miR-150 was found to be highly expressed in the immune-related lymph node, thymus and spleen samples which is also consistent with previous findings (see Baskerville et al., RNA 11:241-247, 2005).

While the preferred embodiment of the invention has been illustrated and described, it will be appreciated that various changes can be made therein without departing from the spirit and scope of the invention.

## Claims

1. A method for producing DNA molecules from a target microRNA molecule having a length in the range of from 21 nucleotides to 25 nucleotides, the method comprising the steps of:
(a) producing a first DNA molecule that is complementary to a target microRNA molecule using primer extension with an extension primer comprising a first portion that hybridizes to a portion of the microRNA molecule and a second portion that hybridizes to the complement of a universal forward primer; and
(b) amplifying the first DNA molecule to produce amplified DNA molecules using the universal forward primer and a reverse primer.

2. The method of Claim 1, wherein at least one of the universal forward primer and the reverse primer comprises at least one nucleic acid molecule comprising a 2'-O,4'-C-methylene-β-D-ribofuranosyl moiety.

3. A method of Claim 1 wherein the primer extension uses an extension primer having a length in the range of from 10 to 100 nucleotides.

4. A method of Claim 1 wherein the primer extension uses an extension primer having a length in the range of from 20 to 35 nucleotides.

5. A method of Claim 1 wherein the first portion of the extension primer has a length in the range of from 3 to 25 nucleotides.

6. A method of Claim 1 wherein the second portion of the extension primer has a length of from 18 to 25 nucleotides.

7. A method of Claim 1 wherein the second portion of the extension primer has a nucleic acid sequence comprising the nucleic acid sequence of SEQ ID NO:1.

8. A method of Claim 1 wherein the universal forward primer has a length in the range of from 16 nucleotides to 100 nucleotides.

9. A method of Claim 1 wherein the universal forward primer consists of the nucleic acid sequence set forth in SEQ ID NO:13.

10. A method of Claim 2 wherein the universal forward primer comprises at least one nucleic acid molecule comprising a 2'-O,4'-C-methylene-β-D-ribofuranosyl moiety.

11. A method of Claim 10 wherein the universal forward primer comprises from 1 to 25 nucleic acid molecules comprising a 2'-O,4'-C-methylene-β-D-ribofuranosyl moiety.

12. A method of Claim 1 wherein the reverse primer has a length in the range of from 10 nucleotides to 100 nucleotides.

13. A method of Claim 2 wherein the reverse primer comprises at least one nucleic acid molecule comprising a 2'-O,4'-C-methylene-β-D-ribofuranosyl moiety.

14. A method of Claim 13 wherein the reverse primer comprises from 1 to 25 nucleic acid molecules comprising a 2'-O,4'-C-methylene-β-D-ribofuranosyl moiety.

15. A method of Claim 1 wherein the reverse primer is selected to specifically hybridize to a DNA molecule complementary to a selected microRNA molecule under defined hybridization conditions.

16. A method of Claim 1 further comprising the step of measuring the amount of amplified DNA molecules.

17. A method of Claim 1 wherein amplification is achieved by multiple successive PCR reactions.

18. A method for measuring the amount of a target microRNA having a length in the range of from 21 nucleotides to 25 nucleotides in a sample from a living organism, the method comprising the step of measuring the amount of a target microRNA molecule in a multiplicity of different cell types within a living organism, wherein the amount of the target microRNA molecule is measured by a method comprising the steps of:
(1) producing a first DNA molecule complementary to the target microRNA molecule in the sample using primer extension with an extension primer comprising a first portion that hybridizes to a portion of the microRNA molecule and a second portion that hybridizes to the complement of a universal forward primer;
(2) amplifying the first DNA molecule to produce amplified DNA molecules using the universal forward primer and a reverse primer; and
(3) measuring the amount of the amplified DNA molecules.

19. The method of Claim 18, wherein at least one of the universal forward primer and the reverse primer comprises at least one nucleic acid molecule comprising a 2'-O,4'-C-methylene-β-D-ribofuranosyl moiety.

20. The method of Claim 18, wherein the amount of the amplified DNA molecules are measured using fluorescence-based quantitative PCR.

21. The method of Claim 18, wherein the amount of the amplified DNA molecules are measured using SYBR green dye.

22. A kit for detecting at least one mammalian target microRNA comprising at least one primer set specific for the detection of a target microRNA, the primer set comprising:
(1) an extension primer for producing a cDNA molecule complementary to a target microRNA, wherein the target microRNA has a length in the range of from 21 nucleotides to 25 nucleotides, the extension primer comprising a first portion that hybridizes to the target microRNA and a second portion having a hybridization sequence that hybridizes to the complement of a universal forward PCR primer;
(2) a universal forward PCR primer for amplifying the cDNA molecule, comprising a sequence selected to hybridize to the hybridization sequence on the second portion of the extension primer; and
(3) a reverse PCR primer for amplifying the cDNA molecule, comprising a sequence selected to hybridize to a portion of the cDNA molecule.

23. The kit according to Claim 22, wherein at least one of the universal forward and reverse PCR primers includes at least one nucleic acid molecule comprising a 2'-O,4'-C-methylene-β-D-ribofuranosyl moiety.

24. The kit according to Claim 22, wherein the extension primer has a length in the range of from 10 to 100 nucleotides.

25. The kit according to Claim 22, wherein the first portion of the extension primer has a length in the range of from 3 to 25 nucleotides.

26. The kit according to Claim 22, wherein the second portion of the extension primer has a length in the range of from 18 to 25 nucleotides.

27. The kit according to Claim 22, wherein the second portion of the extension primer has a nucleic acid sequence comprising the nucleic acid sequence of SEQ ID NO: 1.

28. The kit according to Claim 22, wherein the universal forward PCR primer has a length in the range of from 16 to 100 nucleotides.

29. The kit according to Claim 22, wherein the universal forward primer consists of the nucleic acid sequence set forth in SEQ ID NO: 13.

30. The kit according to Claim 22, wherein the reverse PCR primer has a length in the range of from 10 to 100 nucleotides.

31. The kit according to Claim 22, wherein the reverse PCR primer comprises from 1 to 25 nucleic acid molecules comprising a 2'-O,4'-C-methylene-β-D-ribofuranosyl moiety.

32. The kit according to Claim 22, wherein the at least one mammalian target microRNA is a human microRNA.

33. The kit according to Claim 32, wherein the at least one target microRNA is selected from the group consisting of miR-1, miR-7, miR-9*, miR-10a, miR-10b, miR-15a, miR-15b, miR-16, miR-17-3p, miR-17-5p, miR-18, miR-19a, miR-19b, miR-20, miR-21, miR-22, miR-23a, miR-23b, miR-24, miR-25, miR-26a, miR-26b, miR-27a, miR-28, miR-29a, miR-29b, miR-29c, miR-30a-5p, miR-30b, miR-30c, miR-30d, miR-30e-5p, miR-30e-3p, miR-31, miR-32, miR-33, miR-34a, miR-34b, miR-34c, miR-92, miR-93, miR-95, miR-96, miR-98, miR-99a, miR-99b, miR-100, miR-101, miR-103, miR-105, miR-106a, miR-107, miR-122, miR-122a, miR-124, miR-124, miR-124a, miR-125a, miR-125b, miR-126, miR-126*, miR-127, miR-128a, miR-128b, miR-129, miR-130a, miR-130b, miR-132, miR-133a, miR-133b, miR-134, miR-135a, miR-135b, miR-136, miR-137, miR-138, miR-139, miR-140, miR-141, miR-142-3p, miR-143, miR-144, miR-145, miR-146, miR-147, miR-148a, miR-148b, miR-149, miR-150, miR-151, miR-152, miR-153, miR-154*, miR-154, miR-155, miR-181a, miR-181b, miR-181c, miR-182*, miR-182, miR-183, miR-184, miR-185, miR-186, miR-187, miR-188, miR-189, miR-190, miR-191, miR-192, miR-193, miR-194, miR-195, miR-196a, miR-196b, miR-197, miR-198, miR-199a*, miR-199a, miR-199b, miR-200a, miR-200b, miR-200c, miR-202, miR-203, miR-204, miR-205, miR-206, miR-208, miR-210, miR-211, miR-212, miR-213, miR-213, miR-214, miR-215, miR-216, miR-217, miR-218, miR-220, miR-221, miR-222, miR-223, miR-224, miR-296, miR-299, miR-301, miR-302a*, miR-302a, miR-302b*, miR-302b, miR-302d, miR-302c*, miR-302c, miR-320, miR-323, miR-324-3p, miR-324-5p, miR-325, miR-326, miR-328, miR-330, miR-331, miR-337, miR-338, miR-339, miR-340, miR-342, miR-345, miR-346, miR-363, miR-367, miR-368, miR-370, miR-371, miR-372, miR-373*, miR-373, miR-374, miR-375, miR-376b, miR-378, miR-379, miR-380-5p, miR-380-3p, miR-381, miR-382, miR-383, miR-410, miR-412, miR-422a, miR-422b, miR-423, miR-424, miR-425, miR-429, miR-431, miR-448, miR-449, miR-450, miR-451, let7a, let7b, let7c, let7d, let7e, let7f, let7g, let7i, miR-376a, and miR-377.

34. The kit according to Claim 32, wherein the at least one target microRNA is selected from the group consisting of: miR-1, miR-7, miR-10b, miR-26a, miR-26b, miR-29a, miR-30e-3p, miR-95, miR-107, miR-141, miR-143, miR-154*, miR-154, miR-155, miR-181a, miR-181b, miR-181c, miR-190, miR-193, miR-194, miR-195, miR-202, miR-206, miR-208, miR-212, miR-221, miR-222, miR-224, miR-296, miR-299, miR-302c*, miR-302c, miR-320, miR-339, miR-363, miR-376b, miR-379, miR-410, miR-412, miR-424, miR-429, miR-431, miR-449, miR-451, let7a, let7b, let7c, let7d, let7e, let7f, let7g, and let7i.

35. The kit according to Claim 22, wherein the at least one target microRNA is a murine microRNA.

## Patentansprüche

1. Ein Verfahren zum Herstellen von DNA Molekülen von einem Ziel-mikroRNA-Molekül mit einer Länge in dem Bereich von 21 bis 25 Nukleotiden, das Verfahren umfassend die Schritte:
(a) Herstellen eines ersten DNA Moleküls, das komplementär zu einem Ziel-mikroRNA-Molekül ist, unter Verwendung von Primer Extension mit einem Extension Primer umfassend einen ersten Teil, der mit einem Teil des mikroRNA-Moleküls hybridisiert, und einen zweiten Teil, der mit dem Komplementären eines universellen Forward Primer hybridisiert; und
(b) Vervielfältigen des ersten DNA Moleküls zum Herstellen vervielfältigter DNA Moleküle unter Verwendung des universellen Forward Primers und eines Reverse Primers.

2. Das Verfahren gemäß Anspruch 1, wobei mindestens einer der universellen Forward Primer und der Reverse Primers mindestens ein Nukleinsäuremolekül umfassend einen 2'-O,4'-C-methylen-β-D-ribofuranosyl-Rest umfasst.

3. Verfahren gemäß Anspruch 1, wobei die Primer Extension einen Extension Primer mit einer Länge in dem Bereich von 10 bis 100 Nukleotiden verwendet.

4. Verfahren gemäß Anspruch 1, wobei die Primer Extension einen Extension Primer mit einer Länge in dem Bereich von 20 bis 35 Nukleotiden verwendet.

5. Verfahren gemäß Anspruch 1, wobei der erste Teil des Extension Primers eine Länge in dem Bereich von 3 bis 25 Nukleotiden hat.

6. Verfahren gemäß Anspruch 1, wobei der zweite Teil des Extension Primers eine Länge von 18 bis 25 Nukleotiden hat.

7. Verfahren gemäß Anspruch 1, wobei der zweite Teil des Extension Primers eine Nukleinsäuresequenz hat, umfassend die Nukleinsäuresequenz von SEQ ID Nr: 1.

8. Verfahren gemäß Anspruch 1, wobei der universelle Forward Primer eine Länge in dem Bereich von 16 bis 100 Nukleotiden hat.

9. Verfahren gemäß Anspruch 1, wobei der universelle Forward Primer aus der Nukleinsäuresequenz wie in SEQ ID Nr: 13 gezeigt, besteht.

10. Verfahren gemäß Anspruch 2, wobei der universelle Forward Primer mindestens ein Nukleinsäuremolekül umfassend einen 2'-O,4-C-methylen-β-D-ribofuranosyl-Rest umfasst.

11. Verfahren gemäß Anspruch 10, wobei der universelle Forward Primer von 1 bis 25 Nukleinsäuremoleküle umfassend einen 2'-O,4'-C-methylen-β-D-ribofuranosyl-Rest umfasst.

12. Verfahren gemäß Anspruch 1, wobei der Reverse Primer eine Länge in dem Bereich von 10 bis 100 Nukleotiden hat.

13. Verfahren gemäß Anspruch 2, wobei der Reverse Primer mindestens ein Nukleinsäuremolekül umfassend einen 2'-O,4'-C-methylen-β-D-ribofuranosyl-Rest umfasst.

14. Verfahren gemäß Anspruch 13, wobei der Reverse Primer von 1 bis 25 Nukleinsäuremoleküle umfassend einen 2'-O,4'-C-methylen-β-D-ribofuranosyl-Rest umfasst.

15. Verfahren gemäß Anspruch 1, wobei der Reverse Primer ausgewählt ist, spezifisch mit einem DNA Molekül, das komplementär zu einem ausgewählten mikroRNA-Molekül ist, unter definierten Hybridisierungsbedingungen zu hybridisieren.

16. Verfahren gemäß Anspruch 1, weiter umfassend den Schritt des Messens der Menge von vervielfältigten DNA Molekülen.

17. Verfahren gemäß Anspruch 1, wobei die Vervielfältigung durch mehrfache aufeinander folgende PCR-Reaktionen erreicht wird.

18. Ein Verfahren zum Messen der Menge an Ziel-mikroRNA mit der Länge in dem Bereich von 21 bis 25 Nukleotiden in einer Probe von einem lebenden Organismus, das Verfahren umfassend den Schritt des Messens der Menge eines Ziel-mikroRNA-Moleküls in einer Vielfalt von verschiedenen Zelltypen innerhalb eines lebenden Organismus, wobei die Menge des Ziel-mikroRNA-Moleküls gemessen wird durch ein Verfahren umfassend die Schritte:
(1) Herstellen eines ersten DNA Moleküls das komplementär zum Ziel-mikroRNA-Molekül ist in der Probe unter Verwendung von Primer Extension mit einem Extension Primer umfassend einen ersten Teil, der mit einem Teil des mikroRNA-Moleküls hybridisiert und einem zweiten Teil, der mit dem Komplementären eines universellen Forward Primers hybridisiert;
(2) Vervielfältigen des ersten DNA Moleküls zum Herstellen vervielfältigter DNA Moleküle benutzend den universellen Forward Primer und einen Reverse Primer; und
(3) Messen der Menge der vervielfältigten DNA Moleküle.

19. Verfahren gemäß Anspruch 18, wobei mindestens einer von universellem Forward Primer und dem Reverse Primer mindestens ein Nukleinsäuremolekül umfassend einen 2'-O,4'-C-methylen-β-Dribofuranosyl-Rest umfasst.

20. Das Verfahren gemäß Anspruch 18, wobei die Menge der vervielfältigten DNA Moleküle mittels fluoreszenzbasierter quantitativer PCR gemessen wird.

21. Das Verfahren gemäß Anspruch 18, wobei die Menge der vervielfältigten DNA Moleküle mittels Farbstoff SYBR green gemessen wird.

22. Ein Kit zum Detektieren mindestens einer Säugetier-Ziel-mikroRNA umfassend mindestens ein Primer Set spezifisch zur Detektion von einer Ziel-mikroRNA, das Primer Set umfassend:
(1) Einen Extension Primer zum Herstellen eines cDNA Moleküls komplementär zu einer Ziel-mikroRNA, wobei die Ziel-mikroRNA eine Länge in dem Bereich von 21 bis 25 Nukleotiden hat, der Extension Primer umfassend einen ersten Teil, der mit der Ziel-mikroRNA hybridisiert, und einen zweiten Teil, mit einer Hybridisierungssequenz, die mit dem Komplementären eines universellen Forward PCR Primers hybridisiert;
(2) Einen universellen Forward PCR Primer für die Vervielfältigung des cDNA Moleküls umfassend eine Sequenz ausgewählt, um mit der Hybridisierungssequenz auf dem zweiten Teil des Extension Primers zu hybridisieren; und
(3) Einen Reverse PCR Primer zur Vervielfältigung des cDNA Moleküls umfassend eine Sequenz ausgewählt, um mit einem Teil des cDNA Moleküls zu hybridisieren.

23. Kit gemäß Anspruch 22, wobei mindestens einer der universellen Forward und Reverse PCR Primer mindestens ein Nukleinsäuremolekül umfassend einen 2'-O,4'-C-methylen-β-D-ribofuranosyl-Rest beinhaltet.

24. Kit gemäß Anspruch 22, wobei der Extension Primer eine Länge in dem Bereich von 10 bis 100 Nukleotiden hat.

25. Kit gemäß Anspruch 22, wobei der erste Teil des Extension Primers eine Länge in dem Bereich von 3 bis 25 Nukleotiden hat.

26. Kit gemäß Anspruch 22, wobei der zweite Teil des Extension Primers eine Länge in dem Bereich von 18 bis 25 Nukleotiden hat.

27. Kit gemäß Anspruch 22, wobei der zweite Teil des Extension Primers eine Nukleinsäuresequenz umfassend die Nukleinsäuresequenz mit der SEQ ID Nr: 1 hat.

28. Kit gemäß Anspruch 22, wobei der universelle Forward PCR Primer eine Länge in dem Bereich von 16 bis 100 Nukleotiden hat.

29. Kit gemäß Anspruch 22, wobei der universelle Forward Primer aus einer Nukleinsäure wie in SEQ ID Nr: 13 gezeigt, besteht.

30. Kit gemäß Anspruch 22, wobei der Reverse PCR Primer eine Länge in dem Bereich von 10 bis 100 Nukleotiden hat.

31. Kit gemäß Anspruch 22, wobei der Reverse PCR Primer von 1 bis 25 Nukleinsäuremolekülen umfassend einen 2'-O,4'-C-methylen-β-D-ribofuranosyl-Rest umfasst.

32. Kit gemäß Anspruch 22, wobei die mindestens eine Säugetier-Ziel-mikroRNA eine humane mikroRNA ist.

33. Kit gemäß Anspruch 32, wobei die mindestens eine Ziel-mikroRNA ausgewählt ist aus der Gruppe, bestehend aus miR-1, miR-7, miR-9*, miR-10a, miR-10b, miR-15a, miR-15b, miR-16, miR-17-3p, miR-17-5p, miR-18, miR-19a, miR-19b, miR-20, miR-21, miR-22, miR-23a, miR-23b, miR-24, miR-25, miR-26a, miR-26b, miR-27a, miR-28, miR-29a, miR-29b, miR-29c, miR-30a-5p, miR-30b, miR-30c, miR-30d, miR-30e-5p, mi-30e-3p, miR-31, miR-32, miR-33, miR-34a, miR-34b, miR-34c, miR-92, miR-93, miR-95, miR-96, miR-98, miR-99a, miR-99b, miR-100, miR-101, miR-103, miR-105, miR-106a, miR-107, miR-122, miR-122a, miR-124, miR-124, miR-124a, miR-125a, miR-125b, miR-126, miR-126*, miR-127, miR-128a, miR-128b, miR-129, miR-130a, miR-130b, miR-132, miR-133a, miR-133b, miR-134, miR-135a, miR-135b, miR-136, miR-137, miR-138, miR-139, miR-140, miR-141, miR-142-3p, miR-143, miR-144, miR-145, miR-146, miR-147, miR-148a, miR-148b, miR-149, miR-150, miR-151, miR-152, miR-153, miR-154*, miR-154, miR-155, miR-181a, miR-181b, miR-181c, miR-182*, miR-182, miR-183, miR-184, miR-185, miR-186, miR-187, miR-188, miR-189, miR-190, miR-191, miR-192, miR-193, miR-194, miR-195, miR-196a, miR-196b, miR-197, miR-198, miR-199a*, miR-199a, miR-199b, miR-200a, miR-200b, miR-200c, miR-202, miR-203, miR-204, miR-205, miR-206, miR-208, miR-210, miR-211, miR-212, miR-213, miR-213, miR-214, miR-215, miR-216, miR-217, miR-218, miR-220, miR-221, miR-222, miR-223, miR-224, miR-296, miR-299, miR-301, miR-302a*, miR-302a, miR-302b*, miR-302b, miR-302d, miR-302c*, miR-302c, miR-320, miR-323, miR-324-3p, miR-324-5p, miR-325, miR-326, miR-328, miR-330, miR-331, miR-337, miR-338, miR-339, miR-340, miR-342, miR-345, miR-346, miR-363, miR-367, miR-368, miR-370, miR-371, miR-372, miR-373*, miR-373, miR-374, miR-375, miR-376b, miR-378, miR-379, miR-380-5p, miR-380-3p, miR-381, miR-382, miR-383, miR-410, miR-412, miR-422a, miR-422b, miR-423, miR-424, miR-425, miR-429, miR-431, miR-448, miR-449, miR-450, miR-451, let7a, let7b, let7c, let7d, let7e, let7f, let7g, let7i, miR-376a und miR-377.

34. Kit gemäß Anspruch 32, wobei die mindestens eine Ziel-mikroRNA ausgewählt ist aus der Gruppe, bestehend aus: miR-1, miR-7, miR-10b, miR-26a, miR-26b, miR-29a, miR-30e-3p, miR-95, miR-107, miR-141, miR-143, miR-154*, miR-154, miR-155. miR-181a, miR-181b, miR-181c, miR-190, miR-193, miR-194, miR-195, miR-202, miR-206, miR-208, miR-212, miR-221, miR-222, miR-224, miR-296, miR-299, miR-302c*, miR-302c, miR-320, miR-339, miR-363, miR-376b, miR-379, miR-410, miR-412, miR-424, miR-429, miR-431, miR-449, miR-451, let7a, let7b, let7c, let7d, let7e, let7f, let7g und let7i.

35. Kit gemäß Anspruch 22, wobei die mindestens eine Ziel-mikroRNA eine MausmikroRNA ist.

## Revendications

1. Procédé de production de molécules d'ADN à partir d'une molécule de micro-ARN cible ayant une longueur comprise dans la gamme de 21 nucléotides à 25 nucléotides, le procédé comprenant les étapes consistant à :
(a) produire une première molécule d'ADN qui est complémentaire de la molécule de micro-ARN cible en utilisant l'extension d'amorce avec une amorce d'extension comprenant une première partie qui s'hybride à une partie de la molécule de micro-ARN et une seconde partie qui s'hybride au complément d'une amorce avant universelle, et
(b) amplifier la première molécule d'ADN pour produire des molécules d'ADN amplifiées en utilisant l'amorce avant universelle et une amorce inverse.

2. Procédé selon la revendication 1, dans lequel au moins un élément parmi l'amorce avant universelle et l'amorce inverse comprend au moins une molécule d'acide nucléique comprenant une portion 2'-O,4'-C-méthylène-β-D-ribofuranosyle.

3. Procédé selon la revendication 1, dans lequel l'extension d'amorce utilise une amorce d'extension ayant une longueur comprise dans la gamme de 10 à 100 nucléotides.

4. Procédé selon la revendication 1, dans lequel l'extension d'amorce utilise une amorce d'extension ayant une longueur comprise dans la gamme de 20 à 35 nucléotides.

5. Procédé selon la revendication 1, dans lequel la première partie de l'amorce d'extension a une longueur dans la gamme de 3 à 25 nucléotides.

6. Procédé selon la revendication 1, dans lequel la seconde partie de l'amorce d'extension a une longueur de 18 à 25 nucléotides.

7. Procédé selon la revendication 1, dans lequel la seconde partie de l'amorce d'extension a une séquence d'acide nucléique comprenant la séquence d'acide nucléique de la SEQ. ID N°1.

8. Procédé selon la revendication 1, dans lequel l'amorce avant universelle a une longueur comprise dans la gamme de 16 nucléotides à 100 nucléotides.

9. Procédé selon la revendication 1, dans lequel l'amorce avant universelle est constituée de la séquence d'acide nucléique indiquée dans la SEQ. IN N°13.

10. Procédé selon la revendication 2, dans lequel l'amorce avant universelle comprend au moins une molécule d'acide nucléique comprenant une portion 2'-O,4'-C-méthylène-β-D-ribofuranosyle.

11. Procédé selon la revendication 10, dans lequel l'amorce avant universelle comprend de 1 à 25 molécules d'acide nucléique comprenant une portion 2'-O,4'-C-méthylène-β-D-ribofuranosyle.

12. Procédé selon la revendication 1, dans lequel l'amorce inverse a une longueur dans la gamme de 10 nucléotides à 100 nucléotides.

13. Procédé selon la revendication 2, dans lequel l'amorce inverse comprend au moins une molécule d'acide nucléique comprenant une portion 2'-O,4'-C-méthylène-β-D-ribofuranosyle.

14. Procédé selon la revendication 13, dans lequel l'amorce inverse comprend de 1 à 25 molécules d'acide nucléique comprenant une portion 2'-O,4'-C-méthylène-β-D-ribofuranosyle.

15. Procédé selon la revendication 1, dans lequel l'amorce inverse est choisie de façon à s'hybrider spécifiquement à une molécule d'ADN complémentaire d'une molécule de micro-ARN choisie dans des conditions d'hybridation définies.

16. Procédé selon la revendication 1, comprenant en outre l'étape consistant à mesurer la quantité de molécules d'ADN amplifiées.

17. Procédé selon la revendication 1, dans lequel l'amplification est réalisée par de multiples réactions PCR successives.

18. Procédé de mesure de la quantité d'un micro-ARN cible ayant une longueur dans la gamme de 21 nucléotides à 25 nucléotides dans un échantillon provenant d'un organisme vivant, le procédé comprenant l'étape consistant à mesurer la quantité d'une molécule de micro-ARN cible dans une multiplicité de types de cellules différentes dans un organisme vivant, dans lequel la quantité de la molécule de micro-ARN cible est mesurée par un procédé comprenant les étapes consistant à :
(1) produire une première molécule d'ADN complémentaire de la molécule de micro-ARN cible dans l'échantillon en utilisant une extension d'amorce avec une amorce d'extension comprenant une première partie qui s'hybride à une partie de la molécule de micro-ARN et une seconde partie qui s'hybride au complément d'une amorce avant universelle ;
(2) amplifier la première molécule d'ADN pour produire des molécules d'ADN amplifiées en utilisant l'amorce avant universelle et une amorce inverse ; et
(3) mesurer la quantité de molécules d'ADN amplifiées.

19. Procédé selon la revendication 18, dans lequel au moins une amorce avant universelle et l'amorce inverse comprennent au moins une molécule d'acide nucléique comprenant une portion 2'-O,4'-C-méthylène-β-D-ribofuranosyle.

20. Procédé selon la revendication 18, dans lequel la quantité de molécules d'ADN amplifiées est mesurée en utilisant une PCR quantitative à base de fluorescence.

21. Procédé selon la revendication 18, dans lequel la quantité de molécules d'ADN amplifiées est mesurée en utilisant un colorant vert SYBR.

22. Kit pour détecter au moins un micro-ARN cible mammifère comprenant au moins un kit d'amorce spécifique pour la détection d'un micro-ARN cible, le kit d'amorce comprenant :
(1) une amorce d'extension pour produire une molécule d'ADNc complémentaire d'un micro-ARN cible, dans laquelle le micro-ARN cible a une longueur dans la gamme de 21 nucléotides à 25 nucléotides, l'amorce d'extension comprenant une première partie qui s'hybride au micro-ARN cible et une seconde partie ayant une séquence d'hybridation qui s'hybride à un complément d'une amorce PCR avant universelle ;
(2) une amorce PCR avant universelle pour amplifier la molécule d'ADNc, comprenant une séquence choisie pour s'hybrider à la séquence d'hybridation sur la seconde partie de l'amorce d'extension ; et
(3) une amorce PCR inverse pour amplifier la molécule d'ADNc, comprenant une séquence choisie pour s'hybrider à une partie de la molécule d'ADNc.

23. Kit selon la revendication 22, dans lequel au moins une des amorces PCR avant universelle et inverse comprend au moins une molécule d'acide nucléique comprenant une portion 2'-O,4'-C-méthylène-β-D-ribofuranosyle.

24. Kit selon la revendication 22, dans lequel l'amorce d'extension a une longueur comprise dans la gamme de 10 à 100 nucléotides.

25. Kit selon la revendication 22, dans lequel la première partie de l'amorce d'extension a une longueur dans la gamme de 3 à 25 nucléotides.

26. Kit selon la revendication 22, dans lequel la seconde partie de l'amorce d'extension a une longueur dans la gamme de 18 à 25 nucléotides.

27. Kit selon la revendication 22, dans lequel la seconde partie de l'amorce d'extension a une séquence d'acide nucléique comprenant la séquence d'acide nucléique de la SEQ. ID N°1.

28. Kit selon la revendication 22, dans lequel l'amorce PCR avant universelle a une longueur dans la gamme de 16 à 100 nucléotides.

29. Kit selon la revendication 22, dans lequel l'amorce avant universelle consiste en une séquence d'acide nucléique indiquée dans la SEQ. ID N°13.

30. Kit selon la revendication 22, dans lequel l'amorce PCR inverse a une longueur dans la gamme de 10 à 100 nucléotides.

31. Kit selon la revendication 22, dans lequel l'amorce PCR inverse comprend de 1 à 25 molécules d'acide nucléique comprenant une portion 2'-O,4'-C-méthylène-β-D-ribofuranosyle.

32. Kit selon la revendication 22, dans lequel l'au moins un micro-ARN cible mammifère est un micro-ARN humain.

33. Kit selon la revendication 32, dans lequel l'au moins un micro-ARN cible est choisi dans le groupe constitué de miR-1, miR-7, miR-9*, miR-10a, miR-10b, miR-15a, miR-15b, miR-16, miR-17-3p, miR-17-5p, miR-18, miR-19a, miR-19b, miR-20, miR-21, miR-22, miR-23a, miR-23b, miR-24, miR-25, miR-26a, miR-26b, miR-27a, miR-28, miR-29a, miR-29b, miR-29c, miR-30a-5p, miR-30b, miR-30c, miR-30d, miR-30e-5p, miR-30e-3p, miR-31, miR-32, miR-33, miR-34a, miR-34b, miR-34c, miR-92, miR-93, miR-95, miR-96, miR-98, miR-99a, miR-99b, miR-100, miR-101, miR-103, miR-105, miR-106a, miR-107, miR-122, miR-122a, miR-124, miR-124, miR-124a, miR-125a, miR-125b, miR-126, miR-126*, miR-127, miR-128a, miR-128b, miR-129, miR-130a, miR-130b, miR-132, miR-133a, miR-133b, miR-134, miR-135a, miR-135b, miR-136, miR-137, miR-138, miR-139, miR-140, miR-141, miR-142-3p, miR-143, miR-144, miR-145, miR-146, miR-147, miR-148a, miR-148b, miR-149, miR-150, miR-151, miR-152, miR-153, miR-154*, miR-154, miR-155, miR-181a, miR-181b, miR-181c, miR-182*, miR-182, miR-183, miR-184, miR-185, miR-186, miR-187, miR-188, miR-189, miR-190, miR-191, miR-192, miR-193, miR-194, miR-195, miR-196a, miR-196b, miR-197, miR-198, miR-199a*, miR-199a, miR-199b, miR-200a, miR-200b, miR-200c, miR-202, miR-203, miR-204, miR-205, miR-206, miR-208, miR-210, miR-211, miR-212, miR-213, miR-213, miR-214, miR-215, miR-216, miR-217, miR-218, miR-220, miR-221, miR-222, miR-223, miR-224, miR-296, miR-299, miR-301, miR-302a*, miR-302a, miR-302b*, miR-302b, miR-302d, miR-302c*, miR-302c, miR-320, miR-323, miR-324-3p, miR-324-5p, miR-325, miR-326, miR-328, miR-330, miR-331, miR-337, miR-338, miR-339, miR-340, miR-342, miR-345, miR-346, miR-363, miR-367, miR-368, miR-370, miR-371, miR-372, miR-373*, miR-373, miR-374, miR-375, miR-376b, miR-378, miR-379, miR-380-5p, miR-380-3p, miR-381, miR-382, miR-383, miR-410, miR-412, miR-422a, miR-422b, miR-423, miR-424, miR-425, miR-429, miR-431, miR-448, miR-449, miR-450, miR-451, let7a, let7b, let7c, let7d, let7e, let7f, let7g, let7i, miR-376a, et miR-377.

34. Kit selon la revendication 32, dans lequel l'au moins un micro-ARN cible est choisi dans le groupe constitué de : miR-1, miR-7, miR-10b, miR-26a, miR-26b, miR-29a, miR-30e-3p, miR-95, miR-107, miR-141, miR-143, miR-154*, miR-154, miR-155, miR-181a, miR-181b, miR-181c, miR-190, miR-193, miR-194, miR-195, miR-202, miR-206, miR-208, miR-212, miR-221, miR-222, miR-224, miR-296, miR-299, miR-302c*, miR-302c, miR-320, miR-339, miR-363, miR-376b, miR-379, miR-410, miR-412, miR-424, miR-429, miR-431, miR-449, miR-451, let7a, let7b, let7c, let7d, let7e, let7f, let7g et let7i.

35. Kit selon la revendication 22, dans lequel l'au moins un micro-ARN cible est un micro-ARN murin.
